# EUROPEAN PATENT APPLICATION

(11) **EP 4 752 534 A1**
(43) Date of publication of application: **03.06.2026**
(21) Application number: 24845176.7
(22) Date of filing: 24.05.2024
(51) Int. Cl.: G01N 21/66, C12M 1/00, C12M 1/34, G01N 21/64

(54) **ANALYSIS DEVICE AND ANALYSIS METHOD**

(30) Priority: 26.07.2023 JP 2023121744
(71) Applicant: Konica Minolta, Inc., Tokyo 100-7015 (JP)
(72) Inventor: TANAKA, Tatsuo, Chiyoda-ku, Tokyo 100-7015 (JP); KAWAHARA, Yusuke, Chiyoda-ku, Tokyo 100-7015 (JP); IZUMI, Tomoo, Chiyoda-ku, Tokyo 100-7015 (JP); MINAMI, Haruki, Chiyoda-ku, Tokyo 100-7015 (JP); OKUDA, Hirohito, Chiyoda-ku, Tokyo 100-7015 (JP); KITA, Hiroshi, Chiyoda-ku, Tokyo 100-7015 (JP)
(74) Representative: MFG Patentanwälte Meyer-Wildhagen Meggle-Freund Gerhard PartG mbB
(86) International application number: PCT/JP2024/019218
(87) International publication number: WO 2025/022794

(57) **Abstract**

The present invention addresses the problem of providing an analysis method capable of analyzing the state of a target object in detail in a short time without requiring complicated operations or devices. An analysis method for solving the abovementioned problem comprises: a step of mixing and causing an interaction between a target object and one or more types of luminescent probe having a luminescence behavior that changes in response to an interaction with the target object; a step of exciting the one or more types of luminescent probe in a state of interaction with the target object to generate two or more types of luminescence, and simultaneously detecting the two or more types of luminescence; and a step of analyzing the two or more types of detected luminescence to identify the state of the target object.

## Description

### Technical Field

The present invention relates to an analysis apparatus and an analysis method.

### Background Art

### 1-1. Advantages of Adapting Data Driving to Object Manufacturing

Conventional "manufacturing" is generally achieved by a technician called an expert or a craftsman on the basis of things or facts that have been established for many years by his/her own experience and learning and by handling the things or facts a priori, that is, in a so-called "forward direction." Basically, it is a "subjective" way that relies on the sense of a main (reader) person, and although it sometimes succeeds, it often repeats trial and error. Aside from deduction and induction, abduction (hypothesis verification) is a method of advancing research and development and technical development, and this is recommended as the most excellent method in academia and industry. In universities and the like, students learn this in a laboratory and travel to society.

On the other hand, the data driving is completely different from the deduction and the abduction described above, is noticed from data inductively, and derives a solution directly from data. Therefore, data driving is called an "inverse problem solving method" or a "reverse direction." The most familiar and successful example of the inverse problem solving method is e-commerce (electronic commerce) utilizing big data, and the power and the degree of spread thereof are just noticeable. A method of applying the same concept to "manufacturing" is called development DX or production DX. Although these are acclaimed to be epoch-making methods particularly in the industry, they stay leading topics in actual development sites and production sites. In the actual situation, the conventional method is applied as it is, and is regarded as a "legacy wall" or the like.

On the other hand, there was a NEDO project in which 18 companies formed a research association for chemical substances and chemical materials, and the National Institute of Advanced Industrial Science and Technology participated in the association to demonstrate manufacturing DX for target chemical materials in Japan. This is an activity called "ultra-advanced materials ultra-high-speed development-based technology project" (abbreviated as "ultra-ultra project"). Over 6 years from 2016 to 2022, 18 companies brought different model materials as development targets, and the National Institute of Advanced Industrial Science and Technology also conducted the above-described activities with one material as a theme. The activity is a project which has been advanced with a worldwide unique and challenging goal of achieving a development period or the number of trials of 1/20 or less by integrating "measurement-calculation-process" into three places, in particular, by utilizing data-driven development. Then, for a total of 19 themes, an estimation has been obtained that it is possible to perform development in a period of 1/23 in a simple average with respect to a conventional development period. The results are no longer fortuitous, but imply the necessity of this approach. The minutes of the post-evaluation of the project are also published to the public. As is clear from this large-scale demonstration experiment, it is interpreted that the importance of data driving in "manufacturing" also transcends the above-described e-commerce, and it is predicted that such a method will dominate the industry in the near future.

### 1-2. Data required for data driving

Targets of "manufacturing" in the industrial field include buildings, houses, and various other things such as ships, automobile, robots, printing apparatus, video equipment, and camera. Furthermore, targets of "manufacturing" include parts and the like that constitute these products, and products are formed by designing and processing these. In "manufacturing" where the final product is a "machine," the scale (size) to be controlled is usually in the range of centimeters to millimeters, since the manufacturing process has traditionally been done by hand. In manufacturing these products, the size of a component or the like has been rapidly measured and converted into data. This method is carried out on a large scale and is also referred to as factory automation. It is relatively easy to generate data at such a manufacturing site and use the data to perform data-driven development or data-driven production, and such a method has already been applied worldwide.

On the other hand, the targets of the above-described ultra-ultra project are chemical substances and chemical materials (composites), and are micron-sized fillers and fiber, high-molecular polymers having a size of submicron to several tens of nm, and substances having a size of Å to several nm such as low-molecular functional substances and low-molecular additives. In addition, in the case where these materials are used in a combined manner according to the use or data-driven, it is impossible to find a solution in a data-driven manner unless precise measurements can be made of the parts that are the source of the function.

### 1-3. Problem of data driving in manufacturing of complex system

Taking chemical composite materials as an example, it is extremely difficult to distinguish whether the desired properties are due to micron-sized fillers, the entanglement of submicron-sized polymer chains, or minute bonds and interactions in the Å to nm range, such as hydrogen bonds or van der Waals bonds between low-molecular-weight compounds present as additives and other substances present. For this reason, traditionally, each expert would carefully conduct scientific measurements in their area of expertise, then deductively understand the phenomenon, and research and development would be carried out in a forward-looking manner by combining these multiple understandings of the phenomenon.

In recent years, in synthetic biology (hereinafter, also collectively referred to as "bio- or biotechnology"), which has become increasingly popular in recent years and uses biologically-related substances such as microorganisms and enzymes, it is even more difficult to obtain the desired results or accomplishments through deductive understanding of phenomena than with the chemical materials mentioned above. As described above, in the case where digital transformation (hereinafter, also referred to as "DX") for manufacturing things including those in biotechnology is to be performed, since a system is complicated, it is often difficult to determine which parts of the target are responsible for its functions for solving a data-driven inverse problem. Therefore, it is a problem that machine learning is performed in a data group that does not include "correct data," and it can be said that this limits and suppresses the spread of the machine learning to the industrial world.

### 1-4. Data acquisition method other than measurement

On the other hand, when the activities of the ultra-ultra project are analyzed from its report materials, it is considered that the reason why the goal of setting the development period to 1/20 or less of the conventional development period can be achieved in all of the 18 companies and 19 themes is that the inverse problem has been solved with the multiscale simulation as the base (only the forward problem has been solved for the four companies). The problems of the simulation are that a physical model is required, the selection of the model is subjective, the versatility of the model is poor, and it is necessary to select an adaptation rule corresponding to a target phenomenon and to adjust simulation parameters. Therefore, it takes time to select the conformity measurement and adjust the simulation parameters, and it is difficult to generally spread the method.

### 1-5. Advantages and disadvantages of real measurement

In order to overcome the above-described problem, it is conceivable to measure a target realistically and on a multi-scale. According to such a method, the weak points of the simulation can be substantially overcome. However, for data driving, a large amount of data needs to be generated quickly. If large amounts of data cannot be generated quickly, the rate at which data is acquired will ultimately becomes rate-limiting, and it will no longer be called digital transformation. When it is a major premise of DX to promote transformation using digital technology, it is no longer possible when data acquisition, which is the source of the transformation, becomes rate-limiting. Here, even though the term "multi-scale measurement" is used, as described above, a technology for quickly and reliably performing measurement on the cm scale or the mm scale already exists. Then, the measurement results are utilized as data in various industries. Therefore, although on a µm scale, it takes a little time to perform the measurement, a sophisticated device is required, and cost is increased, there is no significant obstacle to data driving.

As described above, in order to succeed in the "manufacturing DX," it is necessary to acquire data of a target in various sizes and scales quickly, in a large amount, and simply. However, in the case where a target is a chemical molecule, a biologically relevant molecule, or even a metabolite of a microorganism, almost all of the main phenomena governing the function are caused by intermolecular interactions such as hydrogen bonds and van der Waals bonds. The sizes that determine these are nm scale or sub-nm (≈ angstrom) scale. For example, when the distance between hydrogen atoms and oxygen atoms forming a hydrogen-bond are compared between 0. 18 nm and 0. 20 nm, the former hydrogen-bond is overwhelmingly strong. Therefore, when the sub-nm scale measurement cannot be performed, it can be said that the data driven type development cannot be performed for the production of a chemical system or a biological system. In addition, examples of interactions and bonds that act between molecules, in order of bond strength, are covalent bonds (coordinate bonds) > ionic bonds > metallic bonds > hydrogen bonds > van der Waals bonds. In the case of performing data drive involving these, measurement data on the nm to sub-nm scale is required. For example, when the processing of biomaterials and the like (for example, the production of leather) is digitized, the above-described chemical bonding is greatly involved in any of the steps of maintaining the structure of collagen protein, tanning, dyeing, fat-adding, finishing, and the like. Therefore, it can be said that the application range of this size measurement is very wide from the most advanced materials to familiar materials.

### 1-6. Direct measurement and indirect measurement

One of the reasons why the above-described nm-scale measurement is difficult is that the area is no longer visible even with the full use of an optical microscope or an electron microscope. Note that a low molecule itself can also be observed with an atomic force microscope (AFM) or the like. However, the pre-processing is complicated, and the measurement itself should also be carefully performed over time. Therefore, it is not suitable for data driving from the viewpoint of the amount of data that can be generated.

### 2-1. Relationship between data driven research and development/production and cyclic society

Here, in the ultra-smart society envisioned for 2030, the current fundamental principle of industrial structure, "mass production/mass consumption," will be fundamentally reconsidered, and the premise will be "to provide only what is needed, to the people who need it, when they need it, and only what is needed." That is, with respect to a product (manufacture), "produce only what is needed" is required, and in addition, in a period of technical development, rapidity and timeliness are required when it is "needed." In a recycling society, not to mention simply "produce without waste," greenhouse gases, in particular, emission of carbon dioxide is regulated. Therefore, it is essential to convert conventional petroleum-derived plastics and chemical substances produced by petroleum cracking into those of natural origin and biological origin as far as possible. That is, from the viewpoint of producing "only what is needed" and "without waste," the manufacturing process uses process informatics that is an inverse problem solver. The timeliness of "when needed" cannot be dealt with by the conventional deduction type, and therefore, materials informatics and bioinformatics, which are also inverse problem solving methods, are applied. In order to build such a cyclic society, a data-driven method represented by informatics is essential.

### 2-2. Problems when data driven-type becomes mainstream

Here, although the removal of chemical substances from the dependence on petroleum is a symbolic approach toward the recycling society, chemistry and biochemistry are less logical than the technical areas of physics, electricity, and machinery, and largely depend on the experience and skill of engineers and researchers. That is, this is the biggest problem in performing data driving. As described above, in order to move towards a data-driven approach, it is necessary to turn a phenomenon occurring in a complicated system into data rapidly, simply, accurately, and in a large amount.

As described above, in particular, in chemistry and biochemistry, since the root of a mechanism that controls a function is an intermolecular interaction, it is a big problem to generate data on a scale from Å to nm so as to meet the above-described demands. In the case where a synthetic biological method using microorganisms or organism-related substances is applied to the production of chemical substances, fungi or bacteria on a submicron scale and microorganisms (a generic name for protists and metazoans) on a submillimeter scale that prey on them also directly or indirectly participate in chemical synthesis (i.e., metabolism). Therefore, in order to generate data therefor, it is necessary to perform some measurement and use them as descriptors or explanatory variables. In other words, in these fields, it can be said that data acquisition, which is completely different from the manufacture of mechanical systems, is essential for the realization of a cyclic society, which is a current burden.

### 2-3. Blind spot on sustainability in object manufacturing

On the other hand, in recent years, synthetic biological methods using biological reactions and microorganisms have been attracting attention as new production methods for the recycling society from the viewpoint of breaking away from petroleum dependence, but there are significant blind spots here. About 30 years ago, green chemistry, which is a chemical technology for performing a synthesis reaction without using an organic solvent, was in the spotlight, but it has now completely died down, and the number of reported papers is also drastically decreasing. The reason for this was the conclusion that even when only the synthesis reaction is carried out in the absence of a solvent, the chemical substance must necessarily be subjected to a removal means called "extraction" and a quality improvement means called "purification" with high purity, which requires a large amount of an organic solvent, and consequently, there is almost no effect.

The same applies to synthetic biology. Contrary to popular belief, it is not possible to produce useful organic compounds using living organisms alone. Rather, in a biochemical reaction, the reaction is often carried out under an ultradilute concentration of a liquid/liquid interface, and rather, a larger amount of an organic solvent is required than in a conventional synthesis process. When the organic solvent used at this time is incinerated, of course, carbon dioxide is discharged into the atmosphere. Therefore, a demand for purification treatment of organic substances using activated sludge, which has been used in the chemical industry for a long time, has increased more than ever.

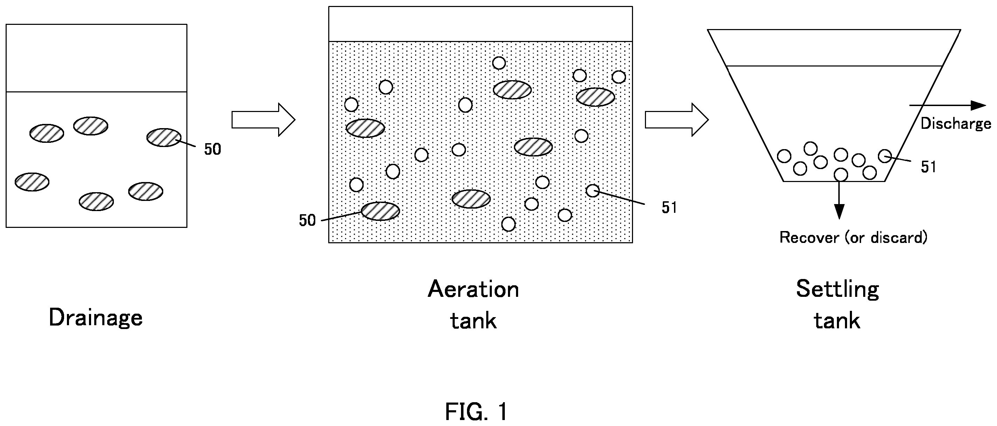

Fig. 1 illustrates a schematic diagram of a standard activated sludge method constituted by connecting an aeration tank using activated sludge and a precipitation tank. Biological treatment, which is one of wastewater treatment methods, is a method of removing dirt from wastewater by utilizing microorganisms, and a representative example thereof is a wastewater treatment method called "standard activated sludge method" illustrated in Fig. 1. The above-described "standard activated sludge method" is a method of feeding microorganisms 51 (activated sludge) at a high concentration (about 4000 mg/L) in a pool or a tank to eat dirt in wastewater . The microorganisms 51 are classified, in terms of their properties, into those that utilize oxygen (aerobic microorganisms) and those that do not require oxygen (anaerobic microorganisms). A standard activated sludge method utilizes aerobic microorganisms. The aerobic microorganisms grow by decomposing the organic matters 50 in the wastewater while taking in oxygen in the water. Therefore, a facility for constantly blowing air into the tank (aeration) is required. In addition, when the microorganisms 51 are proliferated, suspended matter (activated sludge) called floc is generated, and more types of microorganisms remove dirt in the wastewater. While the wastewater is purified in the aeration tank, microorganisms grow more and more. Therefore, it is necessary to separate the increased microorganisms from the cleaned water. Good microorganisms 51 (activated sludge ) have a property of sinking downward while being accompanied by surrounding floating matters when left to stand. Therefore, it is common that the water in the aeration tank is drawn into another tank (a precipitation tank) and allowed to stand, and only the supernatant water is discharged into a river or the sea after sterilization. Further, it is general that a part of the settled microorganisms 51 (activated sludges) is returned to the aeration tank to maintain the concentration of the microorganisms 51 (activated sludges), and the excessively increased activated sludges are disposed as wastes or the like.

Of the microorganisms in the activated sludge, those most contributing to the purification of sewage are bacteria, which gather to form aggregations of bacteria of the activated sludge. Typical examples of flocking bacteria include genus *Zoogloea,* genus *Bacillus,* genus *Pseudomonas,* genus Flavobacterium, and the like. On the other hand, protozoa are microorganisms whose shapes can be recognized by a microscope of about 100 magnifications in activated sludge, and they eat flocs to reduce the amount thereof to sink or comb, or eat floating bacteria to purify treated water. Examples of these include ciliates such as *Vorticella, Epistylis, Aspidisca,* and *Litonotus;* sarcodines such as *Arcella* and *Amoeba;* and flagellates such as *Entosiphon* and *Peranema.* Furthermore, metazoans of multicellular organisms can also be observed at a magnification of about 100 times, and typical examples thereof include rotifers, rotifers, Aeolosomatidae, black beetles, nematodes, and the like.

The above wastewater treatment methods are almost the same all over the world, but because the purification mechanism is biologically derived, as described above, the activity of the waste organic matter treatment is prone to vary greatly depending on factors such as temperature, oxygen concentration, the amount and type of waste organic matter that serves as food for fungi and microorganisms, and the distribution of microbial species in the activated sludge. In addition, seasonal variation and the like also tends to occur. In response to these issues, most management is done primarily on a personal level, based on the experience and knowledge of the staff and experts in charge, and as demand increases in the future, it will become extremely important to digitalize this purification process.

In a broader sense, activated sludge treatment itself can also be considered a form of synthetic biology, in the sense that it uses fungi and microorganisms to convert waste organic matter, which serves as raw material, into useful chemicals that can be released into rivers or the ocean. The purification process DX can be applied to process informatics based on synthetic biology for producing almost other advanced chemicals. This is, an area where new data-driven methods are expected to be applied in both the waste and manufacturing aspects.

### 2-4. DX for food processing

Although whether it is called the manufacturing industry is delicate, there are many challenges in food processing as well. For example, most recently, in response to the globalization of food manufacturing and distribution, the revised Food Sanitation Act was passed in June 2018, and as of June 1, 2020, the introduction of HACCP (Hazard Analysis and Critical Control Point) has become mandatory in Japan. Then, after a grace period of one year, "HACCP fully mandatory" has been required for all food-related companies from June, 2021. The HACCP subdivides manufacturing processes, performs risk management for each step, can prevent shipment of problematic products, and even when a food accident occurs, can quickly determine which step is the cause of the food accident. Since this is a law required by those other than large-scale manufacturers, it is very difficult in terms of cost to manage all the steps by advanced analytical equipment, and since the items to be managed are diverse, how to cope with this is a big problem.

In a conventional method, "sampling inspection" from "packaging" to "shipping" has been the mainstream. On the other hand, the HACCP (Hasap) method is a sanitary management method for ensuring the safety of a product by "predicting harm such as contamination by microorganisms or mixing of foreign substances" and "continuously monitoring and recording a particularly important step leading to prevention of harm" in each step from reception of raw materials to processing and shipment. Therefore, as compared with the conventional sampling inspection of the final product, it is possible to prevent the shipment of a more problematic product. On the other hand, cost and labor (man-hours) for inspection and analysis, large-scale modification of a manufacturing process, and the like have already become significant problems. HACCP is a system and regulation that has only just begun in Japan, so it is not fully understood by those outside the industry that it is a major issue. However, it is obvious that it is essential to take this measure from various angles beyond the food industry.

### 2-5. Problems of data acquisition and data management in primary sector

Quality assurance is also an important action in such food processing and production, and countermeasures have been taken by various methods. However, what should be considered as a problem is that the evaluation items of quality assurance are limited to the management of practices and process condition (for example, heating at 100°C for 2 minutes, annealing at room temperature for 1 hour after shaping, and the like), and there are many cases where essential analysis regarding quality is not performed.

It is needless to say that such a quality assurance system is based on the premise of "mass production/mass consumption," which has been the center of the manufacturing industry. In on-demand manufacturing for ultra-smart society, inspection is no longer applicable, and a mechanism for individually and easily maintaining and guaranteeing quality is required.

When manufacturing "only what is needed, to the people who need it, and when they need it," traceability during the manufacturing should be rather important than the final characteristics and quality of the product. In addition, the fact that a strictly controlled raw material is produced by a manufacturing process with high transparency itself becomes quality, which leads to consumer's credibility. Therefore, it is important to simply record and digitize the traceability of the manufacturing process. As on-demand products are widely distributed throughout the world, preparation for product liability becomes a major challenge.

The traceability described above is one of the provisions, but the raw material to be used, the intermediate in the case of being taken out in the middle, and the final product each record the identity and state of the substance, that is, a wide range of identification is necessarily required. Even today, there are many devices that use QR codes (registered trademarks) or barcodes to record IDs. However, assuming that IDs are also required for raw materials, it is essential to devise and spread a method by which IDs can be easily assigned and acquired also for persons in charge of primary sector. In addition, it is a big challenge to store and manage IDs of all raw materials, intermediates, and final products when they are further produced on-demand, and furthermore, to trace back to the upstream of production by connecting data when a problem or the like occurs. These cannot be dealt with by the current human-centered management, and it is premised that artificial intelligence (AI) is effectively utilized.

In addition, as in the case of the above-described AFM (atomic force microscope), when data is to be obtained from a target which can no longer be directly measured, there is no other way but indirect measurement. In the case where there is a technical restriction that direct measurement cannot be performed, humanity has hitherto utilized a medium or a chip for indirect measurement as an intermediate to transmit information that can be determined by humans therethrough. For example, in the fields of X-ray film, toner, photoreceptor, and the like, intervening media have been utilized.

Here, the following techniques have been proposed as methods for indirectly measuring various physical properties, characteristics, and the like, and aggregating the data. For example, PTL 1 describes that various physical quantities such as electric energy and an air flow rate are measured to monitor air leakage in a system to which electric power is supplied by a bus duct method. Further, PTL 2 describes an apparatus for easily imaging various physical phenomena or chemical phenomena such as a two dimensional distribution of pH, pressure, magnetic field, or temperature of a solution. PTL 3 describes a measurement information transmission device and a multipoint measurement information collection system that efficiently collect physical quantities measured at multiple points. In addition, PTL 4 describes a method of measuring distortion due to displacement of a nanometer scale by light intensity or a polarizing direction.

### Citation List

### Patent Literatures

PTL 1
   Japanese Unexamined Patent Publication No. 2004-152084
PTL 2
   Japanese Unexamined Patent Publication No. 2005-337806
PTL 3
   Japanese Unexamined Patent Publication No. 2004-173123
PTL 4
   Japanese Unexamined Patent Publication No. 2016-156762

### Summary of Invention

### Technical Problem

However, the devices and systems for indirect measurement described in the above Patent Literatures have a problem that it is difficult to reduce the time required for measurement of a minute sample. In particular, when an attempt is made to perform measurement on a minute sample, there are problems in that high-precision measurement is required, it is difficult to reduce time, and it is further difficult to acquire a large amount of data in a short time. In addition, a general-purpose device has a limit in resolution or the like, and there is a problem in that an expensive dedicated measurement apparatus is required.

Here, one of methods for, although relatively, reliably and simply measuring an interaction or binding between molecules, is a "luminescent probe." A typical luminescent probe is used as a means for qualitatively transmitting information to a human as a signal through interaction with or formation of a bond to a specimen. When it is possible to increase the amount of data by arraying the luminescent probes or the like using the principle of the luminescent probes and further perform work of taking a regression using machine learning with a simple device or a chip-like device, indirect measurement can be used for data driving.

In addition, on the premise that machine learning or deep learning is used, raw data itself to be measured does not need to be intelligible to a degree that can be understood by a human. In addition, when the amount of data is large, it is possible to overcome the accuracy by statistical mathematical processing and to convert the data into data or signals which can be substantially used for data driving. Changing this target (that performs determination) from a human to a machine is required in data-driven research and development and production.

The present invention has been made in view of the above-described technologies and problems. Specifically, an object of the present invention is to provide an analysis method and an analysis apparatus capable of analyzing a state of a target in detail in a short time without requiring a complicated operation or apparatus.

### Solution to Problem

### • Multi-Dimensionalization of Data

The AI technology is premised on a finite-dimensional linear space as input and output. In addition, various data (organisms, cells, DNA, reaction pathways, molecular structures, and the like) can be mapped in a finite-dimensional linear space. Examples of the mapping method include multiscale data (directed graph), a discrete structure (graph), embedding in a continuous space, and a submodular function.

### • Sufficient data volume

Furthermore, in complicated object manufacturing, a large amount of various data is required. The reason why a large amount of various data is required is that a model with a high degree of freedom including deep learning is required in order to explain a complicated phenomenon. In addition, in order to make a statistically meaningful inference with respect to the degrees of freedom of the model, it is considered that at least about ten times the number of degrees of freedom is required in practice. Furthermore, the reason why various types of data are required is that it is not easy to predict and define in advance which data is useful for an objective variable. There is a possibility that an unexpected explanatory variable has an influence. Therefore, it is meaningful to collect various quantities whose relationship with the objective variable is not obvious as candidates of candidate variables.

As described above, in order to collect a large amount of various data, a high-speed and inexpensive data collection means is required. Collecting data is both time consuming and expensive. Therefore, a means for collecting data at high speed and low cost is required. The background behind which deep learning has become possible is that it has been possible to collect a large amount of inexpensive data from a network. Here, there is data that is known to be important a priori. A priori knowledge can be reflected in the structure (architecture) of the network. In addition, a priori models and data-driven models can be combined. It can be said that the approach of improving the performance by using an a priori model as a first order approximation and additionally using data driving as a second order approximation is a universal approach.

### < Organic EL element as ultra-sensitive sensor >

### (1) Specificity of organic EL element

As one of the above-described real and inductive data acquisition methods, the present inventors have first considered an organic EL element, the essence of which has been fully known in our previous research and development, as a target. The present inventors have decided to consider, as a subject, a blue phosphorescence emitting element which is said to have the highest level of difficulty in organic EL-related technology and which has not yet been put into practical use in electronic displays of televisions and smartphones.

The biggest reason why blue phosphorescence has not been practically used in electronic display devices (green and red have been in practical use for more than 10 years ago) is simply the short emission lifetime of the devices. As a result of long-term studies on the cause, it has been found that, of course, another reason is that the blue phosphorescence emitting material has to be an aromatic compound or a complex compound having a wide band gap. However, it has been found that a slight aggregation or a change in the existing state of the host material existing in the surroundings, or a very small amount of water vapor or a gas component which is mixed from the outside of the element over time is more sensitive than in the case of green or red phosphorescence emission by several hundred times.

Upon understanding all the problems, we have the world's only track record that blue phosphorescence has been put to practical use in lighting devices (Symphos OLED-The 010K), and also have the background that we fully understand the particularity and specificity.

### (2) Reverse Idea

The main reason why the organic EL blue phosphorescence element causes a decrease in emission luminance, that is, the life of the light emitting element is shortened is not that the light-emitting material itself is decomposed. It is because that a change in the surroundings of a light-emitting material directly affects a phenomenon called light emission. Specifically, the influence of the purity of a host material present around a light-emitting material is much larger than that of green phosphorescence emission or red phosphorescence emission. Furthermore, as the glass transition point of the host material is lower, the state of the host changes more greatly over time after energization, and it is also known that blue phosphorescence emission is extremely sensitive to the influence of the state change and is more likely to be adversely affected as compared with other light emissions.

It is also possible to produce an organic EL element by a coating method using an organic solvent as a method of forming a light-emitting layer. In this case, it is also confirmed that the life characteristics of the blue phosphorescence element are largely changed even with the same coating solvent due to a difference in manufacturer or a difference in lot. Even when the purity and the purification degree are the same in terms of the specifications, large fluctuations in the performance of an element to be produced have also been a big problem for practical use. Furthermore, with respect to ultrapure water used before production of the element, even after removal of impurities to a detection limit or less, the emission lifetime characteristics of the element vary. Therefore, it is known that not only organic substances but also a very small amount of metal ions and the like affect light emission.

Although this is an effect promoted by blue phosphorescence, it is natural that basically the same thing occurs even in a green or red phosphorescent element or an organic EL element of a fluorescence emission method rather than phosphorescence to the extent that it is not recognized by human eyes. That is, the present inventors have considered that such an organic EL element sensitive to a substance or its environment can be used not as a light emission device but as a sensor device.

### (3) Organic EL element as sensor device

Under such a concept, the present inventors have examined an organic EL device as an ultra-sensitive sensor. A typical organic EL element is laminated and formed by a vacuum deposition method, and is produced under an environment completely controlled under high vacuum or under an inert atmosphere in order to be further completely blocked from the atmosphere. Therefore, not only organic EL devices produced as commercial products, but also organic EL elements for research and development cannot be taken out into the atmosphere during film formation.

On the other hand, we have also developed the technology of a phosphorescent organic EL element which can be produced under atmospheric pressure, and in such a production process, it is also possible to add a substance to be measured in the organic EL element or in the vicinity of the element during the production process. Therefore, it is possible to use this as a sensor device. When minute amounts of various substances to be measured can be added to organic EL elements in this way, it should be possible to precisely acquire multidimensional real data as described above. In fact, it has been confirmed that such data can be obtained in an organic EL element produced by a coating process.

### (4) Efficacy of simple OLEDs

However, since a high-performance coating-type organic EL phosphorescent element that we have developed usually adopts a lamination structure of four organic layers, we have also realized, through actual studies, that it is not practical to produce such a precise thin-film lamination element for the purpose of sensing. As a method capable of escaping from disadvantage, the present inventors have recently focused on simple-OLED which has been developed by the present inventors and which can be produced with one or two organic layers in the atmosphere.

Since this method can also be produced using an inkjet method, it is possible to easily form a sensor portion having any size and shape from a size of about several tens of micrometers to a larger size as the size of the sensor. In addition, due to the characteristics of the inkjet, the sensor portion can be formed as tens of thousands to millions of multipoint elements in one manufacturing process. In this way, in addition to the number of dimensions of data, the large amount of data also makes it possible to construct an innovative sensor device that can be said to be a truly destructive innovation.

In addition, although a direct current voltage is usually applied to an organic EL element, it is easy to use an alternating current for this application method, and furthermore, even in the case of an alternating current, it is easy to adopt a voltage application state such as a sine wave, a triangular wave, or a rectangular wave. In addition, such a change can further increase the number of dimensions also by a voltage application method.

In addition to high sensitivity, it is another great characteristic that various multi-dimensionalization can be achieved in this manner. The reason why data can be multi-dimensionalized by such a method is that the organic EL element is an electric field excitation light emitting element. The basic mechanism is derived from the recombination of holes and electrons, and in the process up to the recombination, all of the electrical state changes such as carrier traps and charge and discharge phenomena of charges in the compound affect the recombination position and the recombination region width, and the light emission interferes multiply. It can also be considered that this is due to the emission phenomenon specific to organic EL, such as total reflection at the cathode or organic layer interface, which manifests itself in the emission brightness, emission wavelength, emission spectrum waveform, current characteristics during emission, and impedance changes.

These considerations can be understood and noticed only after many failures and successes have been repeated in the light emission phenomenon of the organic EL element and the extension of the life thereof. It is only the inventors of the present invention that can properly understand this phenomenon and handle it as data used for data driving, and it is self-confident that it is an advanced invention.

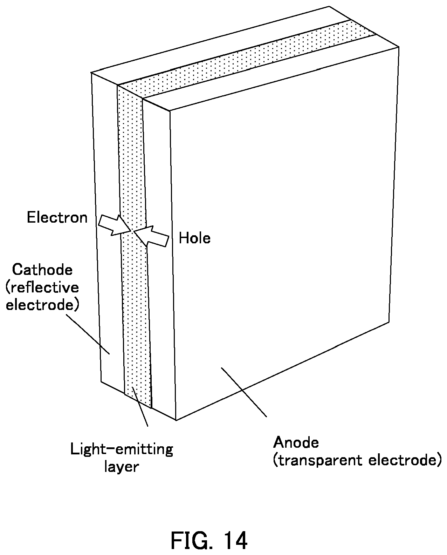

A sensing mechanism of the simple-OLED sensor will be described below with reference to the schematic diagram illustrated in Fig. 14.

In an organic EL (OLED), as illustrated in Fig. 14, holes are injected from an anode which is a transparent electrode, and at the same time, electrons are injected from a cathode which is a reflective electrode. Then, when these recombine on light-emitting molecules (also referred to as fluorescent molecules, phosphorescent molecules, or dopants) in the light-emitting layer and are deactivated to the ground state, light having a wavelength corresponding to the band gap is emitted. In the organic EL element, the thickness of the organic layer is about 100 nm to 300 nm in view of both the restriction of the smoothness of the surfaces of the transparent electrodes (generally, thin film layers of metallic oxides such as ITO and IZO) and the mechanism of exciting the electric field to be as thin as possible. This is the same for an organic EL element formed by a vacuum deposition method and an organic EL element formed by a coating method or an inkjet method.

In the vacuum deposition method, it is substantially impossible to return the pressure to the atmospheric pressure during the procedure and add a specimen into the element, but in the coating method or the inkjet method in which an organic layer is formed under the atmospheric pressure, it becomes possible to do so. In particular, in a simple-OLED including one layer or two layers of the organic thin film layer constituting the organic EL element developed by the present inventors, it is easy to add a specimen, and it is more suitable for use as a sensor than those by other methods.

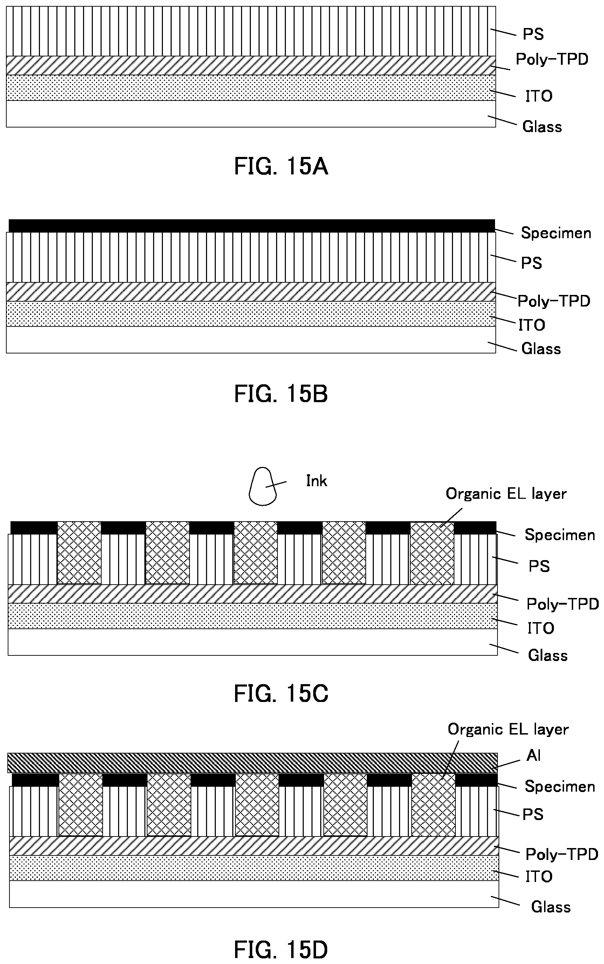

Two typical examples of the method of adding a specimen will be described with reference to Figs. 15A to 15D.

As illustrated in Fig. 15A, a simple-OLED is formed by laminating a substrate, transparent electrodes, an insolubilized film (for example, an organic thin film such as poly-TPD) formed by a large-area coating method such as spin coating or die coating, and a general-purpose polymer layer such as polystyrene. As illustrated in Fig. 15B, the specimen liquid from which data is to be obtained is placed on the polystyrene surface using a dropper, pipette, or the dipping method, allowing a small amount of the liquid to soak in, and then the excess liquid is blown off with an air gun or the like to form a layer. Next, as illustrated in Fig.15C, an ink in which a light emission dopant and a host are dissolved is ejected by an inkjet method. As a result, polystyrene containing the specimen component, the host, and the light emission dopant are mixed together, and the mixture is dried to form an organic EL layer containing a trace amount of the specimen component. As illustrated in Fig. 15D, a metallic cathode is laminated thereon by vapor deposition, sputtering, transfer, or the like, to complete simple-OLED element.

When a current and a voltage are applied to the organic EL element, a light emission phenomenon is observed, and it is output as a signal reflecting the interaction between a specimen component and a host or a dopant (light emitting molecule) of the organic EL element. That is, by measuring the light emission, a signal in the order of nm associated with the interaction is obtained.

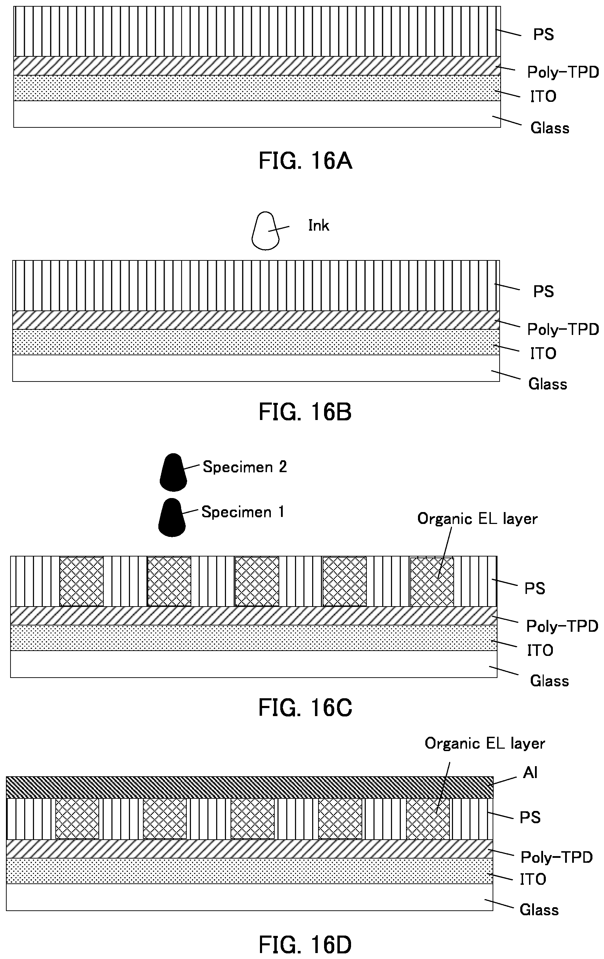

Another method of adding a specimen will be described with reference to Figs. 16A to 16D.

As illustrated in Fig. 16A, a laminate of a substrate, transparent electrodes, an insolubilized film (for example, an organic thin film such as poly-TPD) formed by a large-area coating method such as spin coating or die coating, and a general-purpose polymer layer such as polystyrene is prepared by the above method. Then, as illustrated in Fig. 16B, an ink in which a light emission dopant and a host are dissolved is ejected by an inkjet method to form an organic EL layer. Next, as illustrated in Fig. 16C, a material to be measured is made into ink and applied. The substance to be measured may be made into ink by directly adding the specimen to an ink solution of a host and a dopant for an organic EL. Further, by utilizing the characteristics of the high-accuracy position control of the inkjet, the alignment may be performed so as to perform the overlapping ejection from an ink tank and a head different from those of the ink for the organic EL. Thereafter, as illustrated in Fig. 16D, a metallic cathode is laminated by vapor deposition, sputtering, transfer, or the like, thereby completing the simple-OLED element.

In this case, although it takes time and effort to form the specimen as an ink, the specimen can also be added into the organic EL layer in a continuous amount for each organic EL element by using characteristics of minute amount/precision ejection of inkjet. Therefore, it is possible to generate a signal with less noise or higher accuracy.

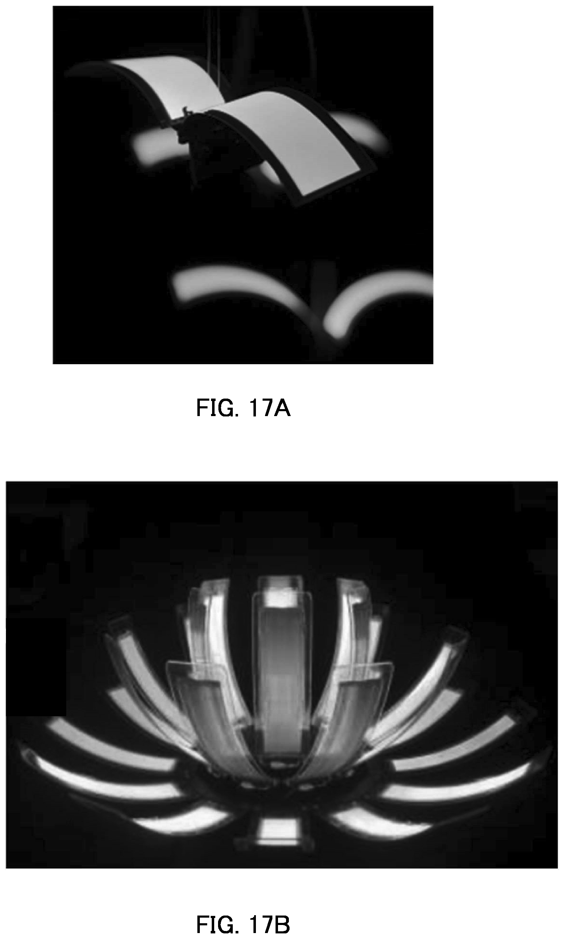

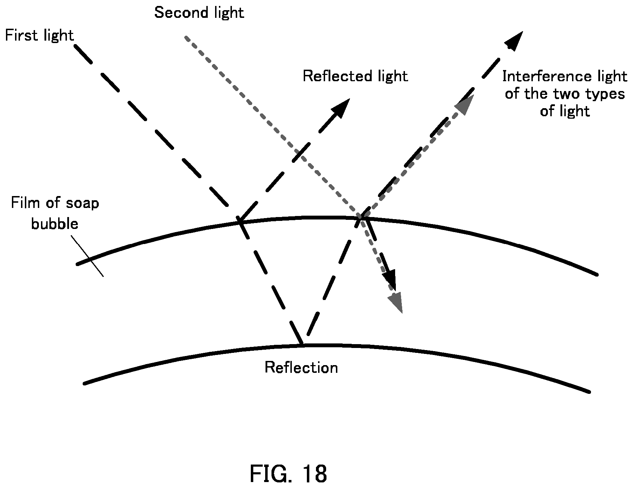

Next, the mechanism why the specimen component added in a very small amount can be sensed by a simple-OLED will be described. Photographs of the light emitting state of an organic EL element are illustrated in Fig. 17A and Fig. 17B. It is known that the color of an organic EL element changes depending on the angle at which a human observes the element. This is due to light interference, and is similar to the principle by which soap bubbles appear iridescent, as illustrated in Fig. 18, for example. As illustrated in Fig. 18, reflection or refraction of light occurs at the interface of the film of the soap bubble, and interference of a plurality of light further occurs.

On the other hand, in the organic EL element, as described above, the organic layer between the anode and the cathode is a thin film of about 100 nm to 300 nm. When the layer is designed so that the re-combination occurs substantially in the vicinity of its center, the light emission efficiency and the life of the light emitting device are improved. In particular, in a phosphorescent element in which a phosphorescent compound is used as a light-emitting dopant, it is considered to be suitable to recombine the electrons and the holes as much as possible in a central portion of the light-emitting layer (means for avoiding triplet-triplet annihilation, TTA). This phenomenon also occurs in the thermally activated delayed fluorescence (TADF) system. In such an "organic EL element in which recombination occurs not at the interface but inside the light-emitting layer," the change in the carrier recombination position due to the interaction between the trace component of the specimen and the light-emitting layer substance (host, dopant, polymer, or the like) of the organic EL is further enhanced. Thus, the dynamic range for signal acquisition is extended.

The present disclosure has the following two features.

Feature 1) Converting a physicochemical characteristic of a specimen into optical information by exerting various actions on the specimen itself. Furthermore, the number of types of optical information can be increased by changing the type and degree of the effect.

Feature 2) Being able to acquire a large amount of information at high speed by utilizing inkjet.

These characteristics function effectively in identifying and distinguishing a substance by utilizing AI.

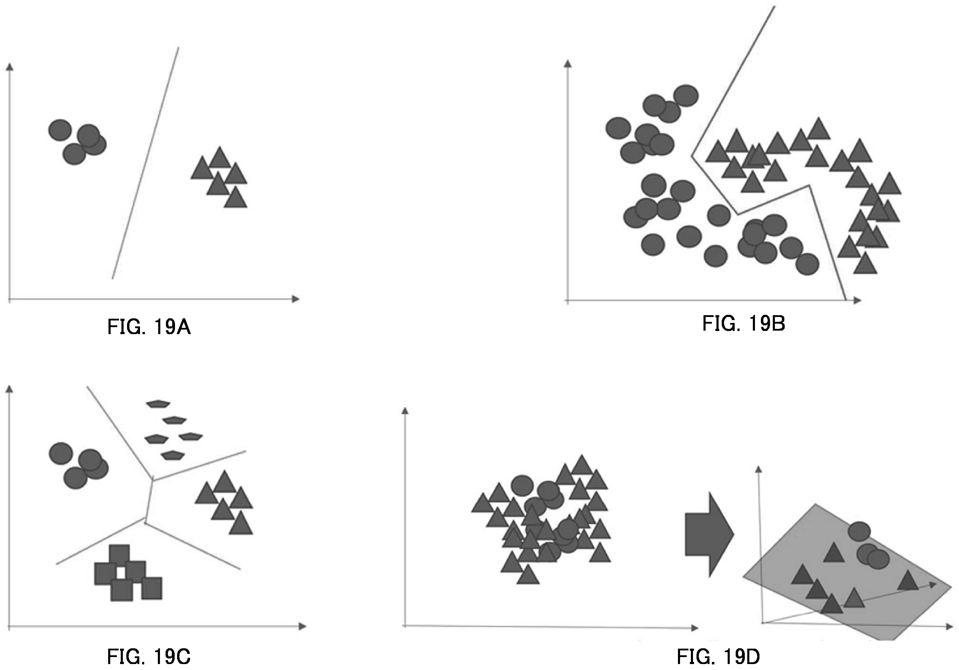

First, diagrams for explaining classification by machine learning are illustrated in Figs. 19A to 19D. Fig. 19A illustrates two classes of normal distributions. This represents the case where the distribution is simple and can be separated by one broken line (the number of parameters = 2). Fig. 19B represents a two class complex distribution. Four broken lines are required for separation (the number of parameters = 8). Fig. 19C illustrates a four class normal distribution. Five broken lines are required for separation (the number of parameters = 10). Fig. 19D illustrates an example for which separation is not possible in two dimensions but is possible when pulled up in three dimensions.

In machine learning classification, compared to the case where the distribution is simple and the number of target substances is small (Fig. 19A); (i) the more complicated the data distribution is (e.g., Fig. 19B) and (ii) the more target substances are (= the more classified classes are) (Fig. 19C), the greater the number of parameters needed to describe a separation surface separating classes (broken lines in the example of the drawings). Therefore, a large amount of data is required. Furthermore, even when class separation cannot be performed by low dimensions, class separation can be performed by increasing the types (= dimensions) of datasets in some cases (e.g., Fig. 19D). Since the number of parameters necessary for describing a surface to be separated increases in the case of a higher dimension, the number of necessary data increases. As described above, it is necessary to increase the amount and types (dimensions) of data in order to realize more accurate classification. This amount and type of problem can be solved by feature 1 and feature 2 of the present disclosure.

It is possible to reduce an estimation error of an average value of a class by averaging data of the same class on the assumption that the data independently follow the same probability distribution. This is equivalent to increasing the accuracy of estimation of the cluster center in the K-means method, and can be said to be the simplest form of machine learning. This is close to an image of increasing the accuracy of the position of the red line in Fig. 19A.

Note that machine learning is divided into (i) a learning phase and (ii) an inference phase. The learning phase is, so to speak, a preparation phase, in which the data used are used for parameter estimation (of the separation surface in the example described above). In the inference phase, all the collected data is used for inference.

That is, what can be achieved by the present disclosure is as follows.
1. The analysis apparatus is "to generate a large amount of signals for one specimen at once and quickly."
2. A majority of a large amount of generated signals are used to increase the quality of data, and data used for machine learning, deep learning, or the like is obtained by an analysis method using part of signals generated by the apparatus.
3. An analysis apparatus whose function is reinforced by artificial intelligence (AI), which is characterized by using the AI for improving the quality of data. is provided.
4. Signals generated from an analysis apparatus and data whose quality has been improved by using a majority of the signals are not used by a human to understand a phenomenon deductively but are "used to solve an inverse problem by machine learning."
5. Since the present invention is mainly applied to chemical or biochemical production, the characteristic of an obtained signal should be "sensitive to a size of the order of nm or Å" which is suitable (preferable) for describing an interaction between substances, and since it is difficult to rapidly, simply, and directly observe such a size, the size is converted into light and the light is measured, whereby a large amount of signals due to the size are acquired.

### Advantageous Effects of Invention

According to one embodiment of the present invention, an analysis method and an analysis apparatus capable of analyzing a state of a target in detail in a short time without requiring complicated work and apparatus are provided.

### Brief Description of the Drawings

Fig. 1 is a schematic diagram for explaining a standard activated sludge method;
Fig. 2 is a flowchart of an analysis method according to one embodiment of the present invention;
Fig. 3 is a diagram for explaining the structure of a luminescent probe (a multiple luminescent compound) used in the analysis method according to one embodiment of the present invention;
Fig. 4 is a diagram for explaining a method for producing a luminescent probe (multiple luminescent compound) used in an analysis method according to one embodiment of the present invention;
Fig. 5A to Fig. 5D are diagrams for explaining a mechanism by which a luminescent probe (multiple luminescent compound) used in an analysis method according to one embodiment of the present invention exerts its effect;
Fig. 6 is a schematic diagram for explaining an analysis apparatus used in the second mode of the analysis method according to one embodiment of the present invention;
Fig. 7 illustrates the results of principal component analysis in examples;
Fig. 8A illustrates linear discriminant analysis model plots when analyzing 7 types and 95 brands of beverages using the luminescent probes 1 to 15, and Fig. 8B illustrates a confusion matrix at this time;
Fig. 9A illustrates linear discriminant analysis model plots when analyzing 7 types and 95 brands of beverages using the luminescent probes 1 and 17 to 31, and Fig. 9B illustrates a confusion matrix at this time;
Fig. 10A illustrates linear discriminant analysis model plots when analyzing 7 types and 95 brands of beverages using the luminescent probes 1 to 15 and 17 to 31, and Fig. 10B illustrates a confusion matrix at this time;
Fig. 11A illustrates linear discriminant analysis model plots when analyzing 7 types and 95 brands of beverages using the luminescent probes 1 and 32 to 45, and Fig. 11B illustrates a confusion matrix at this time;
Fig. 12A illustrates linear discriminant analysis model plots when analyzing 7 types and 95 brands of beverages using the luminescent probes 46 to 60, and Fig. 12B illustrates a confusion matrix at this time;
Fig. 13A illustrates a linear discriminant analysis model plot when explanatory variables at the time of creating a discriminant model are replaced and seven types and 95 brands of beverages are analyzed, and Fig. 13B illustrates a confusion matrix at this time;
Fig. 14 is a diagram of the principle of light emission of an organic EL element;
Fig. 15A to Fig. 15D are schematic diagrams when a specimen is added to the organic EL element;
Fig. 16A to Fig. 16D are schematic diagrams when a specimen is added to the organic EL element;
Fig. 17A and Fig. 17B are photographs of the emission state of an organic EL element;
Fig. 18 is a diagram for explaining reflection and refraction of light at the interface of the film of a soap bubble;
Figs. 19A to 19D are diagrams for explaining classification by machine learning;
Fig. 20 is schematic diagram of various display elements;
Fig. 21 is a cross-sectional view illustrating an example of a microarray device;
Fig. 22 is a plan view illustrating an example of a microarray device;
Fig. 23 is a perspective view illustrating an example of the microarray device;
Fig. 24 is a cross-sectional view illustrating an example of a microarray device;
Fig. 25 is a plan view illustrating an example of a microarray device;
Fig. 26 is a cross-sectional view illustrating an example of a microarray device;
Fig. 27 is a cross-sectional view illustrating an example of a microarray device;
Fig. 28 is a cross-sectional view illustrating an example of a microarray device;
Fig. 29A to Fig. 29D are cross-sectional views illustrating an example of microarray devices;
Fig. 30 is a cross-sectional view illustrating an example of a microarray device;
Fig. 31 is a plan view illustrating an example of a microarray device;
Fig. 32 is a perspective view illustrating an example of a microarray device;
Fig. 33 is a diagram for explaining a method for producing an OLED element;
Fig. 34 is a diagram for explaining a method for producing an OLED element;
Fig. 35A to Fig. 35D are schematic diagrams illustrating a mechanism of a multidimensional, rapid, and massive data-generating method using simple-OLED;
Fig. 36 is a schematic diagram of sensing elements manufactured in example 4 and example 5;
Fig. 37 illustrates the data set obtained in examples;
Fig. 38 is a diagram illustrating the discriminant analysis result in example 4;
Fig. 39 illustrates the data set obtained in example 5; and
Fig. 40 is a diagram illustrating the discriminant analysis result in example 5.

### Description of Embodiments

### < Problems in data-driven manufacturing (or research and development) >

### (Change in rate-limiting process (bottleneck))

As described above, as means for generating a large amount of "essentially a priori data corresponding to a substance itself or an existing state of the substance," a microtiter plate (hereinafter, referred to as a "microplate") which is widely used in the research and development of biochemistry, an automatic dispenser which injects and drops a chemical solution or a specimen into an experimental field (well) of the plate, and a plate reader which automatically reads color or light are commonly used. However, although this method has been widely utilized in biochemistry, biotechnology, and the like for more than 20 years, it is hardly used in the chemical industry, other manufacturing industries, and the food processing industry.

This is because methods of preparing and processing samples are different among industries. For example, there are various reasons such as that the solvent is generally water in biochemistry but is an organic solvent in the chemical industry, that a high temperature of 80°C or more is often used in food processing, and that the capacity and scale of a target to be evaluated and inspected are different. In fact, a data acquisition method effectively utilized in the biochemical field is hardly used in other industrial areas.

### (1) Rate limiting in inductive approaches

That is, it is found that in many industries and academia, in particular, in the area targeting liquids or liquid-like substances, speedy measurement of a large number of samples is a bottleneck in transferring to the data-driven type, that is, the inductive method. On the other hand, in analysis apparatuses, precision analysis has become possible day and night due to technological innovation and spread of auto-samplers. Although this is one of measures to solve the bottleneck, it is still insufficient from the viewpoint of acquiring a large amount of data in a short time due to the necessity of high-precision analysis, and it is not easy to secure a sufficient amount of data for applying the inductive method.

Then, how can this bottleneck be eliminated? Although it can be said that the rate limiting in the deductive research and development that has been mainstream so far is "resolution of trade-off," it can be considered that the rate limiting has shifted to "resolution of bottleneck" because the number of data is a necessary condition in the data-driven, that is, inductive research and development. That is, in a category of human operation, it is difficult to collect a necessary amount of data for data driving, and it is necessary to attain machine-based automation excluding human power in all from sample creation to data generation, data loading, and data analysis.

### (2) Dry system high throughput and Wet system high throughput

However, such large-scale automation usually requires advanced mechatronic technology fully utilizing a robot hand and a control apparatus, and a large-scale device corresponding thereto, and requires a robot suitable for applications such as daily experiments and machining. Therefore, disadvantages occur in terms of cost and time for manufacturing the robot, and eventually, it is only an ideal theory and cannot be realized.

On the other hand, as an epoch-making method for overcoming such a problem, so-called computational science in which a human does not perform experiment, manufacturing, or processing in the first place and an a priori action that the human thinks is also predicted by large-scale calculation, and computer simulation utilizing the same are in the spotlight, and a method in which calculation on a computer actually substitutes for an experimental action of the human is also adopted.

In the area of biochemistry, medicine, and biotechnology, conventional experimental actions performed by humans are referred to as "Wet," and experimental predictions performed on a computer and the results thereof are referred to as "Dry," and these are used separately in appropriate places. Of course, similar things have already been widely implemented in the areas of chemistry and physics, and quantum chemistry calculation, dynamics calculation, and the like are their representatives, and high effects and results have been achieved in various areas in cooperation with experiments performed by humans.

However, even in the development act and the production act which seem to be speeded up in this way, the "Wet" portion which humans take charge of becomes a rate-limiting factor after all, and it is necessary to break the rate-limiting factor in order to realize the ultra-smart society in the future. In other words, beyond the fields of biochemistry and medicine, a method for using the plate reader to quickly obtain sufficient amounts of high-quality data in an inexpensive and easy-to-use, data-driven manner is essential for aligning manufacturing and research and development with the major inflection points of the SDGs, the super-smart society, and the Web 3.0 era. Then, realization of it is the largest gain, and the methodology is the time to become the invention.

### (3) Digital Transformation in inspection and diagnosis

As described above, it can be said that almost all of the inspections and diagnoses so far have been an act of requiring a definitive diagnosis of a target. That is, it can be said that devices have been developed in order to lead to an analysis result having a scientific basis that can be determined by a human (expert), and conversely, society has created a specialist who can use these devices and make a definitive diagnosis or determination.

However, as medicines have changed from low molecular weight compounds to bio-pharmaceuticals such as antibody medicines and nucleic acid medicines such as m-RNA, materials have also shifted from single materials such as metal plates to composite materials. Substances that can no longer be dealt with by definitive diagnosis or rigorous analysis have become essential in our active society. That is, a major changing point is that such things that humans want to use conveniently and safely can no longer be dealt with by conventional analysis apparatuses and diagnostic apparatuses. It should be considered that a new technology or methodology for non-definitive diagnosis corresponding to this has just become necessary.

When the number of dimensions exceeds three, analysis becomes suddenly difficult for humans because they are no longer able to keep track of coordinates in their heads. However, by using machine learning and deep learning with AI, it becomes possible to perform calculations and analysis even in ultra-multiple dimensions of hundreds or thousands, although the number of dimensions that can be used is not infinite. That is, it is considered to be a Digital Transformation in inspection and diagnosis to create a system for generating a large amount of multi-dimensional information that can fully exhibit its characteristics without human intervention.

### (4) To enable high-throughput data acquisition

In the case where a method using machine learning or deep learning, that is, informatics is used to perform non-definitive diagnosis and non-definitive inspection for those for which definitive diagnosis and definitive inspection cannot be performed as described above, the result is acquired not as a quantitative value but in the form of clustering. However, as the number of data increases, the matrix of the clustering also becomes finer, and therefore, when the matrix can be made finer and finer, a result which is clustering but is substantially similar to a quantitative value is output. That is, performing such high-definition clustering is a basic way of digital transformation in inspection and diagnosis.

In order to realize this, it is necessary to increase the number of wells by miniaturizing the above-described microplate for biochemistry as much as possible. In addition, a manipulator for arranging a specimen or a reagent in the microwell (experimental field) with high positional accuracy, and an apparatus for accurately and rapidly measuring information on the color, light, or electromagnetic wave of the microwell are also required.

In addition to such mechanical and apparatus requirements, scientific ingenuity for amplifying signals is also required in order to output color, light, or electromagnetic wave with high sensitivity as a result of interaction between a specimen and a reagent which can be supplied to a microwell. In response to such a wide variety of new requirements, the inventors of the present invention have devised means capable of rationally solving all the requirements, and have completed the technique of the present disclosure. A representative example of the technology of the present disclosure is described below.

### < High-throughput multi-well with self-contained light source >

### (Light source corresponding to reaction field (microwell) of microplate)

When data acquisition is performed using a microplate having a large number of microwells for the purpose of non-definitive diagnosis and non-definitive inspection as described above, for example, assume that milked milk is selected as a specimen. At this time, each of the large number of fine wells needs to be charged with, in addition to the milk, a reagent (also referred to as a "contrast agent" in the present specification) that can interact with some component in the milk to generate color, light, or electromagnetic wave. In addition, when the specimen is milk, there is no component that performs light emission itself. In addition, the color itself is basically white, and the change in chromaticity due to lot is also small. Therefore, a light source is required for high-sensitivity detection. In addition, since it is assumed that data is acquired in multiple dimensions, it is advantageous to use a wide variety of light sources.

On the other hand, providing such a multi-wavelength light source requires a large-scale device, which limits the place where it can be used and also causes inconvenience in terms of cost.

### (1) Ideas from electronic displays

As described above, information provision that has been intended for human eyes can be changed to provision for measurement machines and computers, so that the information can be used as a large amount of data, which should be considered as fuel for data-driven development. Electronic displays are a typical example of devices that provide stimuli in the form of optical information to the human eye. Such an electronic display almost always emits the three primary colors of light, that is, blue, green, and red as minute pixels in order to generate all wavelengths that can be recognized by humans as light.

Electronic displays are created by adapting this light emission to wavelengths that are sensitive to the rods and cones of the human eyes. The present inventors have found that such an electronic display can be used as a versatile on-demand light source for data science by manipulating the wavelength so that the display emits light at a wavelength appropriate for the contrast agent rather than the human eye.

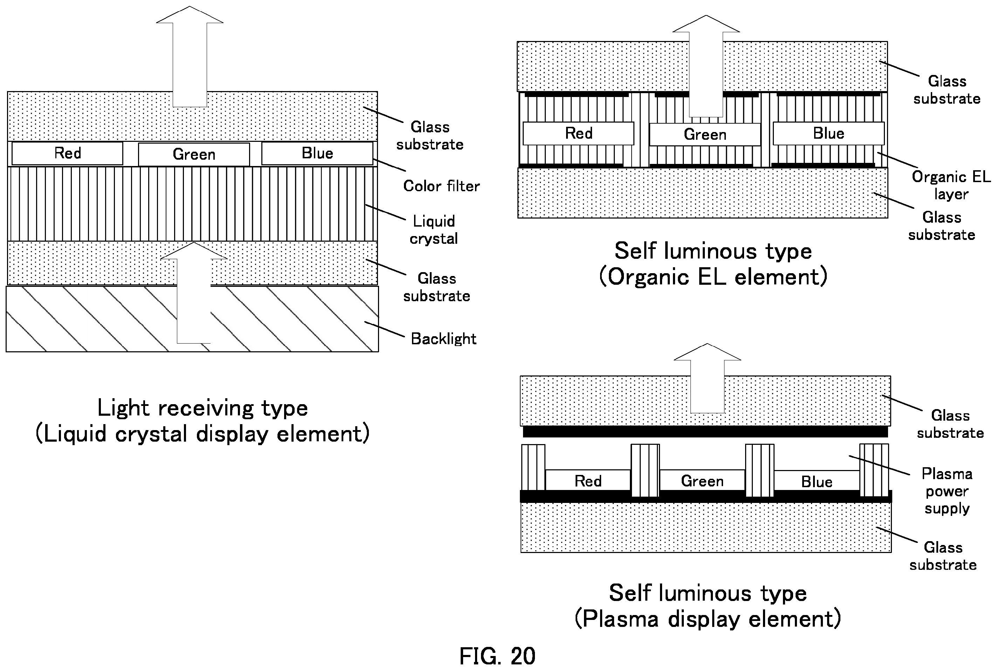

Fig. 20 illustrates a schematic diagram of a liquid crystal display element, an organic EL element, and a plasma display element. It is needless to say that a self-luminous plasma display or organic EL display as illustrated in Fig. 20 can serve as an excitation light source. In addition, in a light receiving type liquid crystal display, light eventually emitted to a backlight emitting white light through a color filter may serve as an excitation light source. Therefore, after understanding the configuration and principle of such an electronic display, the present inventors have conceived of using the electronic display as a multi-wavelength and multi-planar excitation light source (or viewing light source) by adjusting the wavelength to a wavelength suitable for the contrast agent rather than the conventional BGR.

It cannot be easily predicted that the combination of an electronic display, which is a consumer device, and a micro-plate for biochemical use can be effectively applied to the Digital Transformation of an original manufacturing system. However, the feasibility and effectiveness of the above-described logic may be easily understood.

### (2) Inspiration from high quality printing

Although electronic displays are currently mass-produced and the cost thereof has been greatly reduced, the manufacturing cost becomes a serious problem when the electronic displays are improved for the above-described data science. How to arrange ultra-multi-wells more inexpensively and easily in two dimensions? The answer lies in high quality digital printing.

Digital printing machines that use the electrophotographic method used in copiers to print on demand (even one sheet at a time) with quality comparable to gravure printing are already on the market (e.g., https://www.konicaminolta.jp/business/products/graphic/ondemand print/iq-501/index.html and https://www.konicaminolta.jp/business/products/graphic/ondemand print/iq-501/index.html).

An intelligent quality optimizer called IQ-501 built in the printing machine is a unit for optimizing qualities by automatically measuring adjustment of color, density, and register position, discarding due to human operation error and loss, and redoing work, which requires "manpower and expertise" at a normal printing site. By adapting this technology, it is entirely possible to read color information from pixels of several tens of microns across a large A2 size area at once and convert it into data. Furthermore, when it can be utilized for data science, it will be possible to measure and process unprecedented "big data associated with science due to substances" in a short time.

In particular, the light source side is based on the present invention in that an electronic display is used, and for example, the following sheet-shaped light source can be suitably utilized in the present invention.

### < Form of device for carrying out the invention >

The microarray device includes a plurality of two dimensionally disposed microdot light emitters and a plurality of two dimensionally disposed microwell structure parts. This feature is a technical feature common to or corresponding to the following embodiments.

One embodiment of the microarray device includes an organic EL element, and the organic EL element includes the plurality of microdot light emitters disposed two dimensionally.

One embodiment of the microarray device includes an organic EL element, the organic EL element includes an anode, a receiving layer, a cathode, a sealing member, a circuit, and a power receiver (power receiving part), and the receiving layer includes an insulator and a plurality of the microdot light emitters disposed two dimensionally.

One embodiment of the microarray device includes two or more types of microdot light emitters having different light emission maximum emission wavelengths. As a result, wavelength selectivity and explanatory variables are increased, and analysis accuracy is improved.

One embodiment of the microarray device includes a plurality of microdot light emitters including at least two or more of the following: a microdot light emitter having a maximum emission wavelength of less than 380 nm, a microdot light emitter having a maximum emission wavelength of 380 nm or more and less than 500 nm, and a microdot light emitter having a maximum emission wavelength of 500 nm or more. Thus, the analysis accuracy is further improved.

One embodiment of the microarray device includes a plurality of microdot light emitters including at least a microdot light emitter having a maximum emission wavelength of less than 380 nm, a microdot light emitter having a maximum emission wavelength of 380 nm or more and less than 500 nm, and a microdot light emitter having a maximum emission wavelength of 500 nm or more. Thus, the analysis accuracy is further improved.

In one embodiment of the microarray device, in-plane coordinates of the microdot light emitter and in-plane coordinates of the microwell structure part at least partially overlap each other. With such a positional relationship, the absorption spectrum data and the light emission spectrum data of a specimen can be captured at once at the time of detection. Furthermore, such a positional relationship can shorten the optical path length, thereby enabling the microwell structure parts and the microdot light emitters to be provided at a higher density.

In one embodiment of the microarray device, the number of the microdot light emitters is greater than the number of the microwell structure parts, and a plurality of microdot light emitters are located directly below each of the microwell structure parts. With such a positional relationship, it is possible to irradiate one microwell structure part with excitation light having different wavelengths without using a spectroscopic system. By irradiating one microwell structure part 22 with excitation light of different wavelengths, explanatory variables can be increased, and analysis accuracy can be improved.

In one embodiment of the microarray device, the number of the microdot light emitters matches the number of the microwell structure parts, and in-plane central coordinates of the respective microdot light emitters match in-plane central coordinates of the respective microwell structure parts. With such a positional relationship, the excitation light emission efficiency is maximized, so that the analysis accuracy can be improved.

One embodiment of the microarray device includes a receiving layer including an insulator and a plurality of the microdot light emitters disposed two dimensionally. In the receiving layer, a material of the insulator and a material of the microdot light emitter are present with a concentration gradient in a depth direction and an in-plane direction.

In one embodiment of the microarray device, at least one of the plurality of microwell structure parts contains a contrast agent on its surface. Thus, the sensitivity of data acquisition can be improved. Further, by including a contrast agent on the surface, data to be generated can be multi-dimensionalized. Specifically, data to be generated is changed by interaction, such as energy transfer between the specimen and the contrast agent, and therefore, data on the molecular structure, mixture amount ratio, density, environment, and the like of the specimen can be generated in a more multidimensional manner.

In one embodiment of the microarray device, at least one of the plurality of microwell structure parts contains a contrast agent on its surface. The contrast agent uses electromagnetic waves irradiated from the microdot light emitter as excitation light and emits one or two or more types of the following: fluorescence, phosphorescence, excimer light emission, exciplex light emission, thermally activated delayed fluorescence, excited state intramolecular proton light emission, triplet triplet annihilation light emission, twisted intramolecular charge transfer light emission, and aggregated organic light emission. These light emission mechanisms are suitable for multi-dimensionalization of generation data.

In one embodiment of the microarray device, at least one of the plurality of microwell structure parts includes a contrast agent on its surface, and the contrast agent is an oligonucleotide. An oligonucleotide is suitable as the contrast agent in that it can be adjusted to the shape of a specimen. Furthermore, an oligonucleotide is also preferable in that the distance and the ratio of amounts between molecular groups can be controlled by block polymerization as compared with general compounds.

In the method for manufacturing a microarray device, the microwell structure parts are formed by an inkjet method. As a result, microwell structure parts can be formed easily and quickly. In addition, the inkjet method is also suitable for forming a microwell structure part having an arbitrary shape or a minute size.

In one embodiment of the method for manufacturing a microarray device, the microdot light emitters are formed by an inkjet method. As a result, a large number of microdot light emitters can be formed easily and quickly. In addition, the inkjet method is also suitable for forming a microdot light emitter having an arbitrary shape or a minute size.

The inspection kit is an inspection kit including a microarray device, and includes the microarray device and one or two or more of the following: a contrast agent, a specimen, a dropping instrument, cover glass, a curing agent, a lens, and a microscope.

The inspection system is an inspection system including a microarray device, and includes the microarray device, an imaging apparatus, and an analysis apparatus.

In one embodiment of the inspection system, the analysis apparatus extracts RGB data or hyperspectral data from digital image data generated using the microarray device and the imaging apparatus. Thus, even a large amount of data or complicated data can be quickly analyzed with high accuracy.

In one embodiment of the inspection system, the analysis apparatus evaluates the state of a specimen using artificial intelligence. Thus, even a large amount of data or complicated data can be quickly analyzed with high accuracy.

The inspection method is characterized by using the microarray device.

### [Microarray Device]

The microarray device includes a plurality of two dimensionally disposed microdot light emitters and a plurality of two dimensionally disposed microwell structure parts.

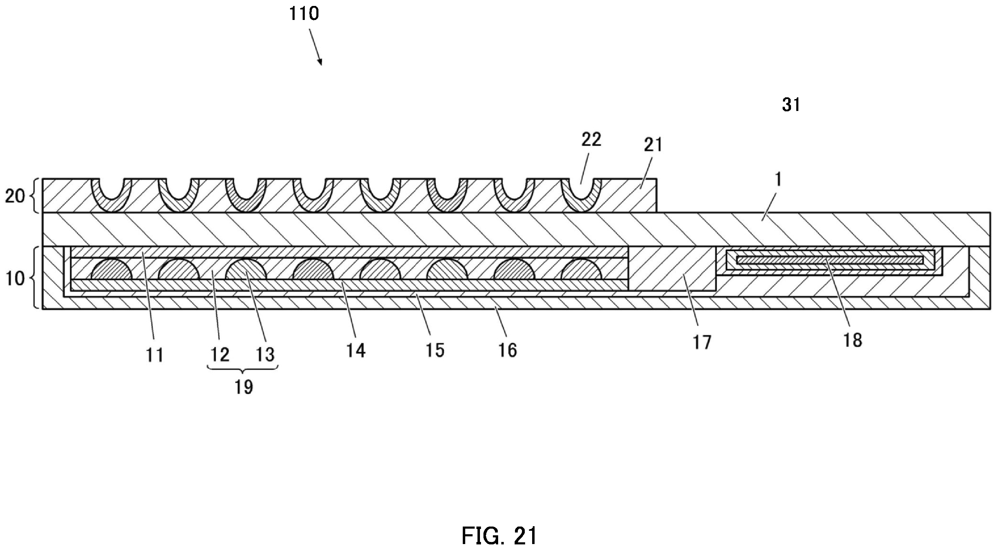

Fig. 21 is a cross-sectional view illustrating an example of the microarray device. A microarray device 110 illustrated in Fig. 21 includes a sheet-shaped light source unit 10 located on the lower surface of a support 31, and a sheet-shaped multi-well unit 20 located on the upper surface of the support 31. The light source unit 10 illustrated in Fig. 21 is an organic EL element (organic light-emitting diode: OLED). The organic EL element includes an anode 11, a receiving layer 19, a cathode 14, a sealing member 15, a barrier material 16, a circuit 17, and a power receiver 18. The receiving layer 19 includes an insulator 12 and a plurality of microdot light emitters 13 disposed two dimensionally. The multi-well unit 20 includes a cured layer 21 and a plurality of two dimensionally disposed microwell structure parts 22.

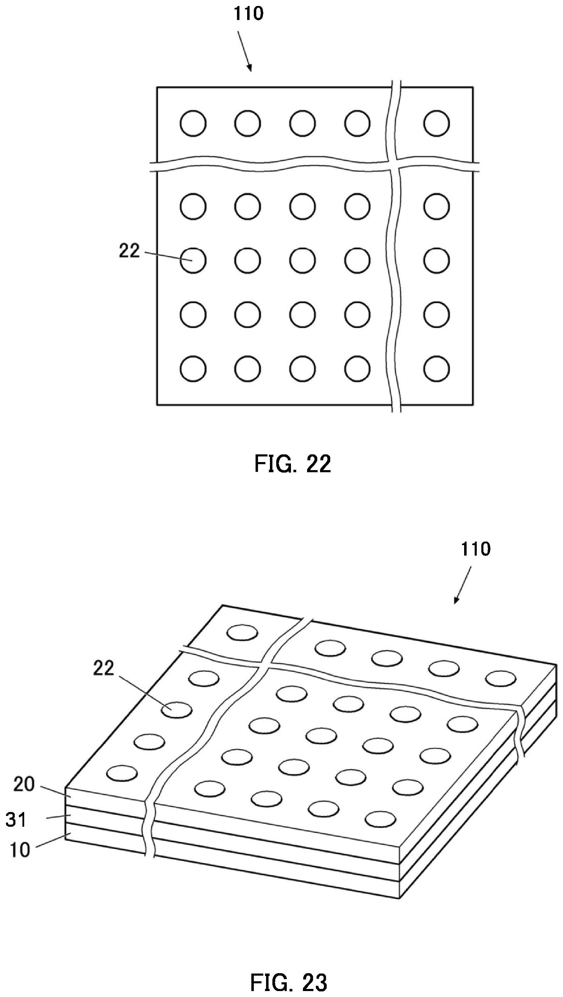

Fig. 22 is a plan view illustrating an example of the microarray device. Fig. 23 is a perspective view illustrating an example of the microarray device.

In the microarray device 110, an electromagnetic wave is emitted from the microdot light emitter 13 serving as a light source to the microwell structure part 22 serving as a reaction field. Using this electromagnetic wave as excitation light, a specimen in the microwell structure part 22 (or a contrast agent that may be present together with the specimen) exhibits light emission such as fluorescence and generates an electromagnetic wave. The electromagnetic wave generated from the microwell structure part 22 in this way becomes scientific data relating to the substance or object of the specimen.

Further, since the microarray device 110 includes a plurality of microwell structure parts 22 serving as reaction sites, a large amount of data can be generated at a time. Further, by providing a plurality of microdot light emitters 13 serving as light sources, the number of microdot light emitters 13 with respect to one microwell structure part 22 and the positional relationship between the microwell structure part 22 and the microdot light emitters 13 can be freely adjusted. Thus, the amount and the irradiation angle of an electromagnetic wave emitted to each microwell structure part 22 can be changed by the microwell structure part 22, so that various data can be generated by one inspection. Moreover, the microarray device 110 is easy to handle, and therefore a large amount of data can be generated quickly.

Some examples of the positional relationship between the in-plane coordinates of the microdot light emitters 13 and the in-plane coordinates of the microwell structure parts 22 will be described.

For example, the in-plane coordinates of a microdot light emitter 13 and the in-plane coordinates of a microwell structure part 22 can be in a positional relationship in which they at least partially overlap. With such a positional relationship, the absorption spectrum data and the light emission spectrum data of a specimen can be captured at once at the time of detection. Such a positional relationship can also reduce the optical path length, thus allowing the microwell structure parts 13 and the microdot light emitters 22 to be provided at a higher density.

Further, as illustrated in Fig. 21, the number of the microdot light emitters 13 and the number of the microwell structure parts 22 can be made to coincide with each other, and the in-plane central coordinates of each microdot light emitter 13 and the in-plane central coordinates of the corresponding microwell structure part 22 can be made to coincide with each other. With such a positional relationship, the excitation light emission efficiency is maximized, so that the analysis accuracy can be improved.

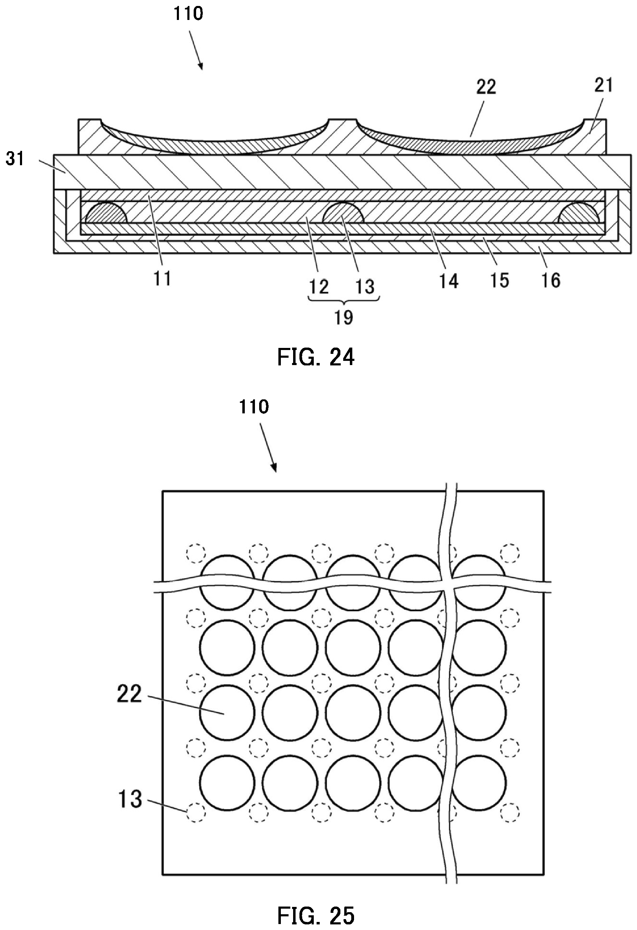

On the other hand, as illustrated in the cross-sectional view of Fig. 24 and the plan view of Fig. 25, the microdot light emitters 13 and the microwell structure parts 22 can also be in a positional relationship of being alternate with each other. Even in this case, electromagnetic waves emitted from the microdot light emitters 13 are emitted to the microwell structure parts 22. With such a positional relationship, it is possible to suppress direct detection of irradiation light from the microdot light emitter 13, which may cause noise. Such a positional relationship improves the signal/noise ratio, and thus is effective particularly when analysis targets (specimen) are with molecules of the same skeleton, and slight differences in structure or substituents are to be distinguished, or when the light emission signal intensity of an analysis target (specimen) is weak.

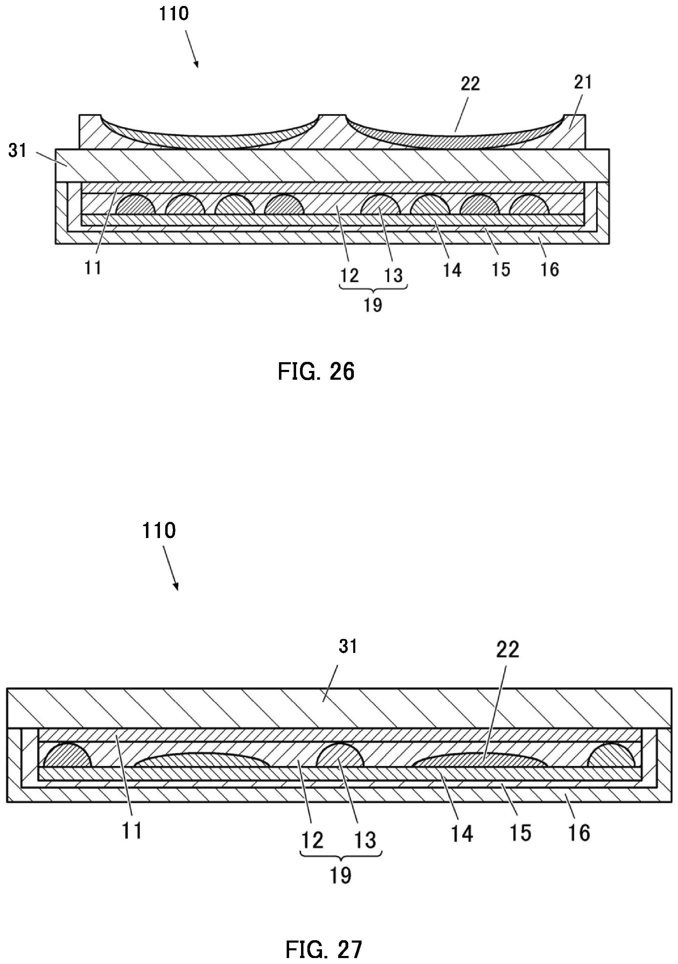

As illustrated in the cross-sectional view of Fig. 26, the number of microdot light emitters 13 may be larger than the number of microwell structure parts 22, and a plurality of microdot light emitters 13 may be located directly below each microwell structure part 22. With such a positional relationship, it is possible to irradiate one microwell structure part 22 with excitation light having different wavelengths without using a spectroscopic system. By irradiating one microwell structure part 22 with excitation light of different wavelengths, explanatory variables can be increased, and analysis accuracy can be improved.

Subsequently, details of the microarray device 110 illustrated in Fig. 21 will be described. Note that the microarray device 110 illustrated in Fig. 21 is of a type including an organic EL element as the light source unit 10, but the present invention is not limited thereto.

As the support to be applied to the organic EL element, a gas barrier film, electrodes, a receiving layer, av insulator and other preferred embodiments and the like, those described in known literatures can be used. For example, those described in Japanese Unexamined Patent Publication No. 2022-99677, Japanese Unexamined Patent Publication No. 2022-116901, WO2022/163199 can be utilized in the same manner.

### (Support)

As the support 31, a support that can transmit an electromagnetic wave emitted from the microdot light emitter 13 can be used. The support 31 is unnecessary in the case where the multi-well unit 20 is directly disposed on the light source unit 10. The support 31 is also unnecessary when the microdot light emitters 13 and the microwell structure parts 22 are formed in the same layer or in the same unit.

The support 31 may include a filter that converts the wavelength of electromagnetic waves from the microdot light emitter 13 or a filter that converts the luminescent color emitted from the microdot light emitter 13 into multiple colors with the use of a phosphor.

### (Light source unit)

A microarray device 110 of Fig. 21 includes a light source unit 10 that is an organic EL element. The light source unit 10, which is an organic EL element, includes an anode 11, a receiving layer 19, a cathode 14, a sealing member 15, a barrier material 16, a circuit 17, and a power receiver 18. The receiving layer 19 includes insulators 12 and microdot light emitters 13.

The thickness of the light source unit 10 may be, for example, about 0.2 µm.

### (Electrode)

As the anode 11, a metal having a large work function, an alloy, an electrically conductive compound, or a mixture thereof is preferably used as an electrode substance. As the cathode 14, a metal having a small work function (referred to as an electron-injecting metal), an alloy, an electroconductive compound, or a mixture thereof is preferably used as an electrode substance.

These electrodes (the anode 11 and the cathode 14) are obtained by, for example, forming an electrode substance into a thin film by a method such as vapor deposition or sputtering, or a method such as application and drying of a precursor solution. The electrodes (the anode 11 and the cathode 14) may be provided as flat films each having a uniform thickness, or may be provided in a desired pattern shape. In the case of a pattern shape, for example, the pattern shape can be formed by a photolithography method, or an inkjet method in the case of precursor solution coating. Alternatively, when high pattern accuracy is not required, the electrode can be formed by vapor deposition or sputtering of an electrode material using a mask having a desired shape.

In Fig. 21, the anode 11 is located on the microwell structure part side, but the cathode 14 may be located on the microwell structure part side. In either case, the electrode on the microwell structure part side is preferably transparent or translucent in order to transmit an electromagnetic wave emitted from the microdot light emitter 13.

### (Receiving layer)

The receiving layer 19 includes an insulator 12 and a plurality of two dimensionally disposed microdot light emitters 13. The receiving layer 19 is formed so as to be stacked on at least one of the electrodes (the anode 11 or the cathode 14). The receiving layer 19 is usually preferably formed as a continuous single layer.

The receiving layer 19 is formed such that the entirety thereof is in contact with one of the electrodes. The region where the receiving layer 19 is formed may be, for example, a region covering the entire surface of the electrode or a region covering a specific area of the electrode. Note that the insulator 12 is preferably made of at least one type of insulating polymer.

### (Microdot light emitter)

The plurality of microdot light emitters 13 are disposed two dimensionally.

Examples of the constituent material of the microdot light emitter 13 include a luminescent compound and a host compound.

The microdot light emitter 13 is preferably formed by an inkjet method. Thus, a large number of microdot light emitters 13 can be formed easily and quickly. In addition, the inkjet method is also suitable for forming a microdot light emitter 13 having an arbitrary shape or a minute size.

Regarding preferred embodiment and the like of the inkjet method, those described in Japanese Unexamined Patent Publication No. 2022-99677, Japanese Unexamined Patent Publication No. 2022-116901, WO2022/163199 can be utilized in the same manner.

The microdot light emitter 13 may be present in the form of a coffee ring.

### (Sealing member)

For example, a sealing adhesive can be used as the sealing member 15. Examples of the sealing adhesive include photocurable and heat-curable adhesives having reactive vinyl groups such as acrylic acid-based oligomers and methacrylic acid-based oligomers, moisture-curable adhesives such as 2-cyanoacrylic acid esters, heat-and chemical-curable (two liquid mixture) adhesives such as epoxy-based adhesives, hot-melt type polyamides, polyesters, polyolefins, and cation-curable type ultraviolet-curable epoxy resin adhesives.

The sealing adhesive is preferably an adhesive that can be cured at a temperature in the range from room temperature to 80°C, since the organic EL element may be deteriorated by heat treatment.

The sealing adhesive may contain a drying agent. The sealing adhesive may be applied with a dispenser or by printing such as screen printing.

### (Barrier material)

As the barrier material 16, a resin film or the like having a gas barrier film can be used. The gas barrier film is the same as that which can be included in the support described above.

### (Circuit)

The circuit 17 is provided to energize the anode 11 and the cathode 14 from the power receiver 18, and its configuration is not particularly limited.

### (Power receiver)

The power receiver 18 is not particularly limited, but one of the following can used: a power receiver that can receive power by near field communication (NFC, near field communication), a power receiver that can receive power from batteries, a power receiver that can be connected to a power feeding cable, and a combination of these. From the viewpoint of reducing the size of the microarray device, the power receiver 18 is preferably capable of receiving power by NFC.

### (Multi-well unit)

The multi-well unit 20 includes a cured layer 21 and a plurality of two dimensionally disposed microwell structure parts 22.

The thickness of the multi-well unit 20 may be, for example, 1 to 100 µm.

The multi-well unit 20 can be formed, for example, by applying the material of the cured layer 21 to regions other than the regions to become the microwell structure parts 22 on the support 31.

The application of the material of the cured layer 21 is not particularly limited, but is preferably performed by an inkjet method. Thus, the multi-well unit 20 and the microwell structure part 22 can be formed easily and quickly. In addition, the inkjet method is also suitable for forming a microwell structure part 22 having an arbitrary shape or a small size.

In the case of the inkjet method, for the inkjet apparatus and the like, application of the material of the cured layer 21 can be performed under the same conditions as those for the formation of, for example, the microdot light emitters 13.

The material of the cured layer 21 can be composed of, for example, an active ray polymerizable compound and a polymerization initiator. Furthermore, the insulating polymer that can constitute the receiving layer can also be used as the material of the cured layer 21, depending on the type of the specimen.

The "active ray polymerizable compound" refers to a compound which is crosslinked or polymerized by irradiation with actinic rays. Examples of the active rays include electron beams, ultraviolet rays, α-rays, γ-rays, and X-rays. Among the active rays, ultraviolet rays or electron beams are preferable. Examples of the active ray polymerizable compound include a cationically polymerizable compound, a radically polymerizable compound, and a mixture thereof. Among the active ray polymerizable compounds, a radically polymerizable compound is preferable. The active ray polymerizable compound may be any of a monomer, a polymerizable oligomer, a prepolymer, and a mixture thereof.

The radically polymerizable compound is a compound having an ethylenically unsaturated double bond group in its molecule. The radically polymerizable compound may be a monofunctional or polyfunctional compound. Examples of the radically polymerizable compound include various acrylates and methacrylates which are unsaturated carboxylic acid ester compounds.

The cationically polymerizable compound is a compound having a cationically polymerizable group in its molecule. Examples of the cationically polymerizable compound include an epoxy compound, a vinyl ether compound, and an oxetane compound.

The polymerization initiator may be any compound as long as it can initiate polymerization of the active ray polymerizable compound. For example, when the active ray curable ink contains a radically polymerizable compound, the polymerization initiator can be a photoradical initiator. When the active ray curable ink contains a cationically polymerizable compound, the polymerization initiator can be a photocationic initiator (photoacid generator). Note that when the active ray curable ink can be sufficiently cured without a polymerization initiator, such as when the active ray curable ink is cured by irradiation with an electron beam, the polymerization initiator is unnecessary. As the polymerization initiator, a known polymerization initiator can be used.

The material for the cured layer 21 applied onto the support 31 is cured by irradiation with active rays. The active rays to be applied can be selected from, for example, electron beams, ultraviolet rays, α rays, γ rays, and X-rays. Irradiation conditions such as irradiation time and illuminance can be appropriately selected according to the material and thickness of the cured layer 21.

At least one of the plurality of microwell structure parts 22 preferably contains a contrast agent on its surface. In this manner, the sensitivity of data acquisition can be improved. Further, by including a contrast agent on the surface, data to be generated can be multi-dimensionalized. Specifically, data to be generated is changed by interaction such as energy transfer between the specimen and the contrast agent, and thereofor, data on the molecular structure of the specimen, mixture amount ratio, density, environment, and the like can be generated in a more multidimensional manner.

It is also possible to use a plurality of types of contrast agents so that the microwell structure parts 22 contain different contrast agents. Thus, data on the specimen can be generated in a more multidimensional manner.

The application of a contrast agent to a microwell structure part 22 is preferably performed by, for example, dispersing or dissolving the contrast agent in a solvent to prepare a contrast agent-containing liquid, and then applying the contrast agent-containing liquid by an inkjet method. The application to the fine microwell structure parts 22 can be accurately performed by the inkjet method. The components of the contrast agent will be described below.

Hereinafter, preferred embodiments, modification examples, and the like other than the above-described embodiments will be described.

The light source unit, which is an organic EL element, may include an electron transport layer, a hole blocking layer, an electron injection layer, a hole transport layer, an electron blocking layer, a hole injection layer, and the like. In this case, the layer configuration including the electrodes and these layers can be, for example, as follows.
(I) Anode/receiving layer/cathode
(ii) Anode/acceptor layer/electron transport layer/cathode
(iii) Anode/hole transport layer/receiving layer/cathode
(iv) Anode/hole transport layer/receiving layer/electron transport layer/cathode
(v) Anode/hole transport layer/receiving layer/electron transport layer/electron injection layer/cathode
(vi) Anode/hole injection layer/hole transport layer/electron blocking layer/receiving layer/hole blocking layer/electron transport layer/cathode

For each of the layers such as the electron transport layer, those described in, for example, Japanese Unexamined Patent Publication No. 2022-99677, Japanese Unexamined Patent Publication No. 2022-116901, WO2022/163199 can be utilized in the same manner.

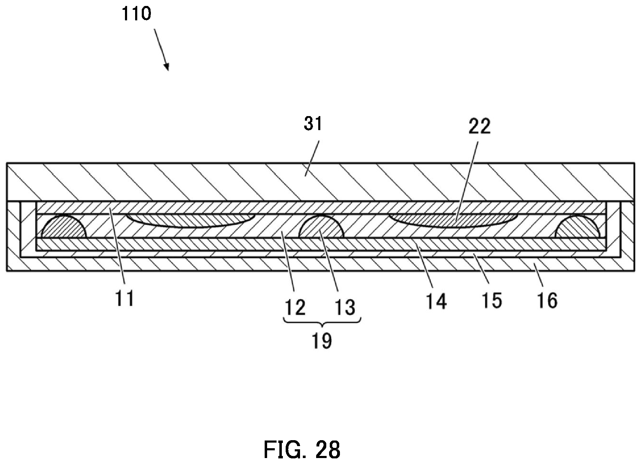

The microwell structure part 22 may be formed in the receiving layer 19, as illustrated in the cross-sectional views of Fig. 27 and Fig. 28. Such a microwell structure part 22 can be formed by a method of forming the microwell structure part at the same time as the formation of the receiving layer 19, a method of forming the microwell structure part by making holes in the support 31 and the electrode, a method of forming the microwell structure part using a porous material, or the like.

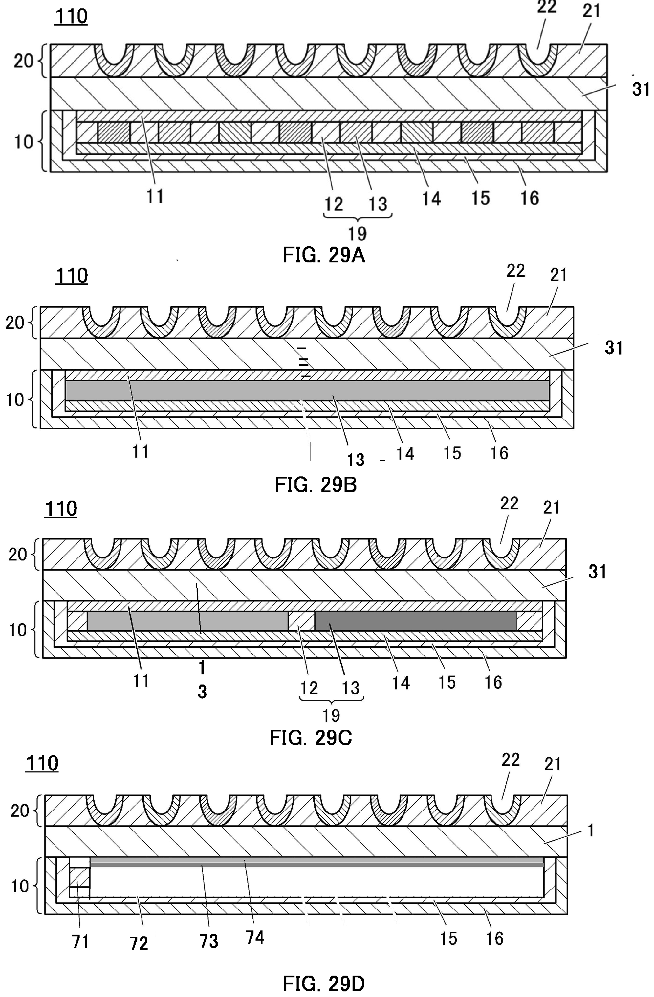

Microdot light emitter 13 is not limited to a light emitter of an organic EL element, and may be a light emitter of an inorganic light emitting diode or a plasma display. Fig. 29A is a cross-sectional view of a microarray device 110 in which the light source unit 10 is an inorganic light emitting diode.

The microdot light emitter 13 may emit near-infrared rays, infrared rays, or ultraviolet rays. For the microdot light emitter 13, PVK, phosphorescent material, anthracene, a Pt complex heat deactivator, a metal oxide, or a composite metal oxide can also be used.

The plurality of microdot light emitters 13 are preferably two or more types of microdot light emitters 13 having different maximum emission wavelengths. As a result, wavelength selectivity and explanatory variables are increased, and analysis accuracy is improved.

The plurality of microdot light emitters 13 preferably include at least two of the following: a microdot light emitters having a maximum emission wavelength of less than 380 nm, a microdot light emitter having a maximum emission wavelength of 380 nm or more and less than 500 nm, and a microdot light emitter having a maximum emission wavelength of 500 nm or more. The plurality of microdot light emitters 13 more preferably include at least all of these. Thus, the microarray device 110 can generate data as described below, thereby further improving the analysis accuracy.

An electromagnetic wave having a wavelength of less than 380 nm contributes to, for example, generation of data related to absorption between an excitation light source and a molecular structure. Examples of the electromagnetic wave in the wavelength range include ultraviolet rays having a wavelength of 10 nm or more and less than 380 nm. The ultraviolet rays include far ultraviolet rays having a wavelength of 10 nm or more and less than 200 nm and near ultraviolet rays having a wavelength of 200 nm or more and less than 380 nm.

An electromagnetic wave having a wavelength of 380 nm or more and less than 500 nm contributes less as an excitation light source to the generation of light emission data of, for example, a compound alone, but contribute to the generation of data in which interactions and data on excitations and absorptions of cleavage products of a conjugated compound are more emphasized.

An electromagnetic wave having a wavelength of 500 nm or more contributes to generation of, for example, data of absorption of a single compound and data corresponding to an aggregate or a decomposition product. Examples of the electromagnetic wave in the above range of wavelength include green to red visible rays having a maximum emission wavelength of 500 nm or more and less than 780 nm, and infrared rays having a maximum emission wavelength of 780 nm or more and less than 100 µm.

The plurality of microdot light emitters 13 may generate heat so that the temperature of the microwell structure part 22 can be controlled.

In the present invention, for example, one or a plurality of microdot light emitters can be provided for one microwell structure part. Thus, the size, shape, light emission wavelength, and the like of the microdot light emitter can be adjusted on demand so as to be compatible with the specimen and the contrast agent.

In the conventional microarray plate analysis, the wavelength of a light source, the light amount, temperature, and the like were not controlled for individual wells. The microarray device of the present invention can also control the wavelength of a light source, the light amount, temperature, and the like for individual wells. Thus, when the purpose is data acquisition for industrial use, food processing use, or the like, the activation energy can be made to exceed the activation energy of a specimen or a reagent to intentionally cause a chemical reaction or a biochemical reaction.

It is preferable that the arrangement concentration of the microwell structure parts 22 is five parts or more/cm². Thus, the microarray device 110 can be reduced in size. The arrangement concentration of the microwell structure parts 22 can be, for example, 5 to 10000 parts/cm².

The shape and position of a microwell structure part 22 can be freely changed according to the purposes such as structure/immobilization of a specimen, centrifugation, an optical path, a reaction path, acceleration of drying, and suppression of drying.

The shape of the microwell structure part 22 is not limited to a perfect circle, a square, a hemisphere, a square bottom, and the like. The shape can be designed to a shape for preventing drying of the specimen, support, degas, and the like, a flow path shape for acquiring reaction time series data or sieve data, or a shape suited to light irradiation/removal, and the like

The size of the microwell structure part 22 can be set to, for example, several tens of micrometers by inkjet resolution. A size larger than that can be freely designed with an input image.

The method for forming a microwell structure part 22 is not particularly limited, and the microwell structure part 22 can be formed by inkjet lamination of curable resin, 3D printer lamination of curable resin, laser processing of resin, photolithography of curable resin, or the like. The microwell structure part 22 can also be formed by a method in which a solvent is ejected to a resin by a inkjet method to dig a hole. Among these, inkjet lamination having all of fineness, on-demand properties, and waste material-free properties is preferable.

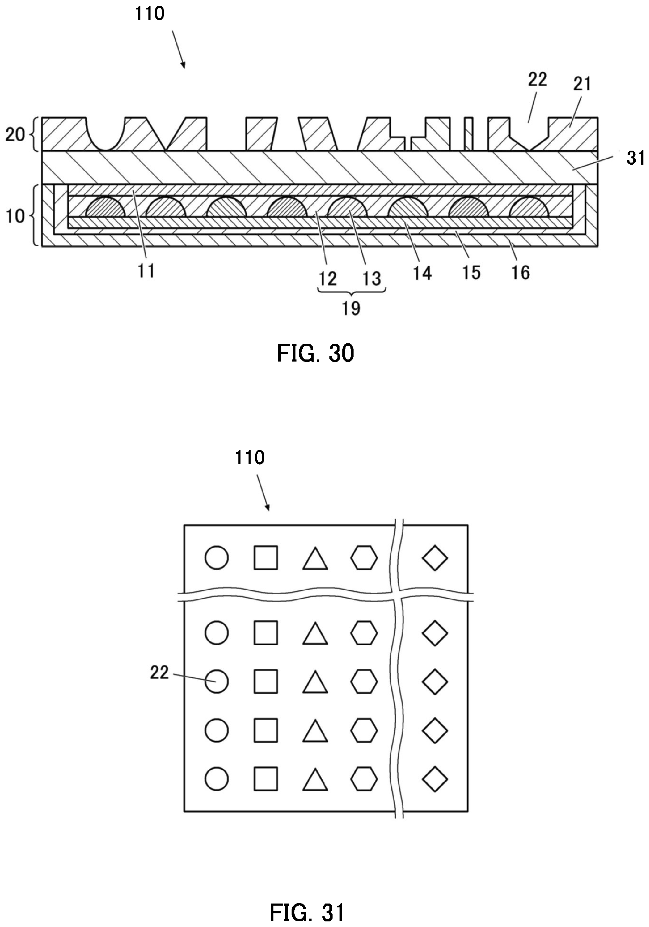

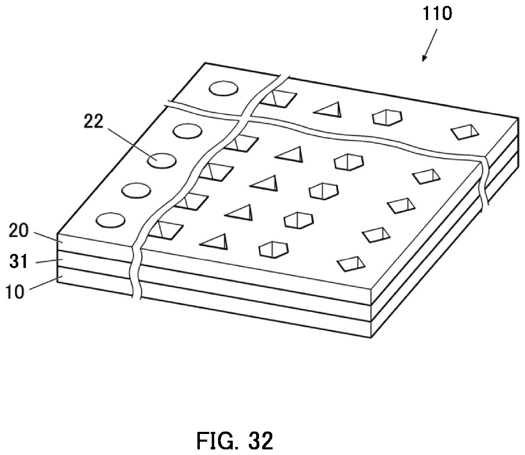

The shape of the microwell structure part 22 can also be different among the microwell structure parts 22, as illustrated in the cross-sectional view of Fig. 30, the plan view of Fig. 31, and the perspective view of Fig. 32.

By utilizing such an existing electronic display as a planar light source, at least the above-described 16 types of light sources for multi-well can be provided. However, since the light source is a light source for generating an electromagnetic wave from a dye or a phosphor serving as a sensor or a contrast agent, it does not require a long-term light emission life as in conventional electronic displays, and there is almost no demand for low power consumption.

In other words, among the electronic display methods known to date, even those that have not been put to practical use due to their lifespan or power consumption can be used for this purpose, and conversely, it goes without saying that a method that can easily form a point light source is preferable in this invention.

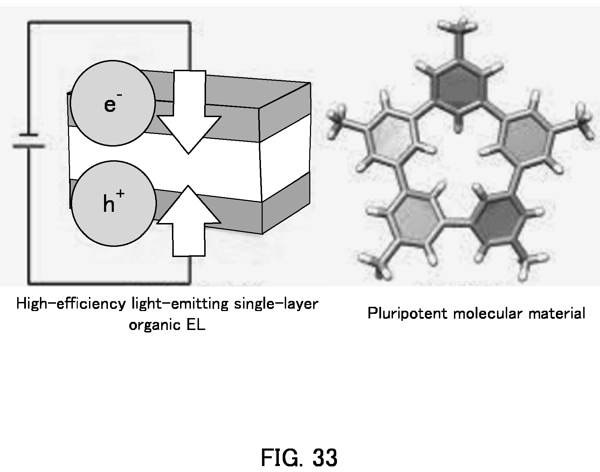

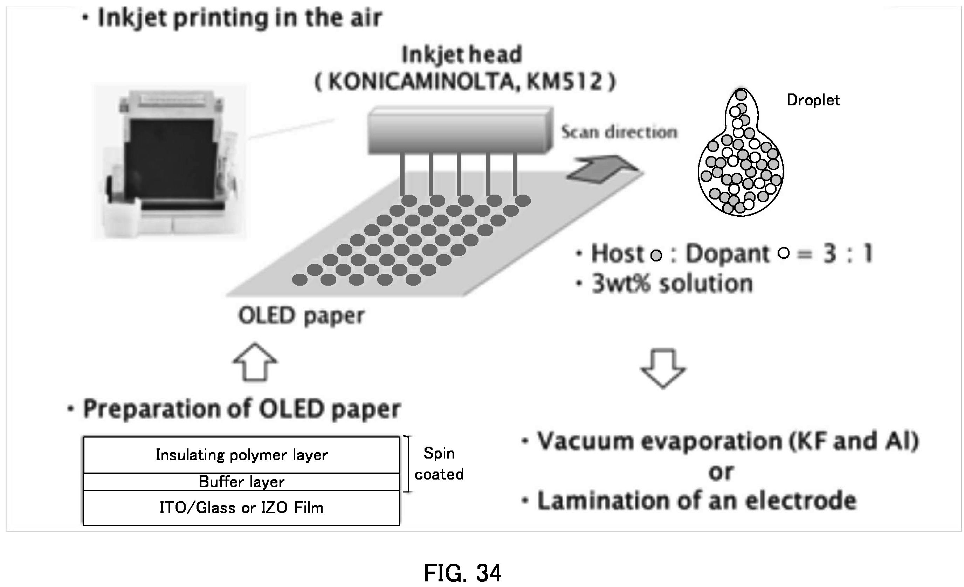

For example, such an example is illustrated in Figs. 33 and 34. An example thereof may be a sheet-shaped OLED which is produced by a highly simplified OLED element production method (for example SID Symposium Digest of Technical Papers, 52 (1),pp.297-300). The sheet-shaped OLED can accommodate either one dot or multiple dots depending on each well. Further, it is possible to simply and easily produce a point light source which emits light having a wavelength most advantageous for generating an electromagnetic wave from a color developer, a phosphor, a contrast agent and the like in each well on demand by using an inkjet system. Therefore, the above is the most preferable method for manufacturing a light source sheet in the present invention.

As a modification example of the above, the organic EL may be the light source unit 10 illustrated in Fig. 29A, and one light emitter 13 may be disposed for one microwell structure part 22 as illustrated in Fig. 29A.

As a modification example of the above, the organic EL may be the light source unit 10 illustrated in Fig. 26, and as illustrated in Fig. 26, a plurality of light emitters 13 may be disposed for one microwell structure part 22.

As a modification example of the above, when the light source unit 10 is an organic EL, one light emitter 13 may be disposed for all the microwell structure parts 22, as illustrated in Fig. 29B.

As a modification example of the above, when the light source unit 10 is an organic EL, one light emitter 13 may be disposed in one area formed by a plurality of microwell structure parts 22, as illustrated in Fig. 29C.

As a modification example of the above, the light source unit 10 may be a microarray device whose light source unit 110 includes a light emitter 71, a light guide plate 72, a color filter 73, and a diffuser plate 74, as illustrated in Fig. 29D. Also in this case, one light emitter may be disposed for one microwell structure part 22, or one light emitter may be disposed for a plurality of microwell structure parts 22. Alternatively, light emitters which emit light of the same color may be disposed over the entire surface, or light emitters which emit light of different colors for each area may be disposed in different areas.

As a modification example of the above, the light source unit 10 illustrated in Fig. 29A may be a plasma display or a micro LED. At this time, as illustrated in Fig. 29A, one light emitter 13 may be disposed for one microwell structure part 22, or one light emitter 13 may be disposed for a plurality of microwell structure parts 22. Furthermore, the light emitter 13 that emit light of the same color may be provided on the entire surface, or light emitters 13 which emit light of different colors for each area may be disposed in different areas.

### (3) Advantages of multi-well sheet incorporating light source

As described above, the present invention makes it possible to convert the wells that serve as reaction sites into ultra-multi-wells, and furthermore, it is possible to provide a light source corresponding to the multi-wells and to adjust, on demand, the size and shape of the light source and the emission wavelength suitable for the use and the specimen and the color developer/contrast agent in the wells. Further, the base material of the multi-well and the light source may be glass or plastic, and in either case, it is possible to prepare a multi-well sheet which is extremely thin and less than 1 mm and has built-in light source.

For example, the multi-well sheet can be used as an identification sheet for acquiring traceability for primary sector such as milk and fruit juices. In addition, the present invention can also be used to understand the status and record reactions in the wine brewing, soy sauce brewing, a manufacturing process of fake meat, and industrial product manufacturing such as paint manufacturing and ink manufacturing. In such a way of use, it is also a key point in utilization that archiving is easy. In particular, since a multi-well sheet having built-in light source requires no external light source and can acquire data, it is greatly advantageous in that it can be applied to almost all of primary sector and secondary sector because it is used in many scenes.

### (4) Data acquisition method

In the present invention, since means for acquiring a large amount of multidimensional data is color, light, or an electromagnetic wave, a spectrophotometer, a fluorescence clock, a photodetector corresponding to a wavelength, an electromagnetic wave sensor, or the like is used as a detection method.

In addition, as the simplest data acquisition method, it is simple and preferable to convert colors, light, or electromagnetic waves into electrical signals with a photoelectric conversion sensor such as a CCD or CMOS sensor in synchronization with the light emission from a sheet-shaped excitation light source.

The data may be a digital camera photograph of a smartphone, and since the data can be acquired from analysis of image data thereof, this type of use is thought to be suitable regardless of the situation in which it is used.

### (5) Remote data processing using cloud

The data converted into the electrical signals as described above or image data is generally stored in a data server or a data lake as it is, but a cloud server can also be utilized nowadays, and therefore, such utilization is also suitable. In particular, in the case where a site at which data is acquired is different from a place or time at which data is processed, remote data processing using a cloud or the like is effective.

### (6) Compatibility with digital rural urban national concept

Until now, digital manufacturing and its leading-edge tools, such as materials informatics and process informatics, have targeted industrial products, i.e., the secondary industry. As described above, in the future, it is necessary to apply this to primary industries such as food and agricultural products, as well as processing industries such as food and beverages that use these products, and to bring about digital transformation in all industries.

In particular, in order to realize the "digital rural urban national concept" proposed by the Japanese government in recent years, it is practically difficult to apply the conventional heavy, large-scale, advanced analysis apparatus that can accurately perform definitive diagnoses to local factories, agricultural processing plants, and the like. According to the present invention, by bringing a lightweight and ultrathin sheet-shaped multi-well to a site, it becomes possible to acquire useful data related to multidimensional and large amounts of material on the site, and then process the data remotely via the cloud. This is an important and innovative method which is consistent with the concept of comfortable and safe living of not only Japanese people but also human beings and SDGs represented by decarbonization, and is an excellent invention which can be developed in a wide range of applications.

The present invention relates to manufacturing DX intended for data-driven research and development and data-driven production. The present invention identifies the problems and bottlenecks specific to manufacturing in the inductive inverse problem solving method, which has traditionally been an excellent technique with some notable results. Further, the present invention relates to a method to achieve rapid and simple measurements below the molecular scale by combining chemistry and optics, thereby solving the conventional data acquisition rate constraints in one fell swoop, as well as an analysis method and an analysis apparatus required therefor.

### < Simple-OLED method of generating large amount of data >

A specific technical concept of a multi-dimensional, rapid, and massive data-generating method using a simple-OLED (organic EL element) will be described below with reference to the drawings.

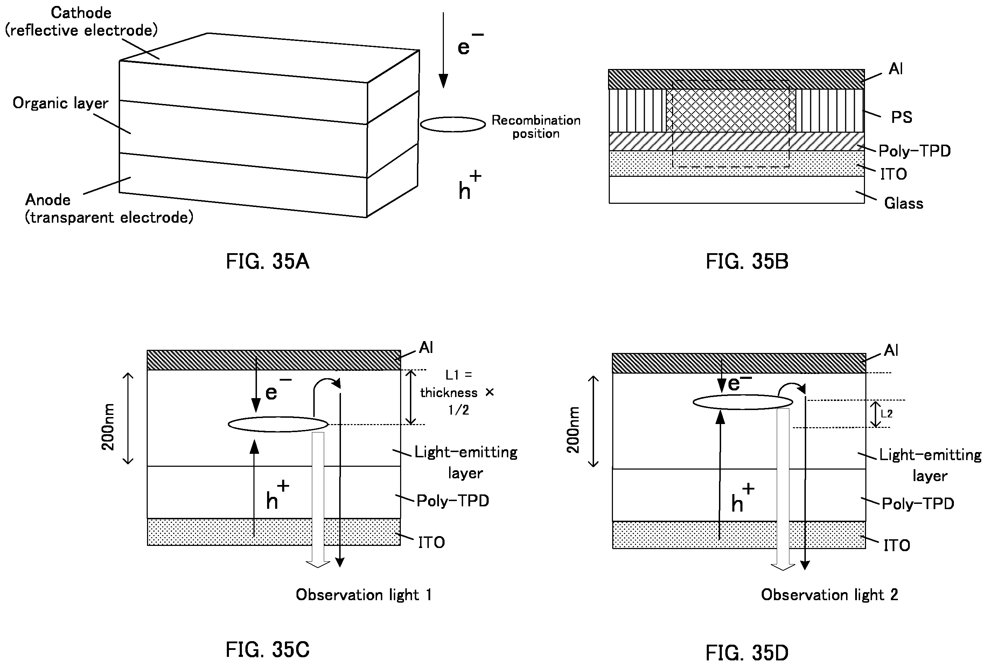

Fig. 35A schematically illustrates the appearance of a simple-OLED. As illustrated in Fig. 35A, in the organic EL element, specific carrier recombination is performed by both carriers of holes (h⁺) and electrons (e⁻). When a trace component of a specimen is mixed in such an organic EL element, the mobility of either holes or electrons is almost surely changed. Therefore, it is possible to produce a signal by utilizing optical interference of a light emission phenomenon caused by interaction (on the order of nm or Å in scale) between the trace component of the specimen and a substance in the organic EL element.

Figs. 35B to 35D illustrate a mechanism by which the state of the interactions is obtained as a signal. Fig. 35B is a schematic cross-sectional view of a simple-OLED, and Fig. 35C is a deformed schematic diagram of a portion (mainly, an organic layer portion) surrounded by a broken line in Fig. 35B. The organic layer (light-emitting layer) in Fig. 35C contains a binder, a dopant, and a host, and does not contain a specimen component. Furthermore, in the simple-OLED element, the types and ratios of the host and the dopant are optimized to some extent so that the host and the dopant are recombined substantially at the center of the organic layer. When the thickness of the organic layer is 200 nm, the vicinity of the 100 nm from the cathode, corresponding to the center of the thickness is the recombination center of the recombination position (light emission site).

Here, as illustrated in Fig. 35D, a trace component of the specimen is introduced. That is, the simple-OLED elements are formed using the method as illustrated in Fig. 15C or Fig. 16C. Thus, an interaction occurs between the specimen component and the host and/or dopant, or the polymer serving as the binder. Thus, the velocity of the flow of the holes or the electrons changes, and the position of the light emission site changes. The change is observed by the interference between the light emerging directly from the transparent anode and the light emerging after striking the reflective cathode, which is twice the optical path length out of phase (see Fig. 18). That is, the observation light is obtained as a signal describing the interaction caused by the specimen. Then, the difference between the observation light 1 that does not include the specimen illustrated in Fig. 35C and the observation light 2 that includes the specimen illustrated in Fig. 35D is the reason why the light generated from the simple-OLED becomes a signal that can describe interactions on the nm scale or the Å scale. In other word, it is a sensing mechanism.

In addition, since a large number of elements, which is tens of thousands of elements or more, can be formed on one substrate by using an inkjet method for element production, it is possible to rapidly acquire a large amount of data. Further, due to the characteristics of the inkjet method, the shape of the organic EL element can be freely designed from a circular shape to a triangular shape, a quadrangular shape, an irregular shape, or the like in accordance with the characteristics of the detection side. In addition, the following can be realized by using the sensor device, and the sensor device can be utilized as a sensor device of a future type which has not been seen before.
(I) When a host and a dopant are used in an ink liquid to be ejected from an inkjet head, the state of interaction with a trace component of a specimen is changed by changing the type of the host or the type of the dopant. Therefore, it is possible to increase the number of dimensions of data by forming elements in which a plurality of such elements are arranged.
(ii) In alignment with the organic layer of the two dimensionally arranged organic EL elements, for example, as illustrated in Fig. 16A to Fig. 16D, the specimen can be made into ink and overwritten on the organic layer. Furthermore, it is possible to add the amount of the component to each organic EL element in a manner similar to ultra-precise titration at the discharge resolution level (a few picoliters) of an inkjet head.
(iii) In addition to the specimen, a material capable of intentionally changing the carrier recombination position of the simple-OLED is formed into an ink, and by overwriting as in (ii), the change in light can be emphasized. In addition, since a regression curve can be intentionally created, unfavorable data such as an outlier can be deleted.
(iv) Two-dimensional drawing using inkjet printing makes it possible to easily create, for example, organic EL elements of 1000-dot × 1000 dot (= 1,000,000 dots). Therefore, it is not necessary to use all of the data as data for machine learning or deep learning, and it is possible to utilize more than half of the data for improving the quality of the data (for example, (ii) or (iii) is an example thereof, and it is also possible to statistically take an average value).
(v) An element that does not contain a specimen is used as a comparison element, and data is subtracted from that of an element that does contain a specimen, so that light-related data is always output as a "relative value" rather than being used as is. As a result, it is possible to cancel the performance variation of each organic EL element, which is a sensor, and the defect of the element itself.

This is not limited to the simple-OLED, and any elements that can be arrayed two dimensionally can be used in the same manner, and the same applies to the case where the DNA-type light emission sensors as described above are arrayed.

Although this discovery is completely novel and is recognized as an invention beyond fantasy that can be noticed only by those who understand the mechanism of light emission and quenching of organic EL, it cannot be denied that there may be the case where it is used as a sensor device in many papers and patents. However, since they are utilized as a sensor and have a gist essentially different from the present invention, they are considered to be distinguished from the present invention.

### < Specific embodiment >

Hereinafter, the present invention will be described in detail with reference to an embodiment. However, the present invention is not limited to these embodiment.

### (1) Analysis method

An analysis method of the present embodiment is a method for analyzing a state of a target. The method includes the following steps: mixing a target and at least one type of luminescent probe whose light emission behavior changes by interaction with the target, to interact with each other (hereinafter also referred to as "interaction step"); exciting the luminescent probes interacting with the target to produce two or more types of light emission and simultaneously detecting the two or more types of light emission (hereinafter also referred to as "light detection step"); and analyzing the two or more types of detected light emission to specify the state of the target (hereinafter also referred to as "analysis step"). The analysis method can detect nanometer-sized chemical structures and physical structures, and changes therein. The two or more types of light emission to be analyzed by the analysis method may be two or more types of light emitted by one luminescent probe, or may be two or more types of light respectively emitted by different types of luminescent probes.

In the analysis method of the present embodiment, since an indirect and simple means for detecting light is used, a complicated apparatus and a complicated step are not required for acquiring data. Furthermore, according to the method, since two or more types of light emitted from the luminescent probe(s) having interacted with the target are detected at the same time, a large amount of data on one target can be collectively acquired. Furthermore, by making full use of machine learning or deep learning at the time of analysis, it is possible to analyze a target in detail on the basis of a slight difference that cannot be recognized by a person.

Here, the target which can be analyzed by the analysis method of the present embodiment is preferably a target whose state changes due to a chemical change, a nanometer-size physical structural change, or the like. The target may be in any form capable of interacting with a luminescent probe, and may be in the form of a liquid, a solid, or a gas.

The target to be analyzed in the present embodiment may be a substance having a known structure or a substance having an unknown structure. Furthermore, the target may be a mixture of various compounds or the like. In addition, the field to which the target belongs is not particularly limited, and the target may be a substance, a compound, or a composition belonging to any field such as a medical field, an industrial field, a food field, or a water quality management field. Examples of the target belonging to the medical field include proteins, antibodies, antibody-attached beads, tumor markers, and the like. On the other hand, examples of the target belonging to the industrial field include metal nanoparticles, carbon nanotubes, magnetic fluid, nanosilica, and crystalline zirconia. Examples of the target belonging to the food field include agricultural products and processed products thereof. Examples of the target belonging to the water quality management field include water and microorganisms.

In addition, the analysis method may be performed, for example, for trial production, development, and research processes of an industrial product, or may be performed in a process of testing a manufacturing line of a product (in a test plant) or in a process of manufacturing a trial product (in a pilot plant). Furthermore, the analysis method may be performed in a production process of a product or a quality inspection process of a product. Furthermore, the analysis method may be performed for the purpose of a production process or a storage process of agricultural products or processed products thereof, a quality control process, or the like, or may be performed for water quality control or the like. In addition, the analysis method may be performed for various analyses in the medical field.

Here, the luminescent probe may be a compound whose light emission behavior is changed by interaction with a target. In the present specification, the expression "the light emission behavior of a luminescent probe is changed by the interaction with the target" means that the luminescent probe interacts with a specific group or a specific structure of the target, and thus the state of electrons before the interaction is changed (chemical interaction), or the steric structure is changed (physical interaction), thereby changing the light emission wavelength, light emission lifetime, light emission intensity, or the like. Such a luminescent probe may be an inorganic substance or an organic substance. In addition, the mechanism of light emission is also not limited, and for example, it may be a compound that emits light by irradiation with excitation light, or as described in a specific mode (second mode) which will be described below, it may be a compound that emits light by the movement of carriers by the application of a voltage, and the like. In addition, the luminescent probe may have a nucleic acid structure or the like, as shown in a specific embodiment (first mode) described below, in order to enhance the interactivity with a target.

Furthermore, in the interaction step, the method of causing the target and the luminescent probe to interact with each other is not particularly limited, and for example, they may be simply mixed, or energy such as heat may be applied to cause or promote the interaction.

In the light detection step, the method for exciting the luminescent probe in the state of interacting with the target to generate two or more types of light emission is appropriately selected depending on the type and number of the luminescent probe to be used, the light emission method of the luminescent probe, and the like. For example, by disposing different types of luminescent probes on a plane and causing the luminescent probes to emit light in a predetermined emission manner while causing the luminescent probes and the target to interact with each other, it is possible to detect light emission from each luminescent probe. At this time, disposing a plurality of different types of luminescent probes in a matrix on a plane facilitates recognition of light emission from each of the luminescent probes. In addition, the amount of data on the interaction between the target and the luminescent probe is increased by also detecting the light emission state of the luminescent probe before the interaction with the target.

Furthermore, in the light detection step, the method of simultaneously detecting two or more types of light emitted from the luminescent probe is not particularly limited, and the light may be detected by, for example, a spectrophotometer or a spectral radiance measurement device. Alternatively, an image may be captured by a CCD camera, a CMOS camera, or the like, and chromaticity, luminance, and the like may be specified from the image or video in analysis in the analysis step described below. Furthermore, in the light detection step, a temporal change in the light emission emitted by the luminescent probe may be detected continuously or intermittently. When the temporal change of light is acquired in this way, the amount of data on the interaction between the target and the luminescent probe increases.

In the analysis step, the method of analyzing the information on the light acquired in the light detection step is not particularly limited, and for example, for a target in an ideal state, standard data may be generated in advance, and the standard data and the data detected in the light detection step may be compared with each other to specify the state of the target. Furthermore, the information on the light acquired in the light detection step may be analyzed using a trained model or the like constructed in advance.

As a method of performing the analysis method of the present embodiment, the following two modes are given as specific example. However, the analysis method of the present embodiment is not limited to these methods.

### (1) First mode

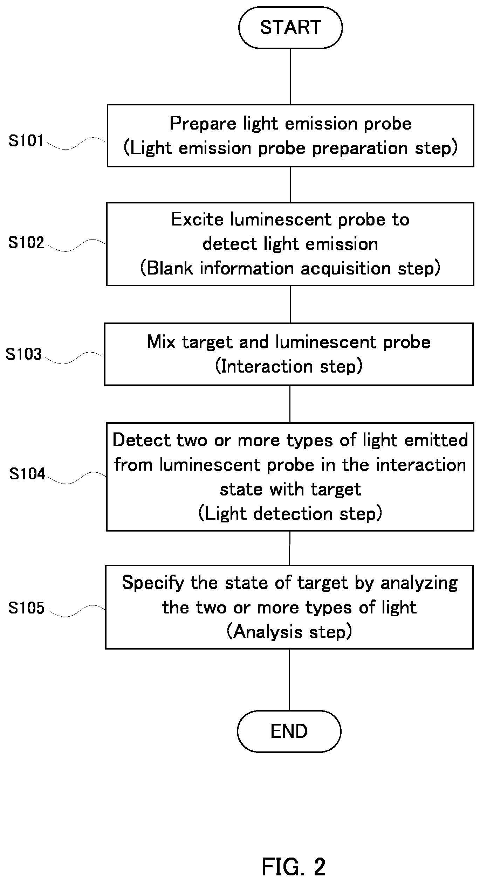

An analysis method according to a first mode is a method characterized by using a molecular luminescent probe including a light emitter that emits light emission when excited by irradiation with excitation light. A flow diagram of the analysis method of the present embodiment is illustrated in Fig. 2. In the present mode, at least the following steps are performed: preparing at least one type of luminescent probe that is excited to emit light by irradiation with excitation light and whose light emission behavior changes by interacting with a target (S101, the above-described luminescent probe preparation step); allowing the target to interact with the luminescent probes (S103, the above-described interaction step); simultaneously detecting two or more types of light emission emitted by the luminescent probes interacting with the target (S104, the above-described light detection step); and analyzing the two or more types of detected light emission to indirectly determine the state of the target (S105, the above-described analyzing step).

Note that the following description will be given taking as an example the case of performing the luminescent probe preparation step S101 and further performing, between the luminescent probe preparation step S101 and the interaction step S103, a step of exciting the luminescent probe in a state not interacting with the target to obtain the light emission information (S102, hereinafter also referred to as the "blank information acquisition step"). When the blank information step S102 is performed, it is possible to compare the information on the light detected in the light detection step S104 with the blank information acquired in the blank information acquisition step S102, and it is possible to further increase the amount of information on the target. However, the blank information acquisition step S102 is not necessarily performed. In addition, the method may further include a step other than the steps described above within a range in which the object and the effect of the mode are not impaired. Hereinafter, each step will be described.

### (Luminescent probe preparation step)

In the luminescent probe preparation step, at least one type of luminescent probe (compounds) which is excited by irradiation with excitation light to emit light and whose light emission behavior is changed by interaction with a target are prepared. The luminescent probe prepared in the present mode is a compound including one or more light emitters that are excited by irradiation with excitation light to emit light. The luminescent probe may include a plurality of the light emitters. Note that when a single luminescent probe (molecule) includes a plurality of light emitters, the plurality of light emitters may be of the same type or different types.

The light emitted from the luminescent probe excited by the irradiation with the excitation light may be light emitted from one light emitter in the luminescent probe or light emitted from a plurality of light emitters in the luminescent probe as a result of interaction between the light emitters. Examples of the light emitted by the luminescent probe include fluorescence, phosphorescence, excimer light emission, exciplex light emission, thermally activated delayed fluorescence, excited state intramolecular proton light emission, triplet triplet annihilation light emission, twisted intramolecular charge transfer light emission, and aggregation-induced light emission.

The size of each light emitter is preferably 100 nm or less, and more preferably 0. 1 nm or more and 100 nm or less. When the size of the light emitter is equal to or smaller than the 100 nm, it is easy to detect a change in the nanometer size of the target. The size of the light emitter can be specified by atomic-molecular scale structure modeling/visualization software (for example, Winmostar (developed by Crossability Inc)). Specifically, when the group that functions as the light emitter is regarded as an independent molecule, the length of a cylinder having the minimum diameter in which the molecule is inscribed can be obtained as the size of the structures.

In the case such as where a single luminescent probe emits a plurality of types of light emission, only a single type of luminescent probe may be prepared for a single target. Provided that it is preferable to prepare a plurality of types of luminescent probes for one target from the viewpoint of obtaining a large amount of data on the target. More specifically, it is preferable to prepare two or more types of luminescent probes for one target, and it is even more preferable to prepare four or more types of luminescent probes. At this time, the sizes of the light emitters of the plurality of luminescent probes are preferably different from each other. Light emitters having different sizes have different molecular structures, types of atoms, three dimensional structures, and the like. Therefore, by making a plurality of luminescent probes having light emitters different in size from each other interact with a target, a large amount of data can be obtained for the target.

Here, the luminescent probe may be a compound capable of producing a plurality of types of light emission from a single luminescent probe (hereinafter also referred to as "multiple luminescent compounds"), or a compound capable of producing a single light emission (hereinafter also referred to as "single luminescent compound"). These will be described in detail below.

### • Multiple luminescent compounds

### (I) Structure

The multiple luminescent compound may be any compound that is capable of emitting plurality of types of light in response to single excitation light. Examples of light emission emitted by a multiple luminescent compound include fluorescence, phosphorescence, excimer light emission, exciplex light emission, thermally activated delayed fluorescence, excited state intramolecular proton light emission, triplet triplet annihilation light emission, twisted intramolecular charge transfer light emission, and aggregation-induced light emission. Usually, two or more types of light emission selected from the group consisting of these are exhibited.

When the multiple luminescent compound are allowed to interact with a target, the structure and electronic state of the light emitter in the multiple luminescent compound are changed, so that complicated light emission behavior different from that in the case of the multiple luminescent compound alone is obtained. For example, a multiple luminescent compound that emits three different types of light emission, i.e., fluorescence, phosphorescence, and excimer light emission, in response to a single excitation light is caused to interact with a target. As a result, the processes in which fluorescence, phosphorescence, and excimer light emission are generated are changed by the interaction between the target and the multiple luminescent compound, and the wavelength and lifetimes of light are changed. Therefore, a large amount of complicated data in which these light beams are combined is obtained according to the structure, the state, and the like of the target. According to such a complicated and large amount of data, it is possible to understand the structure, the state, and the like of the target in very detail.

Examples of such a multiple luminescent compound include a compound having a nucleic acid structure and one or more light emitters (hereinafter, also referred to as a "chromophore" or a "luminophore") bonded to the main chain of the nucleic acid structure. Hereinafter, the compound will be described as an example. Note that in the present specification, examples of the nucleotide structures include not only structures derived from DNA or RNA, but also structures derived from one or more compounds selected from the group consisting of phosphorothioate oligodeoxynucleotides, 2'-O-(2-methoxy)ethyl-modified nucleic acids, siRNA, bridged nucleic acids, peptide nucleic acids, aTNA, SNA, GNA, LNA, and morpholino antisense nucleic acids.

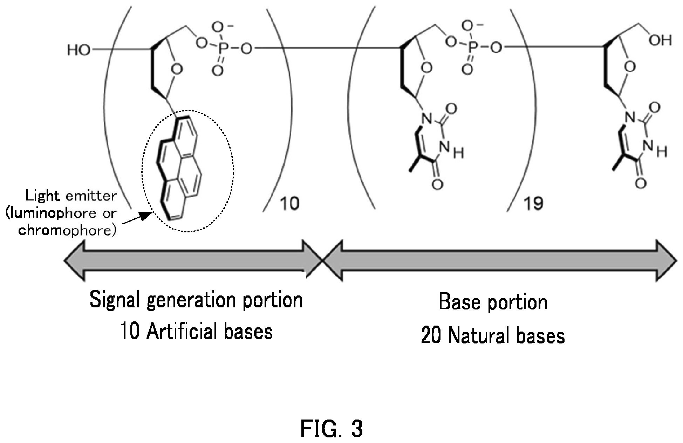

In the multiple luminescent compound, the light emitters may be uniformly bonded to the main chain of the nucleic acid structure. On the other hand, as illustrated in the schematic diagram of Fig. 3, the probe may have a region in which the light emitter is bound to the main chain of the nucleic acid structure and contributes to the light emission of the luminescent probe (hereinafter, also referred to as "signal generation portion"), and a region in which the light emitter is not bound to the main chain of the nucleic acid structure and does not contribute to the light emission of the luminescent probe (herein, also referred to as "base portion"). The signal generation portion is preferably disposed on the tip side of the multiple luminescent compound, that is, on the side which is easily brought into contact with the target.

Example of the multiple luminescent compound include structures (or molecules) having a main chain which has one or more constitutional units including a pentose-or hexose-derived sugar structure and a phosphate ester bond bonded to the sugar structure, and one or more chromophore or luminophore bonded to the sugar structure. In the signal generation portion of the multiple luminescent compound, 50% or more of the sugar structure to which a chromophore or a luminophore is bonded is preferably a β-form. In general, DNA has a structure in which bases are bound to a main chain (deoxyribose) including phosphate ester bonds and a deoxyribose-derived structure, but in natural DNA, all the deoxyribose in the main chain is in the β-form. Therefore, when 50% or more of the sugar structure to which the chromophore or luminophore is bonded is a β-form, it can be said that the sugar structure has high structural similarity to a substance (target) existing in nature, such as DNA or RNA. Such a multiple luminescent compound is less likely to cause steric hindrance or the like when mixed with a target, and can enter the inside or follow the shape of the target. Therefore, it is possible to analyze the target in more detail.

Furthermore, in the signal generation portion of the multiple luminescent compound, it is more preferable that 80% or more of the sugar structures to which the chromophore or luminophore is bound are in the β-form, and it is even more preferable that all of the sugar structures are in the β-form. Whether the sugar structure having the chromophore or the luminophore bonded thereto is a β-form or an α-form can be confirmed by NMR analysis, X-ray crystal structure analysis, or the like.

Hereinafter, specific structures of the signal generation portion of the multiple luminescent compound will be described.

The main chain of signal generation portion of the multiple luminescent compound having a pentose-or hexose-derived sugar structure may have at least one constitutional unit containing a pentose-or hexose-derived sugar structure and a phosphate ester bond bonded to the sugar structure. The main chain may include only one of the constitutional units, or may include a plurality of the constitutional units. The main chain may have one of the above sugar structures and one phosphate ester bond bonded to the sugar structure, or may have a structure containing the above sugar structures and phosphate ester bonds alternately. Usually, both ends of the main chain of the multiple luminescent compound have sugar structures, and therefore, the number of sugar structures is larger than the number of phosphate ester bonds by one. When the main chain includes a plurality of constitutional units, the plurality of constitutional units may be the same as or different from each other.

The number of the constitutional units contained in the main chain of the signal generation portion of the multiple luminescent compound is appropriately selected depending on the type of the target, and is preferably 2 or more and 6 or less. As the amount of the constitutional unit increases, the multiple luminescent compound tends to specifically act on a target. However, in the present mode, it is preferable to allow a multiple luminescent compound to interact with various locations of the target to obtain a large amount of data. Therefore, it is preferable that the multiple luminescent compound and the target have moderate (not excessive) specificity, and the number of the constitutional units is preferably 6 or less.

The main chain of the signal generation portion of the multiple luminescent compound may partially include a structure other than the sugar structure derived from pentose or hexose and a constitutional unit including a phosphate ester bond as long as the object and effects of the present mode are not impaired. The structures at both ends of the main chain are not particularly limited, and may be various structures such as an OH group or an alkoxy group.

Here, examples of the pentose include ribose, deoxyribose, and xylose. On the other hand, specific example of the hexose include allose, glucose, mannose and the like. Among these, the sugar structure is particularly preferably a structure derived from ribose or deoxyribose, in which case the main chain of the multiple luminescent compound has the same structure as the main chain of DNA or RNA and tends to interact with DNA or RNA.

When the constitutional unit contains a structure derived from ribose or deoxyribose, the phosphate ester bond is preferably bonded to the carbon at position 3 and the carbon at position 5 of ribose or deoxyribose. Furthermore, the below-described chromophore or luminophore is preferably bonded to the 1-position of ribose or deoxyribose. That is, the multiple luminescent compound of the present mode preferably contains a structure represented by the following general formula (1a) or (1b).

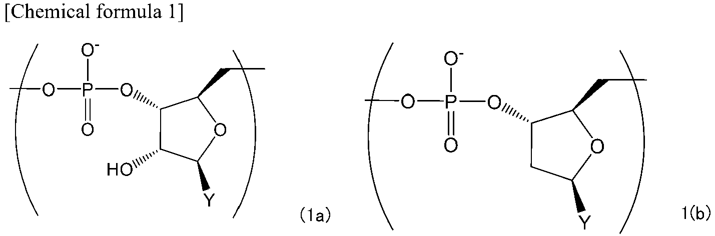

In the general formulae (1a) and (1b), Y represents a chromophore or a luminophore which will be described below.

The light emitter (chromophore or luminophore) in the signal generation portion of the multiple luminescent compound may have a structure that emits a predetermined type of light by itself in response to a single excitation light, or that emits a predetermined light by the action of plurality of chromophores or luminophores. The chromophore or the luminophore is preferably bonded to the sugar structure of the main chain so that the sugar structure is a β-form. Note that in the present specification, the term "chromophore" refers to a structure that absorbs light with a wavelength of 300 nm or more, and the term "luminophore" refers to a structure that absorbs light with a wavelength of 300 nm or more and emits light.

The number of the chromophores or the luminophores included in the signal generation portion of the above-described multiple luminescent compound may be only one, as long as the multiple luminescent compound can exhibit plurality of types of light emission. However, the number is preferably 2 or more, and more preferably 3 or more and 6 or less, from the viewpoint that the multiple luminescent compound easily exhibit plural types of light emission. When the signal generation portion of the multiple luminescent compound has plurality of chromophores or luminescent groups, the types thereof may be only one type, or may be two or more types. Here, in the multiple luminescent compound, usually, one chromophore or one luminophore is bonded to one sugar structure in the main chain. Therefore, when the multiple luminescent compound has two or more plurality of chromophores or luminophores, it is preferable that the sugar structure in the main chain is also two or more. That is, the number of chromophores or luminophores in the multiple luminescent compound is preferably equal to or smaller than the number of sugar structures (or peptide structures) in the main chain of the signal generation portion.

Note that when the number of chromophores or luminophores of the signal generation portion in the multiple luminescent compound is smaller than the number of sugar structures (or peptide structures) in the main chain, some of the sugar structures are in a state where no chromophore or luminophore is bonded thereto. The sugar structure to which no chromophore or luminophore is bound may have no other atomic groups bound thereto. Provided that a naturally occurring nucleic acid base may be bonded thereto to the extent that the object and the effect of the present mode are not impaired. As used herein, natural nucleic acid base refer to adenine, guanine, cytosine, thymine, and uracil. However, the total number of natural nucleic acid bases bonded to the main chain is preferably 50% or less, and more preferably 25% or less, of the total number of constitutional units constituting the main chain structure of the region (signal generation portion) mainly responsible for the interaction in the multiple luminescent compound. An explanation will be given taking as an example the multiple luminescent compound which is illustrated in Fig. 3 and which has a signal generation portion (a portion interacting with a target) and a base portion (a portion hardly contributing to the interaction) and in which the signal generation portion is constituted of the first to the tenth sugar chains from the tip. In this case, the number of natural nucleic acid bases bound to the sugar chain is preferably 50% or less, and more preferably 25% or less, of the total number of sugar structures contained in the sugar chain, in the sugar chain up to the 10th from the tip (signal generation portion). When the number of the natural nucleic acid base in the signal generating element is 50% or less, the association of the multiple luminescent compounds with each other is suppressed, and the interaction between the target and the multiple luminescent compound is more likely to become dominant. Provided that when the target has a large molecular size such as a protein and a liposome, the signal generation portion may continue to the tenth and subsequent sugar chains from the tip. At this time, those having a structure in which a large number of natural bases are linked to the tenth and subsequent sugar chains from the tip are also preferable from the viewpoint of the overall affinity. In this case, the number of natural bases to be bound to the signal generation portions is preferably about 10 to 50 bases in length, and more preferably 15 to 30 bases. Further, in the signal generation portion, the natural nucleic acid base and the non-natural nucleic acid base are preferably bound such that the sugar structure is a β-form as described above. Note that the structure of the base portion is not limited to the above, and various structures may be employed.

Here, examples of the chromophore or luminophore that emits fluorescence include structures derived from fluorescein, rhodamine, boron dipyrromethene, and the like. Examples of the chromophore or luminophore that emits phosphorescent include structures derived from iridium complexes, platinum complexes, and the like. Examples of the chromophore or luminophore that emits excimer light include structures derived from pyrene, anthracene, perylene, and the like. Examples of the chromophore or luminophore that emits exciplex light emission include structures derived from yrene-dimethylaniline and the like. Examples of the chromophore or luminophore that emits thermally activated delayed fluorescence include structures derived from 4CzIPN, DABNA, and the like. Examples of the chromophore or luminophore that emit excited state intramolecular proton light include structures derived from hydroxyphenylbenzoxazole and the like. Examples of chromophores or luminophores that emit triplet triplet annihilation light include structures derived from 9,10-diphenylanthracene, rubrene, and the like. Examples of the chromophores or luminophores that emit twisted intramolecular charge transfer light include structures derived from diaminoanthracene, diaminonaphthalene, and the like. Examples of the chromophores or luminophores that emit aggregation-induced light include structures derived from tetraphenylethene, hexaphenylsilole and the like.

Furthermore, compounds used as a light-emitting material or host, an electron transport material, a hole transport material, and a light-emitting material of an organic EL can also be suitably used as a material of the chromophore or the luminophore. Specific example of such compounds include compounds described in "Leading-edge Organic EL" (CMC Publishing Co., Ltd), Organic EL Material Technology (CMC Publishing Co., Ltd), All of Organic EL (Nihon Kohyo Publishing Co., Ltd), Various Future Dye Materials (Kagaku Dojin) and the like.

The reason why various compounds for organic EL are suitable for the chromophore or the luminophore of the signal generation portion of the multiple luminescent compound is as follows. For example, the electron transport material is a substance containing an electron-accepting aromatic compound that tends to become an anion radical, and thus strongly interacts with an electron-rich compound in the target. Conversely, a hole transport material is a substance containing an electron-donating aromatic compound that easily becomes a cation radical, and thus strongly interacts with an electron-deficient compound in a target. Since light-emitting materials of an organic EL have both of the properties and is a substance having a high emission quantum yield, a strong emission signal is obtained. Phosphorescent materials and delayed fluorescent materials can also be used. These emit light with a time delay of about nanoseconds to microseconds relative to conventional fluorescent light emission, and thus can include a time factor in the number of dimensions as a sensing material and can be multi-dimensionalized as data for use in machine learning, deep learning, or the like, which is suitable.

Typical examples of the organic EL material are described below. These are molecular groups that can be Y in the above-described general formulas (1a) and (1b). Furthermore, it may become Y via a linking group or the like.

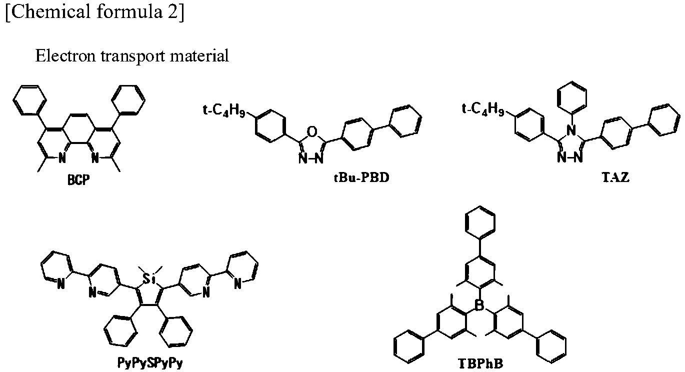

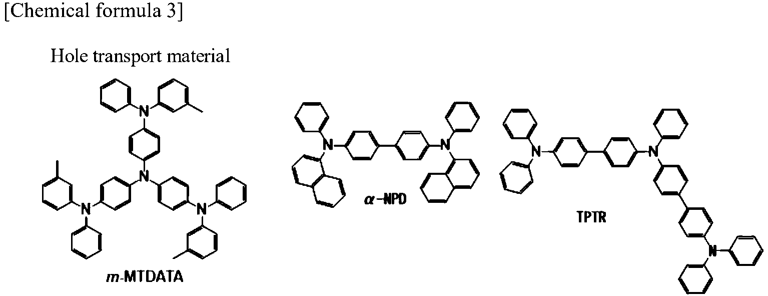

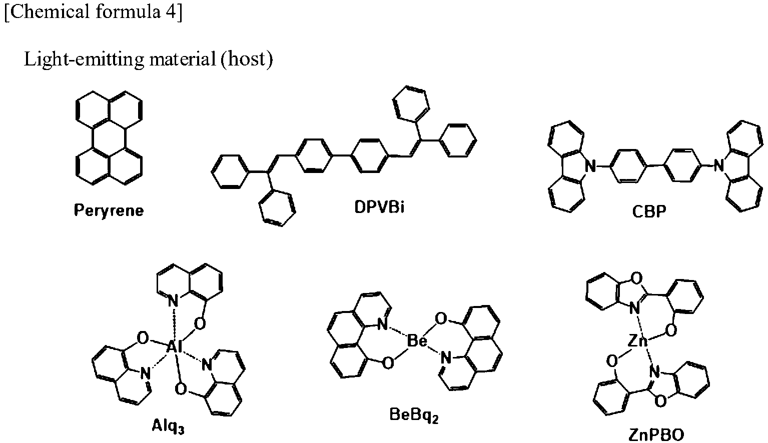

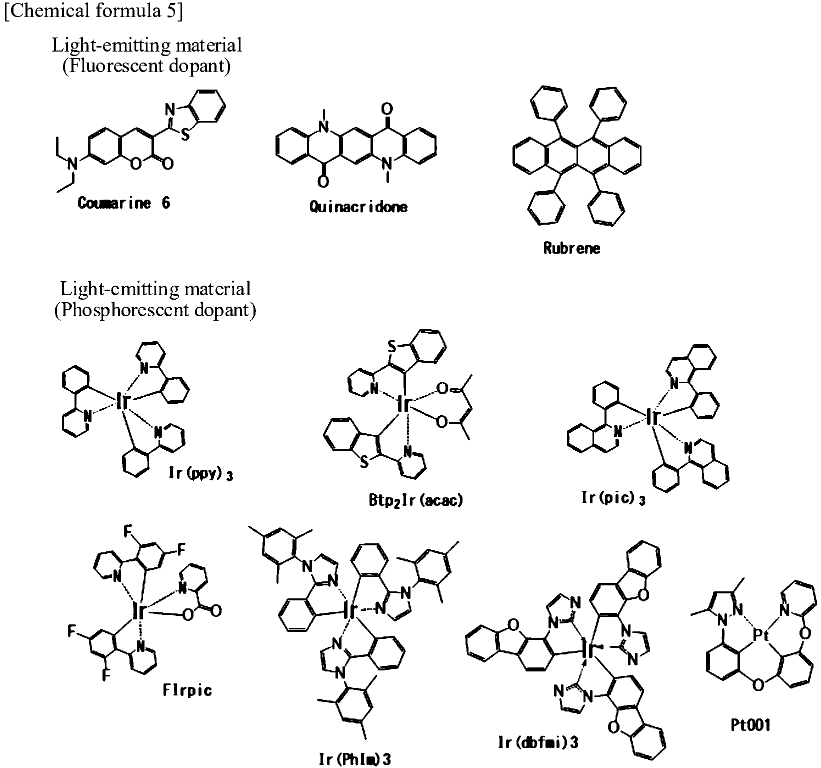

Examples of chromophores or luminophores other than those described above include the following.

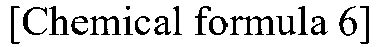

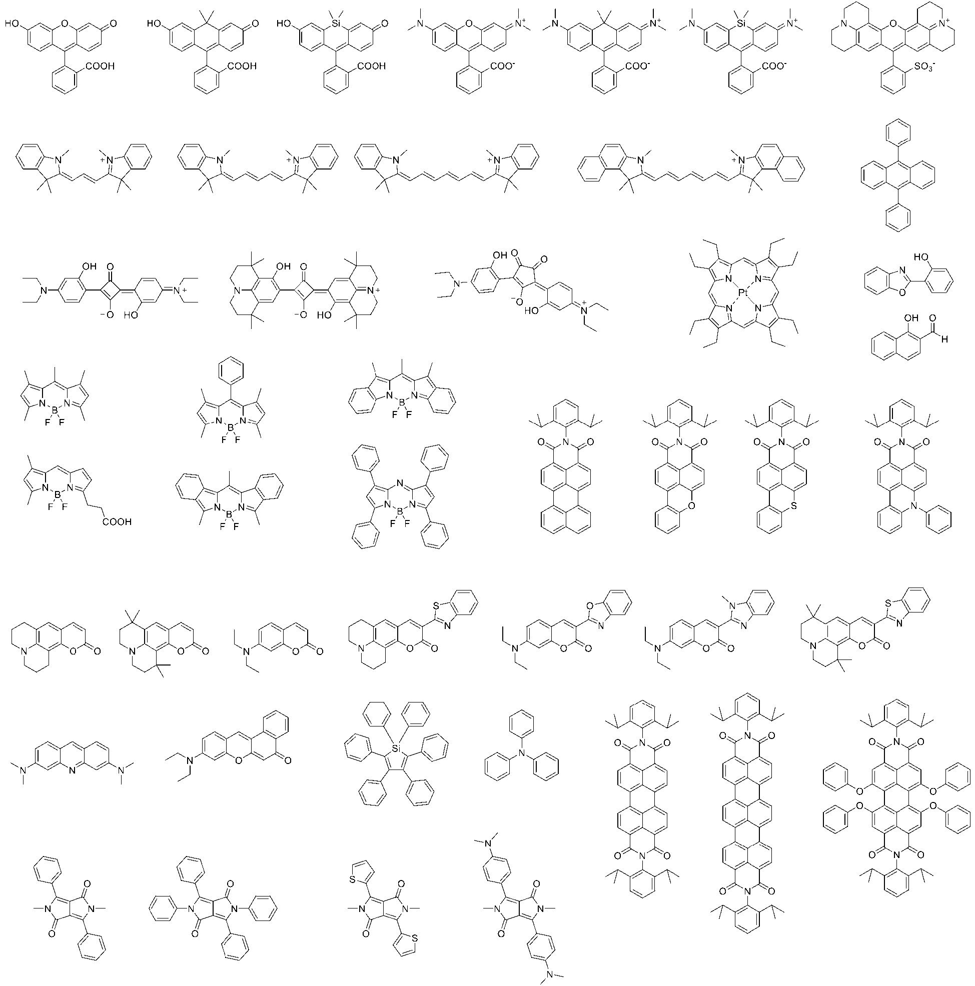

Furthermore, in the present mode, a structure derived from a non-light-emitting monomer may be included in the multiple luminescent compound as a site for controlling the interaction between the multiple luminescent compound and a target. For example, a structure derived from a non-light-emitting monomer can coexist with the structure derived from a multiple luminescent compound in the molecular chain. Representative function and examples of monomer structures for realizing the functions are shown below.

### A1) Compound which captures a metal ion

Typical examples thereof include those having a ligand structure forming a chelate, and examples of the form of coordination includes N, N coordination, N, O coordination, O, O coordination, N, S coordination, O, S coordination, S, S coordination, N, Se coordination, O, Se coordination, S, Se coordination, Se, Se coordination and the like. Specific example thereof include 2,2'-bipyridine, phenanthrolines, 1,8-diaminonaphthalene, amino acids, cryptands, crown ethers, 8-hydroxyquinoline, 3-mercaptopropanol, 3-mercaptopropionic acid, thiocatechol, salicylaldehyde, acetoacetic ester, β-diketones and the like.

### A2) Compound whose molecular shape changes depending on wavelength of light to be emitted

Typical examples include azobenzene, stilbene, fulgide, diarylethene, spiropyran, spirooxazine, dihydropyrene, phenoxyquinone, biindenylidene dione, cobalt complexes, imidazole dimers, and the like.

### A3) Compound acting as Lewis-acid

Typical examples include Group 13 elements and transition metals, and specific example include triarylborane, trialkylborane, trialkoxyborane, trifluoroborane, trichloroborane, and tribromoborane.

### A4) Compound acting as Lewis-base

Typical examples include Groups 15 and 16 elements and the like, and specific example include arylamine, alkylamine, arylphosphine, alkylphosphine, aryl ether, alkylether, arylsulfide, alkylsulfide and the like.

### A5) Compound that interacts with peptide or protein

Typical examples include nucleic acid bases, transition metal complexes, oxo acids and the like, and specific example include adenine, thymine, guanine, thymine, cytosine, zinc complexes, copper complexes, nickel complexes, cobalt complexes, tungstic acid, molybdic acid, phosphoric acid and the like.

### A6) Compound forming hydrophobic-hydrophobic interaction

Typical examples include straight chain alkanes, straight chain alkenes, straight chain alkynes, branched alkanes, branched alkenes, branched alkynes, aromatic rings, heteroaromatic rings and the like.

### A7) Compound forming dipole-dipole or quadrupole-quadrupole interaction

Typical examples include alkyl halides, aryl halides, nitriles, nitroarenes, anilines, anisole, heterocyclic compounds, and the like.

Note that in the present mode, it is preferable that the multiple luminescent compound includes, as a chromophore or a luminophore, at least one structure selected from a structure that emits fluorescence, a structure that emits excimer light, and a structure that emits exciplex light. Above all, it is preferable to include at least a structure that emits fluorescence. In the case where the multiple luminescent compound emits fluorescence, there is an advantage that it is easy to analyze by various measuring devices.

Furthermore, it is preferable that the multiple luminescent compound exhibits plurality of types of light emission by irradiation with light having a wavelength of 300 to 400 nm. When the multiple luminescent compound exhibits plurality of types of light emission by irradiation with the light having the wavelength, a special light source is unnecessary and the target is less likely to be damaged in analyzing the target.

Provided that when LEDs or organic EL element are used as the excitation light sources, it is advantageous to excite in the visible light region, and therefore, in such a case, 400 to 700 nm is effective as the absorbing wavelengths of the above-described multiple luminescent compound, and such dyes can also be used.

The molecular weight of the signal generation portion (in the case of having the signal generation portion and the base portion, the molecular weight of the signal generation portion) is appropriately selected in accordance with the type of the chromophore or the luminophore possessed by the multiple luminescent compound, the length of the main chain and the like. Usually, the molecular weight is preferably 500 or more and 10000 or less, more preferably 500 or more and 4000 or less. When the molecular weight of the multiple luminescent compound is 10,000 or less, specificity for a target becomes moderately low, and it becomes possible to cause the multiple luminescent compound to non-specifically react with plurality of parts of the target.

Note that in the above description, the constitutional units constituting the main chain of the nucleic acid structure of the signal generation portion include constitutional units each containing a sugar structure derived from pentose or hexose and a phosphate ester bond, such as DNA and RNA, but the constitutional units are not limited thereto, as described above. Typical examples of other constitutional units include peptide-nucleic acid type constitutional units as described below.

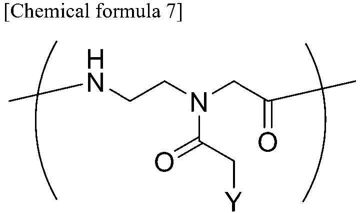

Like DNA/RNA, peptide nucleic acid can be synthesized exhaustively by using a commercially available automatic synthesizer (peptide synthesizer). Since the peptide nucleic acid has no electric charge and has no electrostatic repulsion, the peptide nucleic acid can form a stronger association with a target. Furthermore, since the peptide nucleic acid is resistant to enzymes such as nuclease and protease, it can be used for cells. Furthermore, relatively large-scale synthesis is possible. Provided that due to the nonionic structure, the peptide nucleic acid aggregate in water and the solubility decreases in some cases. Therefore, when the signal generation portion has a structure derived from a peptide nucleic acid, it is preferable to appropriately select the type of the base portion linked to the signal generation portion or to select the solvent.

### (ii) Production method

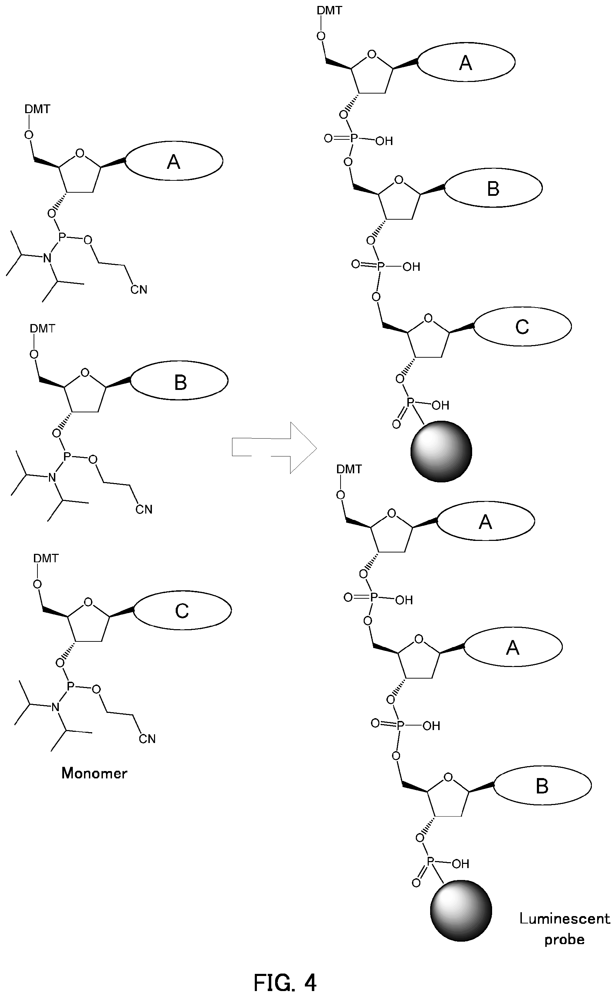

The method for producing the multiple luminescent compound is appropriately selected depending on the type of the nucleic acid structure in the multiple luminescent compound. For example, the multiple luminescent compound having a sugar structure described above can be produced by the following method. A monomer in which the chromophore or the luminophore and the phosphate ester are bonded to pentose or hexose is prepared. The monomer can be synthesized by polymerizing the monomer in a desired sequence using a phosphoramidite method with a DNA/RNA synthesizer or the like. According to such a method, for example, as illustrated in the schematic diagram of Fig. 4, a plurality of types of monomers (three types in Fig. 4) having different types of chromophores or luminophores (represented by A, B and C in Fig. 4) are prepared, and a desired number of the monomers can be bonded (three monomers can be bonded in Fig. 4) by changing the arrangement order of the monomers. That is, a wide variety of multiple luminescent compounds can be synthesized from the plurality of types of monomers having different types of chromophores or luminophores. In the example illustrated in Fig. 4, 27 type of multiple luminescent compounds can be synthesized. By changing the type of the monomer to be used and the number of bonds of the monomer, a very large number of types of multiple luminescent compound s can be synthesized.

When the phosphoramidite method is performed, a part of a monomer, for example, a hydroxyl group of a sugar structure (for example, a hydroxyl group bonded to the carbon atom at the 3-position of ribose or deoxyribose) or a hydroxy group derived from phosphoric acid is usually supported on a particulate carrier (in the present specification, also referred to as "carrier particle") to perform a polymerization reaction. The carrier particles are, for example, porous glass, porous silica gel, polystyrene or the like, and the porous glass includes a porous body of a metal oxide such as silica gel or alumina. After the synthesis of the multiple luminescent compound (polymerization of monomers), the carrier may be removed to acquire only the multiple luminescent compound, but the multiple luminescent compound may be used in a state of being supported on carrier particles. Furthermore, the above-described multiple luminescent compound and a liquid may be mixed to obtain an ink.

### (iii) Effect

As described above, the multiple luminescent compound has a main chain structure similar to that of a substance existing in nature (for example, DNA or RNA). Therefore, it is possible to easily interact with various targets, and according to the multiple luminescent compound, it is possible to understand the state of the target in detail. The above-described multiple luminescent compound emits plurality of types of light by irradiation with light having a specific wavelength. Therefore, it is possible to obtain a large amount of complex light emission data depending on the state of the target, and to analyze the target in great detail.

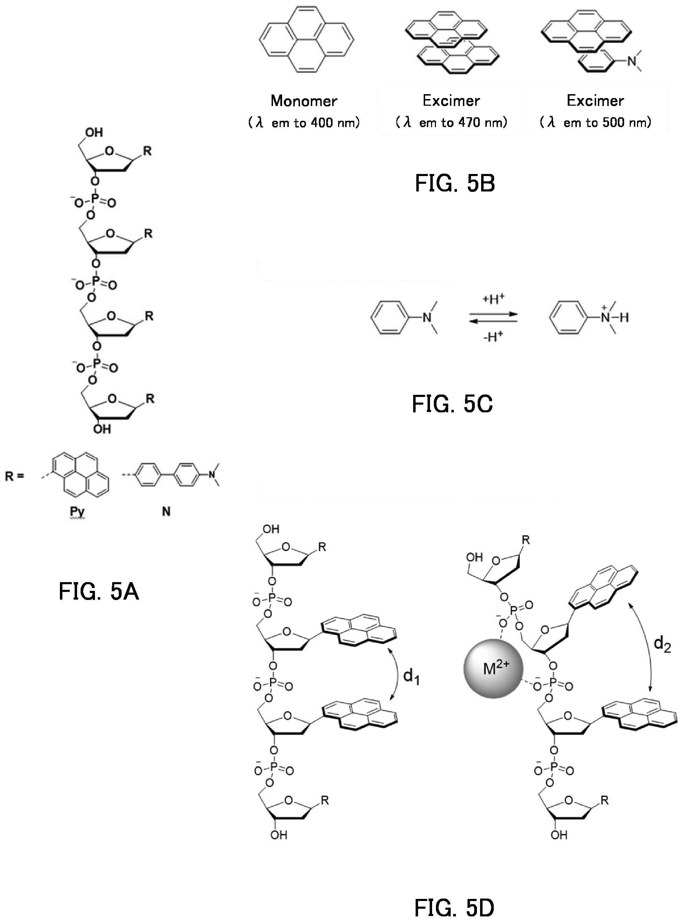

The mechanism of exhibiting the effect is schematically illustrated in Figs. 5A to 5D. First, for the purpose of facilitating understanding, the multiple luminescent compound is a molecule having four sugar structures and four luminophores as illustrated in Fig. 5A. All of the four R's may be pyrene (Py in Fig. 5), dimethylaminobiphenyl (N in Fig. 5), or a mixture thereof. One or two of the four R's may be a hydrogen atom. By such a molecular structure, a form of a composite type multiple luminescent compound can be constructed.

The present mode is characterized in that a liquid substance, a dispersion substance, or a gaseous substance as a target causes a complex interaction with a multiple luminescent compound or the multiple luminescent compound with the carrier thereof, and a large amount of multidimensional data is generated as light or color signals. Fig. 5B to Fig. 5D show the concept most simply and concretely, and there are three main aims envisaged.

For example, the case where all R are pyrene is assumed, when a specimen is brought into contact with a liquid or a medium in which the multiple luminescent compound is present and excitation occurs by ultraviolet light. When a target (for example, an aliphatic compound or the like) which is contained in the specimen and inserted between pyrene molecules present in the multiple luminescent compound and has a wide band gap which does not quench fluorescence of pyrene is present, monomer light emission of pyrene illustrated in the left drawing of Fig. 5B is observed from the multiple luminescent compound.

When no component that interacts with pyrene is contained in the specimen, light emission of pyrene is obtained as illustrated in the center diagram of Fig. 5B. In addition, when a target (fluorescent substance) having an energy level close to the band gap of pyrene that interacts with pyrene is present in the specimen, light emission due to pyrene and the target (the fluorescent substance) is obtained as in the right diagram of Fig. 5B.

Furthermore, in the case where a target (metal ion) such as a sodium ion or a calcium ion is present in the specimen, the metal ion forms a chelate with a phosphate group present in the main chain portion of the multiple luminescent compound. At this time, as illustrated in Fig. 5D, since the intermolecular distance between pyrene and pyrene changes depending on the size of the metal ion, the excimer light emission itself also changes in luminescent color (light emission spectrum).

In addition, in the case where R in the structure illustrated in Fig. 5A is dimethylaminobiphenyl (N), a change in the luminescent color (spectrum) of the fluorescent dye (N) occurs due to the proximity of an acid and a base as illustrated in Fig. 5C. This is different from acid-base ion pairing, such as switching on/off like mineral acids (such as sulfuric acid and nitric acid) and alkali metals. In this case, the distance of approach to N continuously changes depending on the acidity (ease of donation of protons) of a substance contained in the specimen. Therefore, using this light emission phenomenon as a signal leads to expansion of the dynamic range. Since N is a Lewis base, the same interaction occurs with Lewis acidic substances (for example, triarylborane, trialkylaluminum, tetraalkoxytitanium, and the like) in addition to protic acidic substances. Therefore, even when such a target is contained in the specimen, a specific emission color change occurs.

Next, in the case where R of the multiple luminescent compound is pyrene (Py) and dimethylaminobiphenyl (N) alternately, unlike the above case, when there is no interaction between the specimen and pyrene or dimethylaminobiphenyl, the exciplex light emission of pyrene and dimethylaminobiphenyl is observed. On the other hand, in the case of interaction, similarly to the above, complicated light emission due to a mixed exciplex of the specimen component and pyrene and/or dimethylaminobiphenyl is obtained.

Furthermore, when one or two of the four R's on the inner side are a hydrogen atom, excimer light emission or exciplex light emission does not occur from the luminescent properties dye molecule itself, or its contribution is small, and almost only monomer light emission is observed, but since the steric hindrance of the hydrogen atom is small, the interaction between the target in the specimen and the R of the luminophore is enhanced, and the change in the light emission signal is enhanced.

In the case where Py or N in Fig. 5A is not a general fluorescent substance but a phosphorescent compound or a thermally excited delayed fluorescent compound, light emission is emitted at a timing delayed by several tens of nanoseconds to several microseconds from the timing immediately after the excitation. Therefore, the factor of "time" rather than the luminescent color expands the dynamic range, and such a phenomenon can also be applied to the present invention in addition to the mechanism as illustrated in Figs. 5B to 5D.

Such an intermolecular interaction and a resultant subtle change in luminescent color or light emission spectrum, a delay of light emission of several microseconds, or the like, and furthermore, an extremely subtle change in a light emission phenomenon brought about by metal chelate formation due to a main chain structure have not been able to be applied as analysis information for human understanding. However, on the premise that artificial intelligence (AI) that has become generally available in recent years and machine learning and informatics utilizing the AI are used, such a wide variety of light emission phenomena beyond the understanding of human beings are state description data corresponding to a target specimen. Such a new concept is a fundamental concept of the present invention and is extremely useful as a new method of state description for a complicated and uncertain specimen in future various research and development and production processes, furthermore, cell culture, waste liquid/waste water/sludge treatment, and the like.

### • Single luminescent compound

On the other hand, the single luminescent compound is a compound that emits single light in response to single excitation light, and is a compound whose light-emitting state is changed by interaction with a target. Examples of the single luminescent compound include materials that form a hydrogen bond with a target and materials that performs π-π interactions with a target. The single luminescent compound may be any one of inorganic substances, organic substances, metallic complexes, and the like. Examples of the inorganic substances that can be used as the single luminescent compound include YAG phosphors, quantum-dot phosphors, thermochromic materials such as VO₂, and Ag nanoparticles. Examples of organic substances that can be used as the single luminescent compound include fluorescent materials such as coumarin and perylene. In addition, examples of the metal complexes that can be used as the single luminescent compounds include phosphorescent materials such as Eu (europium) complexes and Ir (iridium) complexes.

In the present mode, by combining two or more of such single compounds, two or more types of light emitted by the luminescent probe can be acquired in the light detection step described below.

### (Blank information acquisition step)

In the blank information acquisition step of the analysis method according to the present mode, the luminescent probe prepared in the luminescent probe preparation step is excited, and information on its light emission (blank information) is acquired. When a plurality of luminescent probes are used, blank information may be acquired for each luminescent probe, but it is preferable to acquire blank information for a plurality of luminescent probes at the same time from the viewpoint of efficiency and the like. For example, a plate including a large number of containers for allowing a target and a luminescent probe to interact with each other may be prepared, and a plurality of types of luminescent probes may be individually disposed in the respective containers. Use of the plate makes it possible to apply excitation light to a plurality of luminescent probes at the same time and to detect light emitted from the luminescent probes at the same time. Furthermore, when the plate is used, in the interaction step to be described below, a target can be further placed in the place (container) where each luminescent probe has been placed in the present step, and thus the luminescent probe and the target can be easily made to interact with each other.

The plurality of containers of the plate are preferably disposed at intervals. The plate may have a flat plate shape or may have unevenness in accordance with a shape of the containers. Furthermore, the material, size, shape, and the like of the plate are appropriately selected according to the application of analysis, the types of the luminescent probe and the target, and the like.

The interval between the plurality of containers is appropriately selected according to the size of the containers, the types of the luminescent probe and the target, and the like. Note that in the case where the luminescent probe or the target is dropped onto the plate by an inkjet device or the like, the plate may have no mark (formation of an uneven structure or marking) or the like indicating the position of each container. On the other hand, when a mark (formation of an uneven structure or marking) indicating the position of each container is formed on the plate, it is easy to accurately dispose the luminescent probe or the target at a desired position (container).

In addition, when each container is formed in a recessed shape or a partition wall part is disposed around each container, the luminescent probes or the targets disposed in adjacent containers are less likely to be mixed, and more accurate analysis is easily performed. In addition, for example, even in the case where the water repellent treatment portion is disposed around the container, the luminescent probes and the targets in the adjacent containers are less likely to be mixed, and thus it is easy to perform more accurate analysis. In the present mode, a plate in which a plurality of wells are regularly disposed is used. In a plate having such wells, the wells (containers) are physically separated from each other by the partition walls, and thus the luminescent probes and the like in adjacent containers are less likely to be mixed with each other, making it easier to perform accurate analysis.

Here, the number of the containers included in one plate is appropriately selected according to the type of the luminescent probe, the target, and the like. The number of containers is not particularly limited, but as the larger the number is, the larger the amount of multidimensional data can be acquired, and more precise analysis can be performed.

In addition, the method for disposing the luminescent probe in each container is not particularly limited, and is appropriately selected according to the type, physical property, and the like of the luminescent probe. Examples of the method for disposing the luminescent probe include application by an inkjet device, application by a dispenser, disposing of a carrier for carrying the luminescent probe, and direct fixation of the luminescent probe to the container. Among these, the inkjet method is particularly preferable. According to the inkjet method, it is possible to efficiently dispose each of the liquid luminescent probes in a large number of regions (containers) to form the containers. This makes it possible to acquire a large amount of data.

In the present mode, the different types of luminescent probes are respectively disposed in the different containers, but a plurality of types of luminescent probes may be disposed in one container.

Furthermore, there are no particular limitations on the method for acquiring the blank information from a plate having luminescent probes placed in the containers. For example, specific excitation light (excitation light of a single wavelength) may be irradiated, and the intensity and wavelength (light emission information) of the light emitted by each luminescent probe may be measured using a spectrophotometer or the like, and this may be used as the blank information. In addition, when specific excitation light is irradiated, a change over time in the spectral distribution of light emission by the luminescent probe or a change in chromaticity over time may be continuously or intermittently measured by a spectrophotometer or the like, and this may be acquired as blank information.

Furthermore, in the case of acquiring a chromaticity change of light emitted by the luminescent probe, excitation light having a single wavelength may be applied only for a short time, and the light emitted by the luminescent probe upon receipt of the excitation light may be acquired as an image with a CCD camera, a CMOS camera, or the like. Data on a change in chromaticity over time or the like may be acquired by determining chromaticity or the like from the obtained image.

The wavelength of excitation light is appropriately selected depending on the types of the luminescent probe and the target, and the excitation light can be, for example, light having a wavelength of 300 nm or more and 700 nm or less. Such light is less likely to affect the target. Furthermore, a special light source or the like is not required.

### (Interaction step)

In the interaction step, a target and a luminescent probe are mixed and allowed to interact with each other. In the case where a plate including a plurality of containers is used in the above-described blank information acquisition step, a target may be disposed in each container (container accommodating a luminescent probe) of the plate of which the above-described blank information has been acquired. Note that a target may be disposed also in the container where the above-described luminescent probe is not placed. Furthermore, when the above-described plate has multiple containers, different targets may be disposed in some of them, and multiple targets may be analyzed on one plate. On the other hand, the same object may be disposed in all the containers.

The method for disposing the target is not particularly limited, and is appropriately selected depending on the type of the target and the property. The method may be the same as the method for disposing the luminescent probe described above. Furthermore, the method of allowing the target and the luminescent probe to interact with each other is not particularly limited, and the target and the luminescent probe may be simply mixed, but may be heated or subjected to other treatments as necessary.

### (Light emission information acquisition step)

In the light emission information step, in a state where the target and the luminescent probe are allowed to interact with each other, the excitation light is applied, and two or more types of light emitted by the luminescent probe are simultaneously detected. The method for simultaneously detecting two or more types of light is not particularly limited, and for example, specific excitation light (excitation light having a single wavelength) may be irradiated, and the intensity or wavelength (luminescence information) of light emitted by each luminescent probe may be detected by a spectrophotometer or the like. Further, when specific excitation light is irradiated, a change over time in the spectral distribution of the light emission by the luminescent probe or a change in chromaticity over time may be continuously or intermittently detected by a spectrophotometer or the like.

Furthermore, in the case of acquiring a chromaticity change of light emitted by the luminescent probe, excitation light having a single wavelength may be applied only for a short time, and the light emitted by the luminescent probe upon receipt of the excitation light may be acquired as an image with a CCD camera, a CMOS camera, or the like. By specifying the image and the like from the obtained image, data on the change in chromaticity over time and the like can be obtained.

### (Analysis step)

The analysis method in the present step is appropriately selected depending on the purpose, the type of information on light emission acquired in the light detection step, and the like. For example, the data detected in the light detection step may be used as analysis data as it is. On the other hand, a difference between the blank information acquired in the above-described blank information acquisition step and the information on the light emission acquired in the light detection step may be used as the analysis data. In the present step, for example, standard data is prepared in advance for a target in an ideal state, and the state of the target can be specified by comparing the standard data with the analysis data. According to the method, even in the case where the target is composed of a plurality of components, the case where a plurality of parameters are involved (e.g., quality and palatability of food), or the like, the state of the target can be specified without individually analyzing the components of the target by creating standard data in advance for the case where the target is in a good state and the case where the target is in a bad state.

Here, when the analysis is performed, the standard data and the analysis data may be simply compared with each other. In addition, the comparison result between the standard data and the analysis data may be converted into a distance matrix and analyzed using a heat map (without weighting). Furthermore, the distance matrix may be subjected to principal component analysis (also referred to as PCA, weighting with emphasis on anisotropy), analysis by DL (weighting with emphasis on isotropy), or the like.

On the other hand, the analysis data may be analyzed by a trained model constructed in advance or the like. The trained model can be created by, for example, a machine learning step to be described below, but the trained model to be used is not limited to one created in the machine learning step to be described below. Using the trained model, more appropriate analysis can be performed on the target.

When referring to the trained model, the above-described analysis data is applied to the trained model. Thus, whether the target has a desired structure, how much of a predetermined structure the target includes, whether the target is in a satisfactory state, and the like can be determined (predicted) from the accumulated data and the like. Note that the prediction result may be obtained as, for example, classification, regression, clustering, or abnormality detection (outlier detection).

### (Machine learning step)

The analysis method according to the present embodiment may further include a learning step of performing machine learning on the analysis data to generate a trained model.

For example, in the machine learning step, a plurality of prediction models may be constructed on the basis of the difference between the analysis data and the blank information described above. Then, by combining results of the plurality of prediction models, a trained model capable of predicting information (for example, a structure, an amount, or the like) regarding the target is created.

In the case where the structure or the amount of the target is known in advance, the prediction model can be constructed by performing machine learning in which the feature of the analysis data is set as an explanatory variable and the structure or the amount of the target is set as an objective variable. As the explanatory variables, numerical values representing the features of the above-described analysis data and numerical values calculated from the numerical values can be used. When the blank information or the light emission information acquired in the light detection step is spectral distribution, intensity of light at each wavelength or the like can be adopted as an explanatory variable. On the other hand, the objective variable can be appropriately selected according to the purpose of analysis and is not limited to the structure or amount of the target, but any other variable related to the target may be used.

The machine learning performed in the present step may be supervised learning or may be unsupervised learning. Note that supervised learning refers to a learning method of learning a "relationship between an input and an output" from training data with a ground truth label. Unsupervised learning is a learning method of learning a "structure of a data group" from training data without a ground truth label.

Alternatively, the machine learning may be reinforcement learning, deep learning, or deep reinforcement learning. Note that reinforcement learning refers to a learning method of learning an "optimal action sequence" by trial and error. Deep learning refers to a learning method of learning, from a large amount of data, features included in the data step by step more deeply (in deeper layers). The deep reinforcement learning refers to a learning method in which reinforcement learning and deep learning are combined.

A general analysis method (algorithm) can be applied to machine learning. For machine learning, for example, a prediction model constructed by an analysis method selected from linear regression (multiple regression analysis, partial least squares (PLS) regression, LASSO regression, Ridge regression, main component regression (PCR), and the like), random forest, decision tree, support vector machine (SVM), support vector regression (SVR), neural network, discriminant analysis, and the like can be applied.

### (Modification example)

In the above, it has been explained that a plate including a plurality of containers for allowing the targets and luminescent probes to interact with each other is used, and the targets and luminescent probes are disposed in the containers to perform analysis. However, in place of the luminescent probe, the above-described luminescent probe carried on a carrier, or an ink in which the luminescent probe is dissolved or dispersed in a liquid may be used. Furthermore, a fluorescence measurement cell or the like may be used in place of the plate. In this case, the blank information acquisition step and the light detection step may be performed with a known fluorometer. Furthermore, for example, when the ink is used, a desired base material (e.g., paper) may be used in place of the plate.

### • Analysis apparatus

The above-described analysis method can be performed by an analysis apparatus that includes a holder (plate) including a plurality of containers for storing a target and the above-described luminescent probe(s), an exciter (light source) for exciting the light emitter disposed in the container, a detector for detecting two or more types of light emitted by the luminescent probe(s), and an analyzer for analyzing the detected light.

Note that the analysis apparatus of the present embodiment may further have a configuration other than the holder, the exciter, the detection means, and the analyzer. For example, an inkjet printing section or the like may be further provided. Note that the plate is the same as that described in the analysis method, and a detailed description thereof will be omitted.

On the other hand, the exciter can be, for example, an excitation light source for emitting excitation light, examples of which include a supercontinuum light source (a broadband pulsed light source that utilizes the nonlinear effect of optical fibers to emit strong, phase-coherent light over a very wide wavelength range, also known as an "SC light source") and an LED. According to these light sources, since the light amount can be increased, light emission information is more likely to become clear.

The detector is configured to simultaneously detect two or more types of light emitted by a luminescent probe, and the type of the detector is appropriately selected according to information on the two types of light detected by the detector. For example, the detector may be a spectrophotometer, an ultraviolet-visible light absorption meter, or the like, or may be a fluorescent fingerprint measurement device. Furthermore, the detector may be a circular dichroism spectrometer, a high-performance liquid chromatograph (HPLC), or the like. A CCD camera, a CMOS camera, or the like may be used. Furthermore, the detector may be a combination of two or more of these.

In addition, an analyzer that analyzes the detected light can be a general information processing apparatus, for example, a personal computer or the like. As the analyzers can be a general computer (general-purpose computer) equipped with storage means such as a hard disk drive (HDD), a solid state drive (SSD), and a read-only memory (ROM) for storing programs, data, and the like, and a central processing unit (CPU) for executing programs, performing calculation processing, and the like. The computer may further include an input means such as a keyboard or a mouse, and an output means such as a monitor or a printer.

### (2) Second Aspect

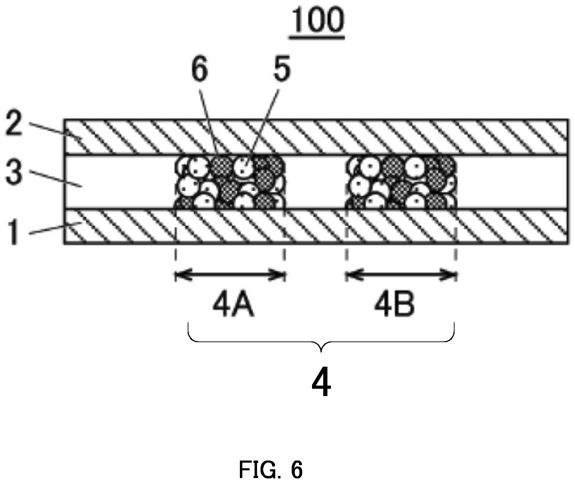

The analysis method of the present mode is a method characterized by using a specific analysis apparatus. An example of an analysis apparatus 100 used in the analysis method of the present mode is illustrated in Fig. 6. The analysis apparatus 100 includes a pair of electrodes 1 and 2 (exciters), a receiving layer (holder) 3 interposed between the electrodes 1 and 2 and configured to accommodate a target 5 and a luminescent probe 6 at predetermined positions, and a detector (not illustrated) configured to detect light emitted from the luminescent probe 6. In the analysis apparatus 100, a voltage is applied between the electrodes 1 and 2 to supply carriers (holes and electrons) to the luminescent probe in the receiving layer 3, thereby causing the luminescent probe to emit light. In the analysis apparatus 100, one or both of the electrodes 1 and 2 are configured to transmit light. Thus, the light emitted by the luminescent probe 6 can be detected. Furthermore, in the analysis apparatus 100, two or more types of light can be generated by housing different luminescent probes 6 in the containers 4 of the receiving layer 3. That is, the analysis apparatus 100 forms a region (light emission site) where the target 5 and the luminescent probe 6 have interacted with each other on the surface of or in the receiving layer 3.

In the analysis method using the analysis apparatus 100, first, at least one type of luminescent probe 6 that emits light by application of a voltage and change the behavior of light emission by interaction with a target are prepared (luminescent probe preparation step). The receiving layer (holder) 3 is separately prepared, the target 5 and the luminescent probe 6 are housed in the containers 4 of the receiving layer 3 to form the above-described light emission sites, and the electrodes 1 and 2 are disposed on both sides of the receiving layer 3 (the above-described interaction step). Next, a voltage is applied between the electrodes 1 and 2 to cause the luminescent probe 6 to emit light, and two or more types of light emissions are simultaneously detected by a detector (not illustrated) (the above-described light detection step). Furthermore, the two or more types of detected light emission are analyzed, and the state of the target is indirectly specified (analysis step). Hereinafter, each step of the method will be described.

### (Luminescent probe preparation step)

In the luminescent probe preparation step, at least one type of luminescent probe that emits light upon application of a voltage and whose light emission behavior changes by interaction with a target are prepared. As the luminescent probe, a known fluorescent compound, a delayed fluorescent compound, a phosphorescent compound, or the like can be used. A plurality of the luminescent probes may be used in combination, for example, different phosphorescent compounds may be used in combination, or a phosphorescent compound and a fluorescent compound may be used in combination. Thus, any luminescent color can be obtained.

In the present step, a plurality of luminescent probes having different luminescent colors may be prepared. Note that the color emitted by the luminescent probe is determined by the color obtained when the result obtained by measurement using a spectroradiometer CS-1000 (manufactured by Konica Minolta, Inc) in Fig. 3.16 on page 108 of "New Edition of Color Science Handbook" (edited by The Color Society of Japan, published by Tokyo University Press, 1985) is applied to the CIE chromaticity coordinates.

In the present step, only two types of luminescent probes may be prepared for one target, but from the viewpoint of obtaining a large amount of data on the target, it is preferable to prepare a large amount of luminescent probes for one target. More specifically, it is preferable to prepare two or more types of luminescent probes for one target, and it is even more preferable to prepare four or more types of luminescent probes.

### • Fluorescent compound

In the present invention, the "fluorescent compound" refers to a compound that emits fluorescence other than delayed fluorescence, and for example, those described in Japanese Unexamined Patent Publication No. 2022-99677, Japanese Unexamined Patent Publication No. 2022-116901, WO2022/163199 can be similarly utilized.

### • Phosphorescent compound

The "phosphorescent compound" in the present invention refers to a compound that emits light emission, and for example, those described in Japanese Unexamined Patent Publication No. 2022-99677, Japanese Unexamined Patent Publication No. 2022-116901, International Publication No. 2022163199 can be similarly utilized.

Examples of the phosphorescent compound which can be suitably used in the present mode will be described below. Note that phosphorescent compounds described in example which will be described below (e.g., a blue phosphorescence emitting probe Ep1, a red phosphorescence emitting probe Ep2, etc.) can also be suitably used in the present mode.

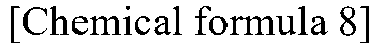

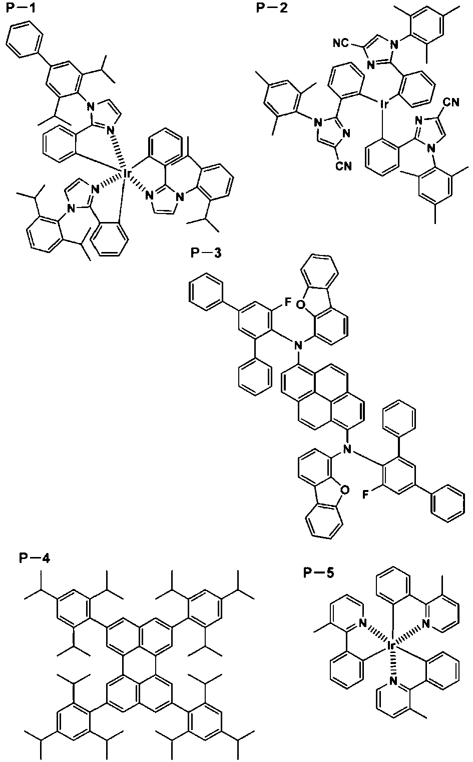

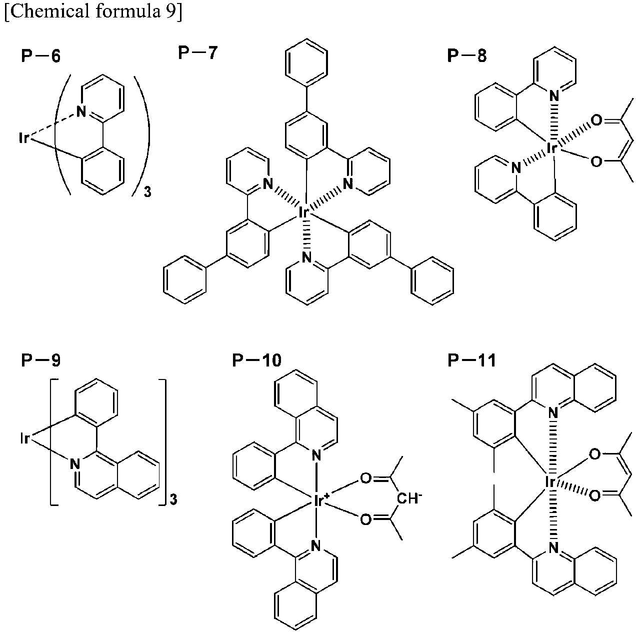

### • Delayed fluorescent compound

The term "delayed fluorescent compound" as used herein refers to a compound that emits delayed fluorescence, and for example, those described in Japanese Unexamined Patent Publication No. 2022-99677, Japanese Unexamined Patent Publication No. 2022-116901, WO2022/163199 can be similarly utilized.

Examples of the thermally activated delayed fluorescent compound are shown below, but the present mode is not limited thereto.

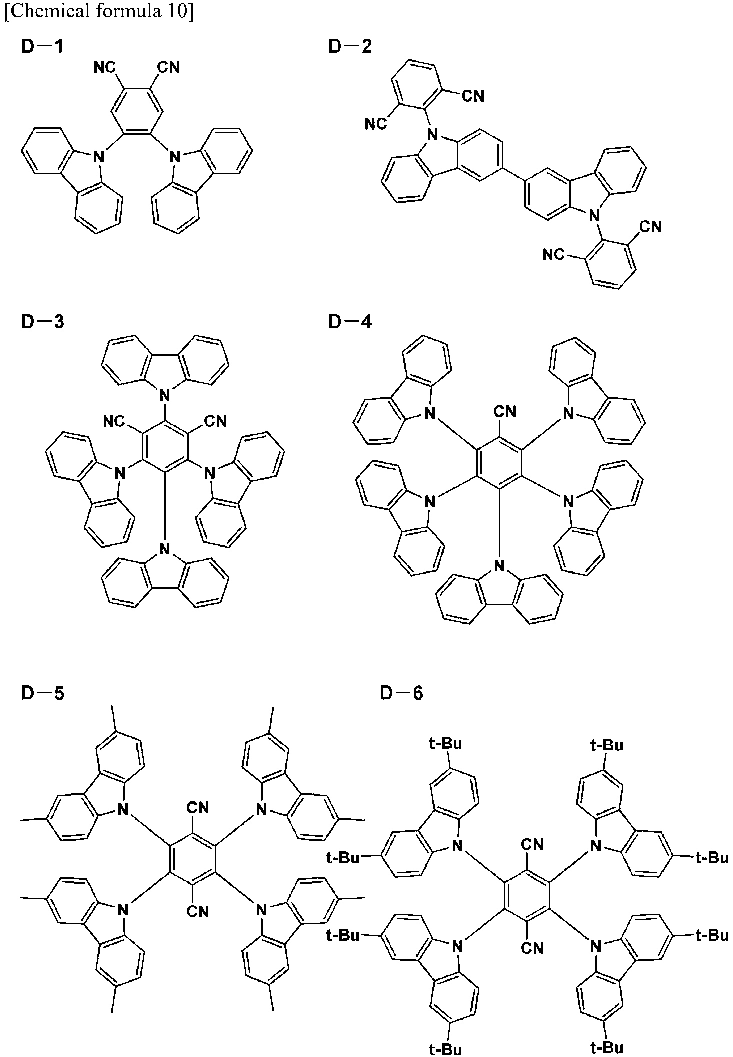

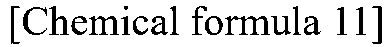

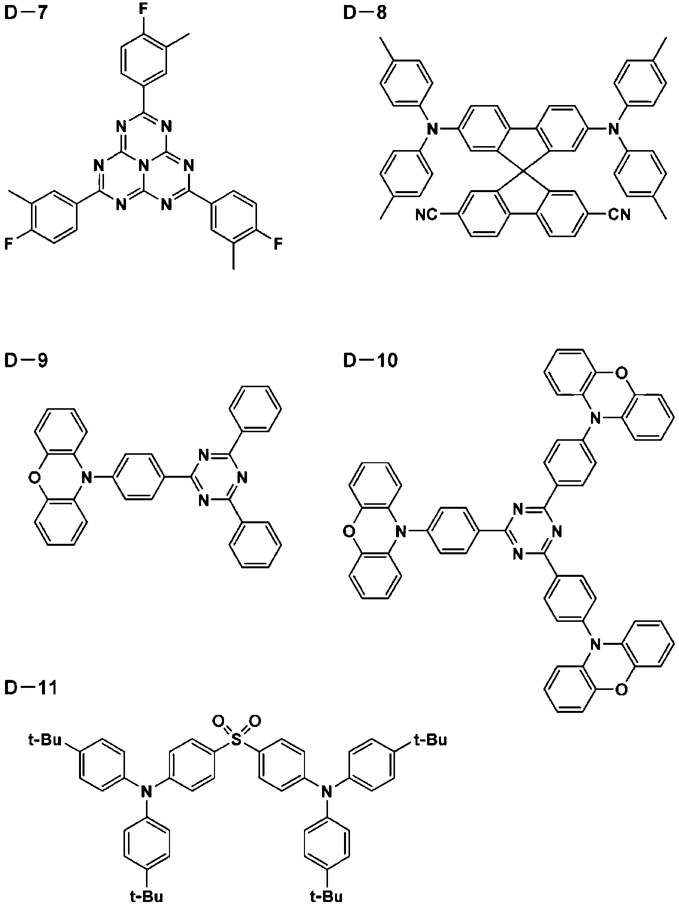

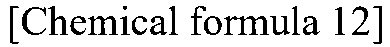

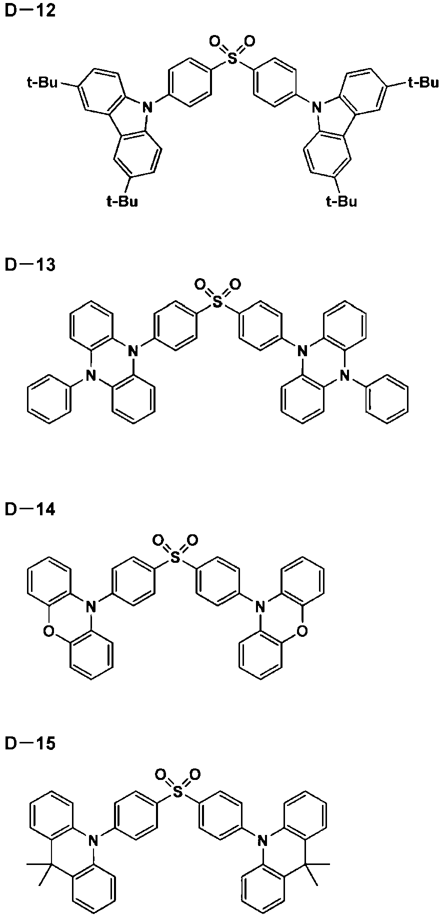

### • Triplet-triplet annihilation delayed fluorescent compounds

The "triplet-triplet annihilation delayed fluorescent compound" in the present specification refers to a compound that emits triplet-triplet annihilation delayed fluorescence (TTA delayed fluorescence), and for example, those described in Japanese Unexamined Patent Publication No. 2022-99677, Japanese Unexamined Patent Publication No. 2022-116901, WO2022/163199 can be similarly utilized.

Examples of the host compound that can be suitably used in the present mode are described below. Note that host compounds described in examples which will be described below can also be suitably used in the present mode.

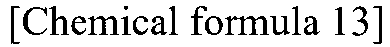

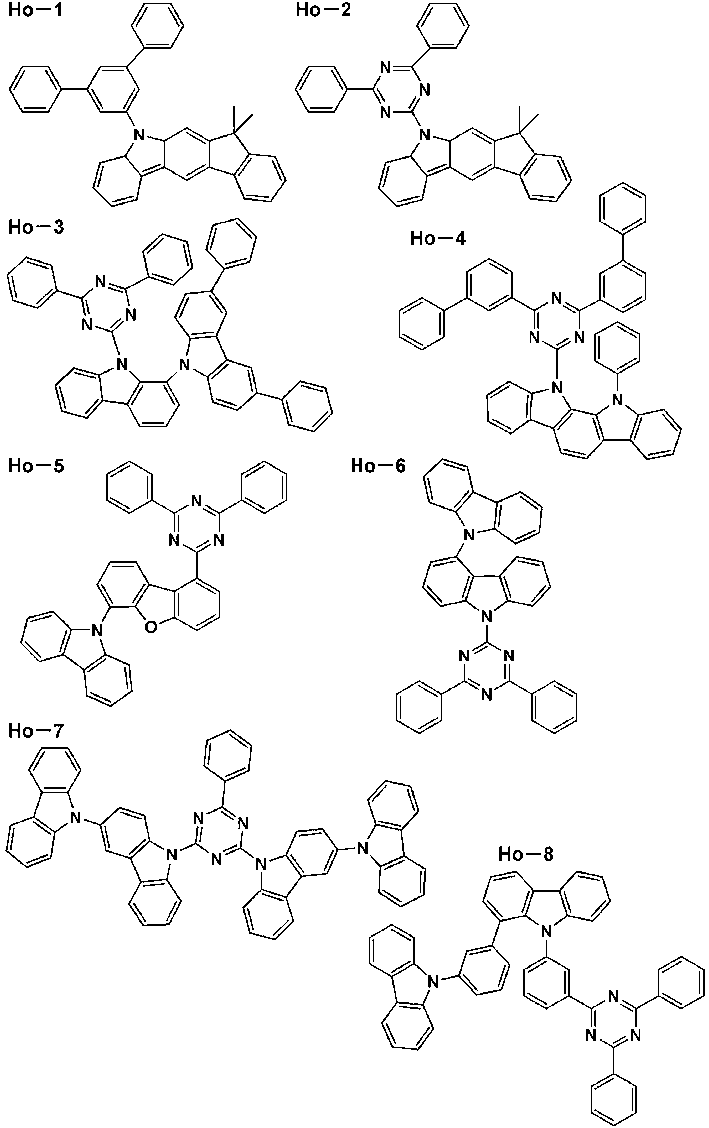

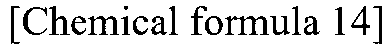

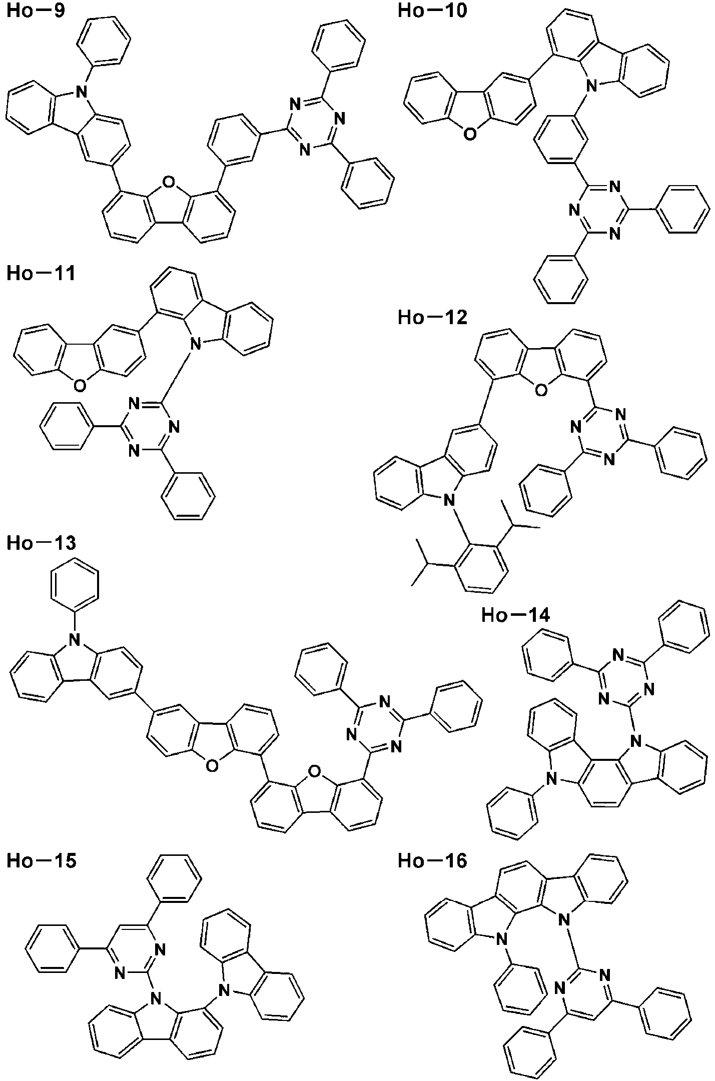

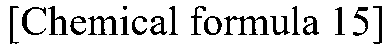

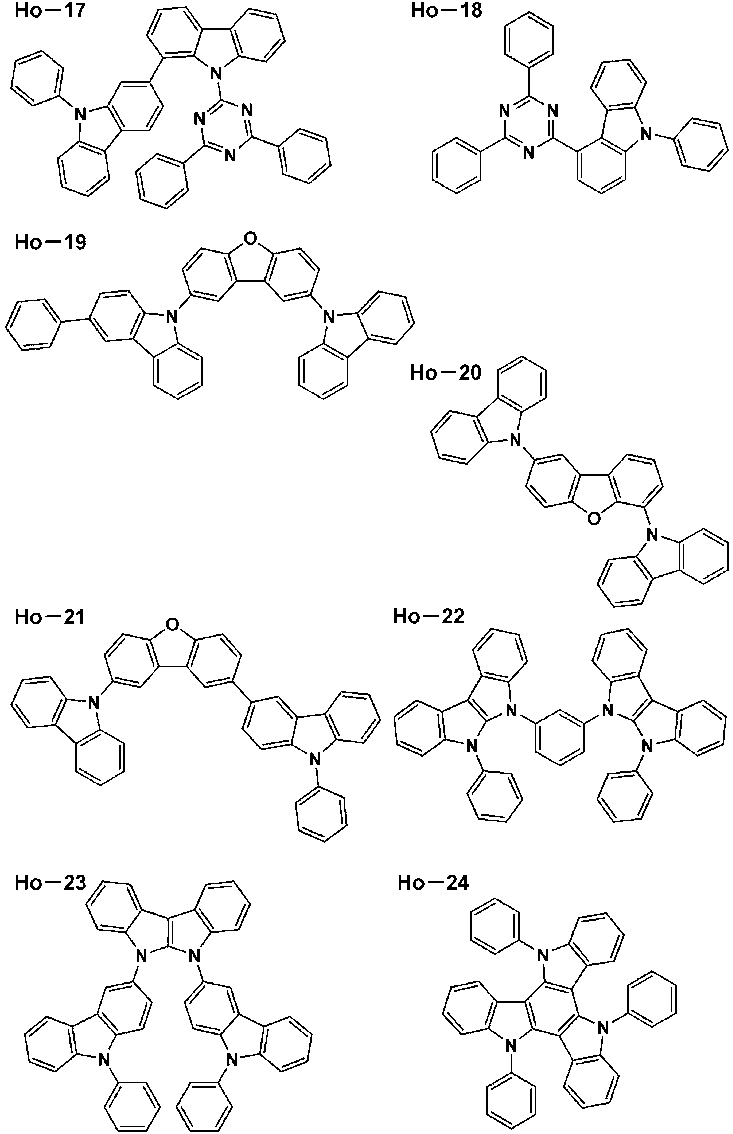

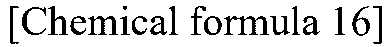

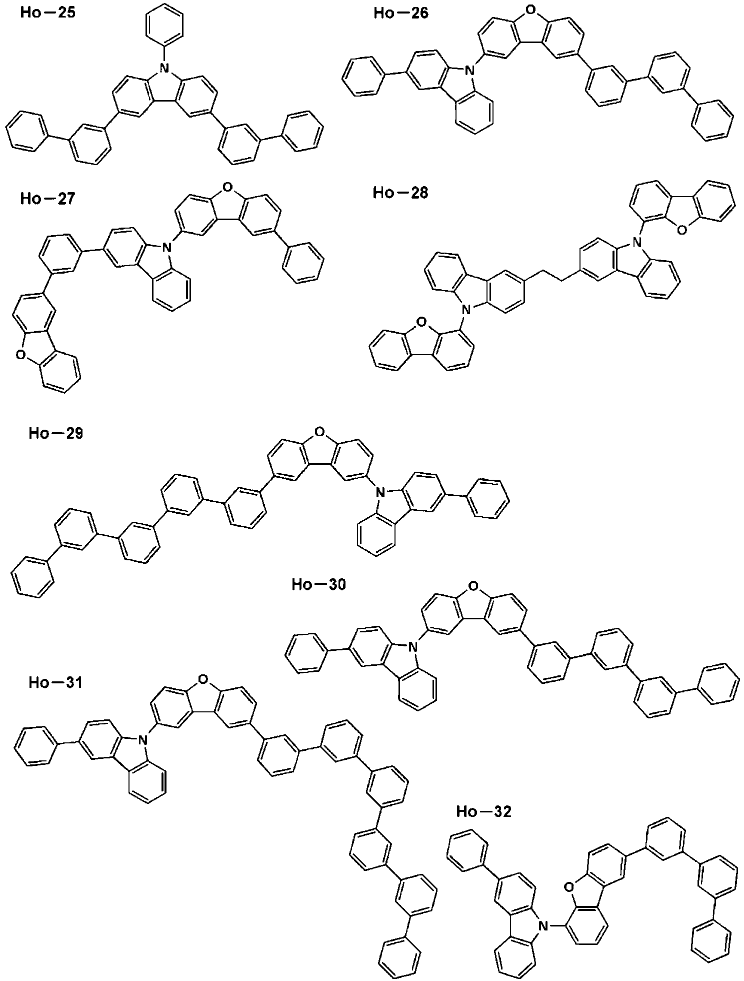

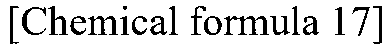

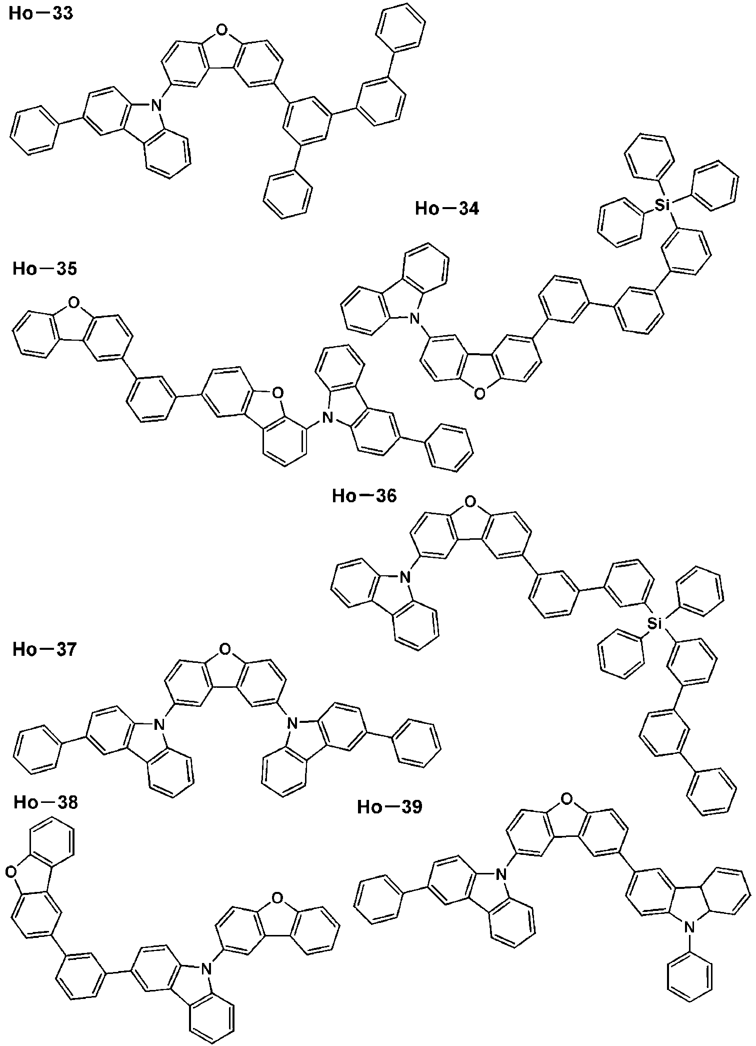

### (Interaction step)

The interaction step is a step of preparing a desired receiving layer 3 (holder) and disposing the target 5 and the above-described luminescent probe 6 at predetermined positions of the receiving layer 3.

In the present step, after the target 5 and the luminescent probe 6 are disposed at predetermined positions in the receiving layer 3, the pair of electrodes 1 and 2 may be disposed on both sides thereof, respectively. However, it is preferable that the electrode 1 and the support substrate are placed on one side of the receiving layer 3, the target 5 and the luminescent probe 6 are disposed at predetermined positions in the receiving layer 3 to form the above-described light emission site, and then the other electrode 2 is further disposed on the receiving layer 3.

Here, the receiving layer 3 is preferably at least one type of insulating polymer (i.e., having insulating properties). The term "insulating properties" of the insulating polymer refers to an electrical resistance of 1 × 10⁶ Ω·m or more, and the electrical resistance of the insulating polymer is preferably 1 × 10⁸ Ω·m or more, and more preferably 1 × 10¹⁰ Ω·m or more. When the electric resistivity of the insulating polymer alone is 1 × 10⁶ Ω·m or more, a leakage current can be prevented from flowing between a plurality of light emission sites when the target 5 or the light emission polymer 6 is disposed at a plurality of positions

The type of the insulating polymer is not particularly limited as long as the target 5 and the light emission polymer 6 can be held. Examples thereof include nonionic polymers such as polystyrene, polymethyl methacrylate, polyvinyl alcohol, polyacrylamide, polyvinylpyrrolidone, polyvinylpolypyrrolidone, polyethylene glycol, polymethyl vinyl ether and polyisopropylacrylamide; cationic polymers such as sodium polyacrylate, sodium polystyrene sulfonate, sodium polyisopropylene sulfonate, polynaphthalenesulfonic acid condensate salts and polyethyleneimine xanthate salts; anionic polymers such as dimethylaminomethyl (meth) acrylate quaternary salts, dimethyldiallylammonium chloride, polyamidine, polyvinylimidazoline, dicyandiamide-based condensates, epichlorohydrin dimethylamine condensates and polyethyleneimine; and amphoteric polymers such as dimethylaminoethyl (meth) acrylate quaternary salt-acrylic acid copolymers and Hoffman degradation products of polyacrylamide. Preferred are polystyrene and polymethyl methacrylate.

The weight-average molecular weight of the insulating polymer is not particularly limited, but is preferably 5 × 10³ or more, and more preferably 10 × 10³ or more. It is also preferably 1000 × 10³ or lower, more preferably 400 × 10³ or lower. When the weight-average molecular weight is in this range, the target 5 and the luminescent probe 6 disposed in the container 4 of the receiving layer 3 are less likely to diffuse in the receiving layer 3.

Note that the receiving layer 3 may contain an additional material and/or additive. Examples of the additive include halogen elements such as bromine, iodine, and chlorine; halogenated compounds; and compounds, complexes, and salts of alkali metals, alkaline earth metals, and transition metals, such as Pd, Ca, and Na.

In the present step, the target 5 and the luminescent probe 6 are individually disposed in the plurality of containers 4 in the receiving layer 3. The adjacent containers 4 (4A and 4B in Fig. 6) are preferably set to be spaced apart from each other. Thus, current leakage that occurs between the adjacent containers 4A and 4B can be suppressed. Fig. 6 illustrates only the two containers 4A and 4B, but it is preferable to arrange a large number of containers 4 in one receiving layer 3. For example, when the container 4 are formed at 10 positions in the vertical direction and at 10 positions in the horizontal direction at intervals in the four sides of 1 cm, the receiving layer 3 would include 100 or more containers 4 per 1 cm². In addition, the positions of the respective containers 4 may be set such that the containers 4 are disposed in a matrix when the receiving layer 3 is viewed in a plan view.

Either the target 5 or the luminescent probe 6 may be disposed first in the container 4. The target 5 and the luminescent probe 6 can be disposed by, for example, a coating method or the like. When the target 5 and the luminescent probe 6 are liquids, they may be applied as they are, but when they are solids or have a high viscosity, they may be applied after being dissolved or dispersed in a liquid. The coating method is not particularly limited, but a non-contact forming method is desirable from the viewpoint of less damage to the receiving layer, and ejection from an inkjet head is preferable. The volume of a droplet of the target at the time of dropping is preferably within a range of 1pL to 100µL.

Here, one luminescent probe and one host material may be disposed in one luminescent site, and either the type of luminescent probe or the type of host material may be changed for disposing in adjacent luminescent sites. Alternatively, a plurality of types of the luminescent probes 6 may be disposed in one container 4 to form a plurality of light emission sites. More specifically, a plurality of luminescent probes 6 may be combined to adjust the luminescent color to be different for each container 4 or to adjust the emission intensity to be different for each container 4. In addition, the concentration of the target may be adjusted so as to be different for each container 4. Adjusting the luminescent color and intensity and the concentration of the target to be different for the containers 4 facilitates acquisition of a large amount of data on the target 5. It is also preferable to combine a plurality of luminescent probes 6 having different luminescent colors to make the luminescent color of the container 4 white. The combination of luminescent probes that emit white light is not particularly limited, and examples thereof include a combination of blue and orange and a combination of blue, green, and red. In this case, when the 2-degree viewing angle front luminance is measured by the method described below, the white color preferably has a chromaticity in the range of x = 0.39 ± 0.09, y = 0.38 ± 0.08 in the CIE 1931 color system at 1000 cd/m².

In addition to the target 5 and the luminescent probe 6, the container 4 may be further coated with a substance that changes a carrier recombination position, an additive for promoting the interaction between the target 5 and the luminescent probe 6 or assisting the light emission of the luminescent probe 6, or the like. As the substance that changes the carrier recombination position, container 4 may contain a substance that changes the carrier recombination position. Specifically, the following three substance groups can be used. (1) Typical examples include the above-described electron transport materials and hole transport materials; (2) ionic materials which are charged to affect the movement of electrons and holes; and (3) aromatic compound and aliphatic compounds which interact with the light-emitting material and the host material. The use of such materials increases the variety of data and can emphasize changes in light. For example, the regression curve can be intentionally created by controlling the ejection amount of the substance to form the light emission sites such that adjacent light emission parts are different from each other by a certain amount. Therefore, inconvenient data such as an outlier can be deleted, and furthermore, the entire regression curve can be handled as one piece of data.

Note that the method of causing the target and the luminescent probe to interact with each other in the receiving layer 3 (the container 4) is not particularly limited, and the target 5 and the luminescent probe 6 may be simply applied to the same region, but heating or another treatment may be performed as necessary.

One of the electrodes 1 and 2 disposed on both sides of the receiving layer 4 may be an anode and the other may be a cathode, and at least one of the electrodes 1 and 2 may have optical transparency. For the anode, metals having a large work function (4eV or more, preferably 4.5 V or more), alloys, electrically conductive compounds and mixtures thereof are preferably used. Specific example of such materials include metals such as gold, and conductive transparent materials such as CuI, indium tin oxide (ITO, Indium Tin Oxide), SnO₂, and ZnO. In addition, the electrode material may be IDIXO (In₂O₃-ZnO), or the like.

Furthermore, a conductive polymer may be used for the anode. Examples of the conductive polymer include PEDOT, PSS, polypyrrole, polyaniline, polythiophene, polythienylenevinylene, polyazulene, polyisothianaphthene, polycarbazole, polyacetylene, polyphenylene, polyphenylenevinylene, polyacene, polyphenylacetylene, polydiacetylene, polynaphthalene, and derivatives thereof. One of these electrode materials may be used alone, or two or more of these materials may be mixed and used. It is also possible to form the electrode by laminating two or more types of layers composed of each material.

On the other hand, for the cathode, metals having a small work function (5eV or less) (referred to as electron-injecting metals), alloys, electrically conductive compounds and mixtures thereof are used. Specific examples of such electrode materials include sodium, sodium-potassium alloys, magnesium, lithium, silver, magnesium/copper mixtures, magnesium/silver mixtures, magnesium/aluminum mixtures, magnesium/indium mixtures, aluminum/aluminum oxide (Al₂O₃) mixtures, indium, lithium/aluminum mixtures, aluminum, rare earth metals, and the like.

Among these, from the viewpoint of the electron injecting property and durability against oxidation or the like, a mixture of an electron injecting metal and a second metal which is a stable metal having a larger work function value than the electron injecting metal, for example, a magnesium/silver mixture, a magnesium/aluminum mixture, a magnesium/indium mixture, an aluminum/aluminum oxide (Al₂O₃) mixture, a lithium/aluminum mixture, aluminum or the like is suitable.

The anode and the cathode may be a film that can be produced by a known method, for example, a film formed by a method such as vapor deposition or sputtering, or may be a film formed by a printing method or the like. Alternatively, it may be patterned into a desired shape.

In addition, a transparent or opaque support substrate may be disposed outside the pair of electrodes 1 and 2. Provided that a transparent support substrate is preferably disposed on the light extraction side. Examples of the transparent support substrate include glass, quartz, and transparent resin films. When necessary, a gas barrier film or the like may be disposed on the surface of the substrate. In addition, a hue improving filter such as a color filter, and the like may be further disposed on the light extraction side.

### (Light detection step)

In the light detection step, a voltage is applied between the electrodes 1 and 2 to cause the luminescent probe 6 in the receiving layer 3 to emit light. Then, two or more types of light emitted from the luminescent probe are detected by a detector (not illustrated).

The voltage applied between the electrodes 1 and 2 is not particularly limited, and may be a DC voltage or an AC voltage, but is preferably an AC voltage. For example, an AC voltage can be applied by connecting the pair of electrodes 1 and 2 to an AC power source. The AC power supply preferably has a frequency of 1 Hz to 100 MHz, an offset voltage of 0 to 20V, and an amplitude voltage of about 0 to 20V. Examples of the usable AC power source include 6243 DC VOLTAGE CURRENT SOURCE/MONITOR (manufactured by ADCM), Function Generator FG300 (manufactured by YOKOGAWA) and the like. During the application of the voltage, current measurement is also preferably performed, and an ammeter capable of measuring a current of about 1 nA to 100 mA is preferably connected. Examples of the ammeter measurement device include Source Meter 2400 (manufactured by Keithley Instruments Inc).

In addition, a method of detecting two or more types of light by the detector is not particularly limited, and the detection can be performed by a spectral radiance measuring device or the like. For example, the radiance, the light emission spectrum, or the like of the luminescent probe in the region where the target 5 and the luminescent probe 6 are disposed may be measured.

Furthermore, the detector may be an imaging apparatus or the like, and may capture, as an image, light emitted by the luminescent probe when a voltage is applied.

### (Analysis step)

The analysis method in the present step is appropriately selected depending on the purpose, the type of the information on the light emission acquired in the light detection step, or the like. For example, the data detected in the light detection step may be used as analysis data as it is. As described in Fig. 35A to Fig. 35D described above, in the present embodiment, the difference between the light emitted when the target is included and the light emitted when the target is not included may be obtained and analyzed as a relative value.

In the analysis step, for example, the discrimination rate may be calculated from the voltage application conditions applied by the power supply, the current characteristics, and the light emission spectrum specified by a spectral radiance measurement device or the like. The discrimination can be performed by a discrimination rate calculator or the like, and the discrimination rate calculator calculates the discrimination rate based on the number of times of voltage supply and the voltage supply time.

The discriminant algorithm by the discriminant ratio calculator is preferably any one of the following: principal component analysis (PCA), cluster analysis (hierarchical method, k-means method, normal mixture method), linear discriminant analysis (LDA), partial least squares method (PLS regression), decision tree, random forest, boosted trap forest, neural network, neural boosting, K-nearest neighbor method, simple Bayes, support vector machine (SVM), nominal logistic (multiple logit), generalized regression (Ridge, lasso), and a combination thereof. These will be described below.

### • Analysis method

The statistical analyses and the analyses in the machine learning can be performed by principal component analysis (PCA), cluster analysis (hierarchical method, k-mean method, or normal mixture method), linear discriminant analysis (LDA), partial least squares method (PLS regression), or the like, or a combination thereof, using statistical analytical software JMP 16.2 or JMPpro 16.2 available from SAS Institute Japan Inc. In addition, a decision tree, a random forest, bootstrap forest, a neural network, a K-nearest neighbor method, simple Bayes, a support vector machine (SVM), nominal logistic (multiple logits), generalized regression (Ridge, lasso), or the like may be used.

Provided that when the number of samples is about 50 or less, the explanatory variable is 100 or more, and the variable is spectral data, PCA which is linear regression using dimension-reduced main components, PLS regression, and LDA (the horizontally long data method in JMP) using main components are more preferable from the viewpoints of suppression of overlearning and the robustness of the result. However, depending on the data set, the above-described analysis methods other than these may be effective.

### < For linear discriminant analysis (LDA) >

As for the linear discriminant analysis (LDA), in JMP 16.2, there are a stepwise method in which an explanatory variable is arbitrarily selected and a the horizontally long data method in which dimensionality reduction is performed on main components, and either of the methods can be applied. The horizontally long data method is preferable from the viewpoints of a reduction in calculation time and the above-described overlearning suppression.

### • Evaluation of discrimination performance

For the evaluation of the discrimination performance, all sample data are divided into training data and verification data in an arbitrary ratio, and linear discriminant analysis (LDA) is performed, to calculate the discrimination rate (correct answer rate and predictive value), the erroneous discrimination rate and the first power of entropy. For the calculation of the verification result using the verification dataset, it is preferable to use the hold out method using an arbitrary or random pair of verification datasets, or k-fold cross validation in which all sample data are divided into k subsets and cross validation is performed using the k subsets.

In addition, as a result of the k-fold cross validation, it is preferable to display an average value of k discriminant models, a discriminant model having the best statistic (a smallest error from the average) among k discriminant models, or a discriminant model having the smallest difference in discrimination rate or entropy to the first power between learning and validation.

### (Others)

In the analysis apparatus 100 used for the above-described analysis method, an organic functional layer such as a hole injection layer, a hole transport layer, an electron transport layer, and/or an electron injection layer may be further provided in the receiving layer 3. As typical configuration examples of the above-described analysis apparatus, the following configuration examples can be given, but the present invention is not limited thereto.
(I) Anode/receiving layer (container)/cathode
(ii) Anode/receiving layer (container/electron transport layer)/cathode
(iii) Anode/receiving layer (hole transport layer/container)/cathode
(iv) Anode/receiving layer (hole transport layer/container/electron transport layer)/cathode
(v) Anode/receiving layer (hole transport layer/container/electron transport layer/electron injection layer)/cathode
(vi) Anode/receiving layer (hole injection layer/hole transport layer/electron blocking layer/container/hole blocking layer/electron transport layer)/cathode

Note that any one of the hole injection layer, the hole transport layer, the electron blocking layer, the detection region, the hole blocking layer, and the electron transport layer may be provided not inside the receiving layer but between the receiving layer and an electrode (anode or cathode) outside the receiving layer.

The "electron transport layer" according to the present mode is a layer having a function of transporting electrons, and in a broad sense, the electron injection layer and the hole blocking layer are also included in the electron transport layer. Further, the electron transport layer may be composed of a plurality of layers.

The "hole transport layer" according to the present mode is a layer having a function of transporting holes, and in a broad sense, a hole injection layer and an electron blocking layer are also included in the hole transport layer. Further, the hole transport layer may be composed of a plurality of layers. These layers are the same as known layers. Examples

### 1. Example 1

### 1-1. Synthesis of luminescent probes 1 to 16

All reactions were performed under a nitrogen atmosphere in oven-dried glassware unless otherwise noted. All chemical products were purchased from Aldrich or TCI or Kanto Chemical Co., Inc. and used as is without further purification.

### (1) Synthesis of monomer 1

Based on the following reaction formula, a monomer 1 having a main chain containing a phosphate ester and a luminophore bonded to the main chain was synthesized via intermediates 1 to 6.

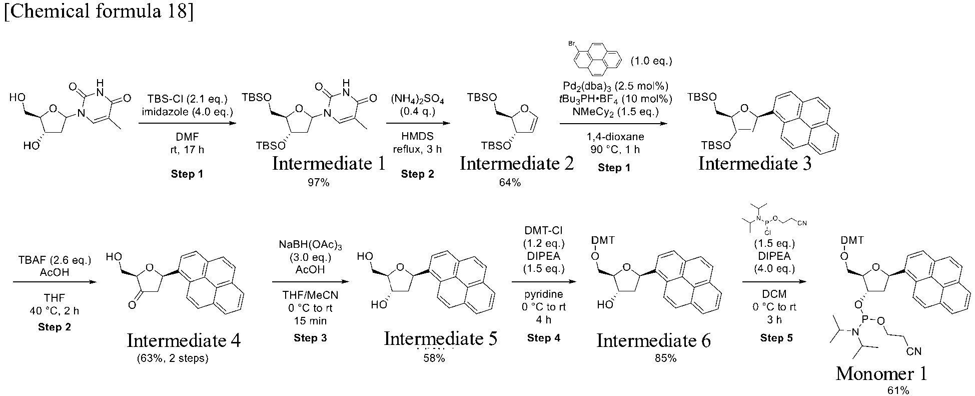

### • Synthesis of intermediate 1

Thymidine (15.0 g, 61.9 mmol) and imidazole (16.9 g, 248 mmol) were dissolved in DMF (124mL), tert-butyldimethylsilylchloride (19.6 g, 130 mmol) was added, and the mixture was stirred at room temperature for 17 hr. The reaction was stopped by adding water, and the mixture was subjected to liquid separation and extraction with ethyl acetate. The resulting organic phase was dried over magnesium sulfate, and the solvent was distilled off to obtain the desired intermediate 1 as a colorless solid (28.3 g, 97%).

### • Synthesis of intermediate 2

Intermediate 1 (28.3 g, 60.1 mmol) and ammonium sulfate (12.7 g, 96.2 mmol) were dissolved in hexamethyldisilazane (314 mL, 1.50 mol) and heated under reflux for 3 hours. Water was then added to the reaction solution, and the mixture was subjected to liquid separation and extraction with ethyl acetate. The resulting organic phase was dried over magnesium sulfate, and the solvent was distilled off to obtain a crude product, which was purified by silica gel column chromatography to obtain the desired intermediate 2 as a brown liquid (13.2 g, 64%).

### • Synthesis of intermediate 4

A mixture of intermediate 2 (10.1 g, 29.3 mmol), 1-bromopyrene (8.24 g, 29.3 mmol), tris(dibenzylideneacetone) dipalladium (0) (671mg, 733 µmol), tri-tert-butylphosphonium tetrafluoroborate (850 mg, 2.93 mmol), dicyclohexylmethylamine (9.35 mL, 44.0 mmol), and 1,4-dioxane (100 mL) was heated at 90°C for 1 hour. Water was added to stop the reaction, and liquid separation and extraction were performed with ethyl acetate. The resulting organic phase was dried over magnesium sulfate, and the solvent was distilled off to give a crude product containing intermediate 3, which was used as is in the next reaction.

To the crude product containing intermediate 3, 100mL of THF, 1M tetrabutylammonium fluoride THF solution (117 mL, 117 mmol), and acetic acid (6.74 mL, 117 mmol) were added, and the mixture was stirred at 40°C for 2 hr. Water was added to stop the reaction, and liquid separation and extraction were performed with ethyl acetate. The resulting organic phase was dried over magnesium sulfate, and the solvent was distilled off to give a crude product, which was purified by silica gel column chromatography to give the desired intermediate 4 as a light brown solid (5.87 g, 63%).

### • Synthesis of intermediate 5

The solution of sodium triacetylborate (11.8 g, 55.8 mmol) and acetic acid (7.87 mL, 138 mmol) in acetonitrile 93 mL was cooled to 0°C. Then, the solution of intermediate 4 (5.87 g, 18.6 mmol) in THF (62mL) was added dropwise. After completion of the dropwise addition, the mixture was warmed to room temperature and stirred for 15 min, and water was added to stop the reaction. After separation and extraction with ethyl acetate, the resulting organic phase was dried over magnesium sulfate, and the solvent was distilled off to obtain a crude product. Purification by silica gel column chromatography and reversed-phase HPLC gave the desired intermediate 5 as a colorless solid (3.44 g, 58%).

### • Synthesis of intermediate 6

A mixture of intermediate 5 (3.44 g, 10.8 mmol), 4,4'-dimethoxytritylchloride (4.40 g, 13.0 mmol), ethyldiisopropylamine (2.82 mL, 16.2 mmol) and dehydrated pyridine (54mL) was stirred at room temperature for 4 hr. Thereafter, methanol was added to stop the reaction. The solvent was distilled off and the resulting crude product was purified by silica gel column chromatography to obtain the desired intermediate 6 as a colorless viscous solid (5.71 g, 85%).

### • Synthesis of monomer 1

To a mixture of intermediate 6 (5.71 g, 9.20 mmol), ethyldiisopropylamine (6.42 mL, 36.8 mmol), and dehydrated dichloromethane (92 mL), 2-cyanoethyldiisopropylchlorophosphoroamidite (3.08 mL, 13.8 mmol) was added dropwise at 0°C. After heating the mixture to room temperature and stirring for 3 hours, the solvent was distilled off to obtain a crude product. Purification by silica gel column chromatography gave the desired monomer 1 as a colorless solid (4.64 g, 61%).

### (2) Preparation of monomer 2

For monomer 2, a reagent with the structure shown below, was purchased from Glen Research (Sterling, Va).

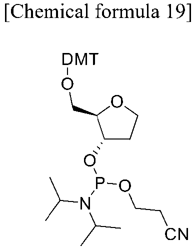

### (3) Measurement of size of light emitter of monomer 1 and monomer 2

For the monomer 1 and the monomer 2, the size of the structure (light emitter contributing to light emission) represented by R in the following general formula was calculated by the following method. Note that when the structure represented by R is a hydrogen atom, the portion cannot become a light emitter, and therefore, the size is set to 0.

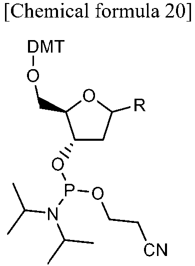

### • Method for measuring size of light emitter

With respect to the size of the structure represented by R of the above-described compound, the structure represented by R was regarded as independent molecules by using Winmostar (developed by Crossability, Inc), which is an atom-and molecule-scale structure modeling and visualization software. Then, the length of a cylinder having the smallest diameter in which the molecule was inscribed at this time was obtained as the size of the structure. The results are shown in the following Table 1.

**[Table 1]**

| | Size of structure R (light emitter) (nm) |
|---|---|
| Monomer 1 | 1.14 |
| Monomer 2 | 0 |

### (4) Synthesis of luminescent probes 1 to 16

According to a conventional method, as shown in Table 2 below, 16 types of oligonucleotides (sequences 1 to 16) having a mixed sequence of the monomer 1 and the monomer 2 were synthesized. DNA synthesis reagents were purchased from Glen Research (Sterling, Va). In addition, all of the oligonucleotides were synthesized with a DNA/RNA synthesizer NTS Terminator manufactured by Nihon Techno Service Co., Ltd. using a standard protocol for a phosphoramidite base coupling procedure. Each luminescent probe-carrier obtained by the automatic synthesis were reacted with ammonium water at room temperature for 2 hours, cut out from the particulate carrier, the solvent was dried to dryness with a centrifugal drying apparatus, and then ultrapure water was added thereto, to obtain each of first components 1 to 16 containing the respective luminescent probes 1 to 16. It was confirmed that the luminescent probes 1 to 15 emit fluorescence and excimer light by specific excitation light (light having wavelength 350 nm). Note that the luminescent probe 16 did not exhibit fluorescence or excimer light emission. The proportion of the β-form of deoxyribose in the table was obtained as {(the number of β-deoxyribose in the luminescent probe) / (the number of deoxyribose in the luminescent probe)} × 100 [%].

**[Table 2]**

| Luminescent probe | | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|---|
| Monomer sequence | 5' | 1 | 1 | 1 | 1 | 1 | 1 | 1 | 1 | 2 | 2 | 2 | 2 | 2 | 2 | 2 | 2 |
| | | 1 | 1 | 1 | 1 | 2 | 2 | 2 | 2 | 1 | 1 | 1 | 1 | 2 | 2 | 2 | 2 |
| | | 1 | 1 | 2 | 2 | 1 | 1 | 2 | 2 | 1 | 1 | 2 | 2 | 1 | 1 | 2 | 2 |
| | 3' | 1 | 2 | 1 | 2 | 1 | 2 | 1 | 2 | 1 | 2 | 1 | 2 | 1 | 2 | 1 | 2 |
| Proportion of β-form of deoxyribose (%) | | 100 | 100 | 100 | 100 | 100 | 100 | 100 | 100 | 100 | 100 | 100 | 100 | 100 | 100 | 100 | - |
| Number of light emitter | | 4 | 4 | 4 | 2 | 3 | 2 | 2 | 1 | 3 | 2 | 2 | 1 | 1 | 1 | 1 | 0 |
| Proportion of natural nucleic acid base (%) | | 0 | 0 | 0 | 0 | 0 | 0 | 0 | 0 | 0 | 0 | 0 | 0 | 0 | 0 | 0 | 0 |

### 1-2. Blank information acquisition step

A 96-well microplate in which wells each having a opening diameter of 7 mm were disposed at the interval of 9 mm in 12 columns and 8 rows was prepared. On the 96-well microplate, 100 µl of each of the luminescent probes 1 to 16 was placed using an automatic dispenser (NichiMart CUBE, manufactured by Nichiryo Co., Ltd.) to form a plurality of reaction fields.

The 96-well microplate on which the above-described luminescent probes were placed was irradiated with excitation light (wavelength 350 nm), and the fluorescence spectrum was obtained as first signal information (blank information) for each luminescent probe.

### 1-3. Interaction step

In the 96-well microplate after the blank information acquisition step, 20 µl of each of the three types of soft drinks (targets 1 to 3) was placed by a method similar to the above.

### 1-4 . Light detection step

The 96-well microplate containing the above-described luminescent probes and targets was irradiated with excitation light (wavelength 350 nm), and the fluorescence spectrum was obtained as second signal information for each luminescent probe.

### 1-5. Analysis step

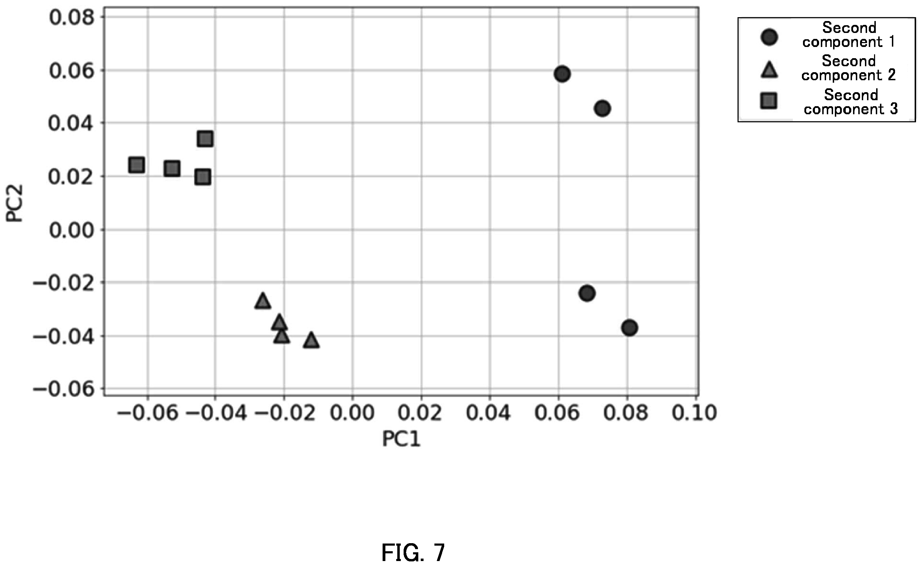

Analysis data was calculated by subtracting the first signal information acquired in the blank information acquisition step from the second signal information acquired in the light detection step. Then, when principal component analysis was performed using the analysis data as explanatory variables, it was possible to separate the second components (target) by type in the two-dimensional space where principal components 1 and 2 were plotted, as illustrated in Fig. 7.

### 1-6. Others

Pigment inks were prepared as follows using respective luminescent probe carriers obtained by the above-described automatic synthesis as pigment dispersion liquids, and it was confirmed that the same analysis method as above could be used.

### [Production of ink]

### • Ink raw material

Triethylene glycol monobutyl ether (TEGmBE, manufactured by Tokyo Chemical Industry Co., Ltd): 5 parts by mass
2-Pyrrolidinone (manufactured by BASF): 8 parts by mass
Purified glycerin (manufactured by Kao Corporation): 2 parts by mass
Surfynol 440 (nonionic surfactant, manufactured by AIRPRODUCTS), (2,4,7,9-tetramethyl-5 decyne-4,7-diol): 0.5 parts by mass
1,2-Hexanediol (manufactured by Degussa AG): 1 part by mass
Triethanolamine (manufactured by Konishi Co., Ltd.): 0.8 parts by mass
Megafac 444 (nonionic surfactant, manufactured by DIC Corporation) (oxide adduct of perfluoroalkylethylene): 0.2 parts by mass
Polyurethane A: 0.5 parts by mass (solid content)
AQUACER552: 1 part by mass (solid content)

The remaining amount of pure water (here, the "remaining amount" in the amount of pure water to be added indicates that the amount of pure water is adjusted and added so that the sum of the parts by mass of the blended raw materials becomes 100 parts by mass)

The above components were placed in a 100 ml plastic container in the above proportions and stirred for 1 hour. Then, 20 parts by mass of the aqueous dispersion liquid of the carrier of the luminescent probe 1 was added, and the mixture was further stirred for 1 hour to prepare a pigment ink composition.

[Inkjet ejection evaluation test] The ejection evaluation was performed using the ink composition prepared by the above-described method. For the ejection evaluation, a commercially available thermal-jet-type inkjet printer (Photosmart D5360, manufactured by Hewlett-Packard Company) was used. In addition, printing was performed on a photographic sheet (glossy) (HP Advance Photo Paper, manufactured by Hewlett-Packard Company), which is a sheet dedicated to inkjet printing. As a result, it was confirmed that the ink compositions prepared by the above-described method could be ejected by a inkjet method.

[Preservability] It was also confirmed that inkjet discharge was possible as described above even after storage at room temperature for one month.

[Discussion] From the results of the analysis step, the results of the inkjet ejection evaluation test, and the like, it was possible to separate the data on the soft drinks by type with the use of, as the luminescent probes, probes each including a light emitter of the order of nanometers whose light emission behavior changes chemically and/or physically by interaction with a target and further combining a plurality of light emitters different in size from each other. That is, such a change in physicochemical phenomenon (state information of the target) could be acquired in a short time and by a simple method by analysis using the luminescent probe.

### 2. Example 2

### 2-1. Synthesis of luminescent probes 1 to 75

### (1) Synthesis of monomer 3

Based on the following reaction formula, a monomer 3 having a main chain containing a phosphate ester and a luminophore bonded to the main chain was synthesized via intermediates 7 to 10.

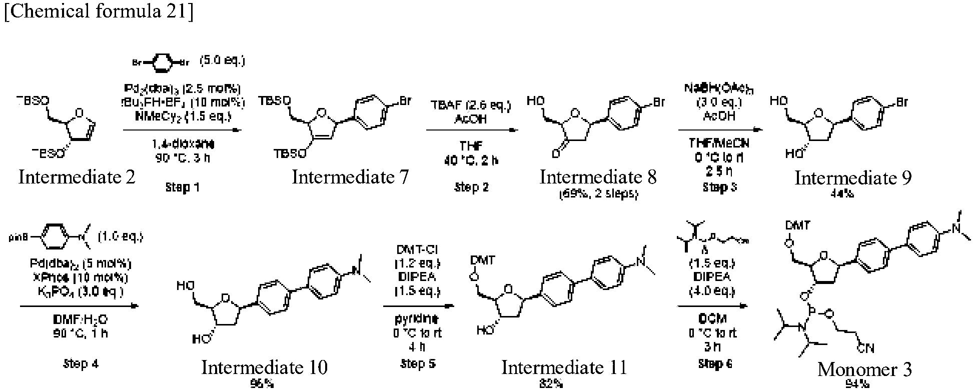

### • Synthesis of intermediate 8

A mixture of the above intermediate 2 (2.00 g, 5.80 mmol), 1,4-dibromobenzene (8.24 g, 29.0 mmol), tris(dibenzylideneacetone) dipalladium (0) (133mg, 145 µmol), tri-tert-butylphosphonium tetrafluoroborate (168mg, 580 µmol), dicyclohexylmethylamine (1.85 mL, 8.70 mmol), and 1,4-dioxane (29 mL) was heated at 90°C for 3 hours. Water was added to stop the reaction, and liquid separation and extraction were performed with ethyl acetate. The resulting organic phase was dried over magnesium sulfate, and the solvent was distilled off to obtain a crude product containing intermediate 7, which was used as it was for the next reaction.

Then, 29mL of THF, 1M tetrabutylammonium fluoride THF solution (23.2 mL, 23.2 mmol), and acetic acid (1.32 mL, 23.2 mmol) were added to the crude product containing the intermediate 7, and the mixture was stirred at 40°C for 2 hr. Water was added to stop the reaction, and liquid separation and extraction were performed with ethyl acetate. The resulting organic phase was dried over magnesium sulfate, and the solvent was distilled off to give a crude product, which was purified by silica gel column chromatography to give the desired intermediate 8 as a yellow-brown oily substance (1.08 g, 69%).

### • Synthesis of intermediate 9

Sodium triacetylborate (2.52 g, 11.9 mmol) and acetic acid (1.82 mL, 31.8 mmol) were dissolved in acetonitrile 20 mL. The solution was cooled to 0°C, and a solution of intermediate 8 (1.08 g, 3.98 mmol) in THF (13mL) was added dropwise. After completion of the dropwise addition, the mixture was warmed to room temperature and stirred for 2 hours and 30 minutes, and then water was added to stop the reaction. After separation and extraction with ethyl acetate, the resulting organic phase was dried over magnesium sulfate, and the solvent was distilled off to obtain a crude product. After washing with heptane, purification by recrystallization with ethyl acetate gave the desired intermediate 9 as a colorless solid (481mg, 44%).

### • Synthesis of intermediate 10

A mixture of intermediate 9 (1.00 g, 3.66 mmol), N,N-dimethyl-4v(4,4,5,5-tetramethyl-1,3,2-dioxaborolan-2-yl)aniline (905 mg, 3.66 mmol), bis(dibenzylideneacetone)palladium(0) (106 mg, 184 µmol), ethyldiisopropylamine 2-dicyclohexylphosphino-2',4',6'-triisopropylbiphenyl (175 mg, 367 µmol), potassium phosphate (2.33 g, 367 µmol), N,N-dimethylformamide (33 mL), and water (4 mL) was stirred at 90°C for 1 hour. Water was then added to stop the reaction, and liquid separation and extraction were performed with dichloromethane. The resulting organic phase was dried over magnesium sulfate, and the solvent was distilled off to obtain a crude product, which was purified by silica gel column chromatography to obtain the desired intermediate 10 as a colorless solid (1.10 g, 96%).

### • Synthesis of intermediate 11

A mixture of intermediate 10 (1.10 g, 3.52 mmol), 4,4'-dimethoxytritylchloride (1.32 g, 3.90 mmol), ethyldiisopropylamine (0.92 mL, 5.29 mmol) and dehydrated pyridine (17.5 mL) was stirred at room temperature for 4 hours, and then methanol was added to stop the reaction. The solvent was distilled off and the resulting crude product was purified by silica gel column chromatography to obtain the desired intermediate 11 as a yellow viscous solid (2.89 g, 82%).

### • Synthesis of monomer 3

To a mixture of intermediate 11 (1.23 g, 2.00 mmol), ethyldiisopropylamine (1.39 mL, 8.00 mmol) and dehydrated dichloromethane (80 mL), 2-cyanoethyldiisopropylchlorophosphoroamidite (468 µL, 2.10 mmol) was added dropwise at room temperature. After stirring the mixture for 2 hours, the solvent was distilled off to obtain a crude product. Purification by silica gel column chromatography gave the desired monomer 3 as a yellow viscous solid (1.53 g, 94%).

### (2) Preparation of monomer 4

For monomer 4, a reagent with the structure shown below, was purchased from Glen Research (Sterling, Va). The thymidine contained in the monomer 4 is a type of natural nucleic acid base.

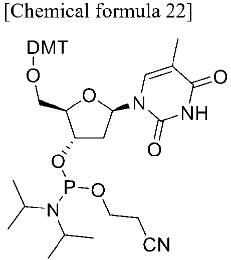

### (3) Synthesis of monomer 5

Monomer 5 was synthesized according to a Non Patent Literature (J. Am. Chem. Soc. 1996, 118, 7671-7678). Note that monomer 5 is a structural isomer of the above-described monomer 1 and is a monomer in which the sugar structure is an α-form.

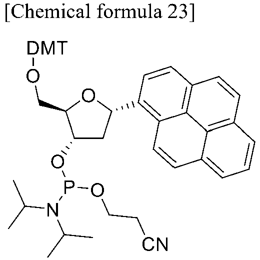

### (4) Measurement of size of light emitter of monomers 3 to 5

For the monomers 3 to 5, the size of the structure (light emitter contributing to light emission) represented by R in the following general formula was calculated by the same method as described above. The results are shown in Table 3. Table 3 also shows the results for monomer 1 and monomer 2. Note that when the structure represented by R is a hydrogen atom, the portion cannot be a light emitter, and therefore, the size is set to 0.

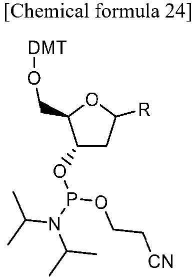

**[Table 3]**

| Compound | Molecular size (nm) of structure R (light emitter) |
|---|---|
| Monomer 1 | 1.14 |
| Monomer 2 | 0 |
| Monomer 3 | 1.36 |
| Monomer 4 | 0.85 |
| Monomer 5 | 1.14 |

### (5) Synthesis of luminescent probes 1 to 60

### (5-1) Preparation of the luminescent probes 1 to 16

Luminescent probes 1 to 16 were prepared as in example 1 described above.

### (5-2) Synthesis of luminescent probes 1 and 17 to 31

As shown in Table 4 below, 16 types of oligonucleotides (sequences 1 and 17 to 31) having a mixed sequence of the monomer 1 and the aniline-containing monomer 3 described above were synthesized by a method similar to the method for synthesizing the luminescent probes 1 to 16 described above. It was confirmed that each of the luminescent probes 1 and 17 to 31 emitted fluorescence and exciplex light emission by specific excitation light (light having wavelength 350 nm).

**[Table 4]**

| Luminescent color dye molecule | | 1 | 17 | 18 | 19 | 20 | 21 | 22 | 23 | 24 | 25 | 26 | 27 | 28 | 29 | 30 | 31 |
|---|---|---|---|---|---|---|---|---|---|---|---|---|---|---|---|---|---|
| Monomer sequence | 5' | 1 | 1 | 1 | 1 | 1 | 1 | 1 | 1 | 3 | 3 | 3 | 3 | 3 | 3 | 3 | 3 |
| | | 1 | 1 | 1 | 1 | 3 | 3 | 3 | 3 | 1 | 1 | 1 | 1 | 3 | 3 | 3 | 3 |
| | | 1 | 1 | 3 | 3 | 1 | 1 | 3 | 3 | 1 | 1 | 3 | 3 | 1 | 1 | 3 | 3 |
| | 3' | 1 | 3 | 1 | 3 | 1 | 3 | 1 | 3 | 1 | 3 | 1 | 3 | 1 | 3 | 1 | 3 |
| Proportion of β-form of deoxyribose (%) | | 100 | 100 | 100 | 100 | 100 | 100 | 100 | 100 | 100 | 100 | 100 | 100 | 100 | 100 | 100 | 100 |
| Number of chromophores or luminophores | | 4 | 4 | 4 | 4 | 4 | 4 | 4 | 4 | 4 | 4 | 4 | 4 | 4 | 4 | 4 | 4 |
| Proportion of natural nucleic acid base (%) | 0 | 0 | 0 | 0 | 0 | 0 | 0 | 0 | 0 | 0 | 0 | 0 | 0 | 0 | 0 | 0 | |

### (5-3) Synthesis of luminescent probes 1 and 32 to 45

As shown in Table 5 below, 15 types of oligonucleotides (sequences 1 and 32 to 45) having a mixed sequence of the monomer 1 and the thymidine-containing monomer 4 described above were synthesized by a method similar to the method for synthesizing the luminescent probes 1 to 16 described above. In addition, it was confirmed that each of the luminescent probes 1 and 32 to 45 emitted fluorescence and excimer light emission by specific excitation light (light of wavelength 350 nm).

**[Table 5]**

| Luminescent color dye molecule | | 1 | 32 | 33 | 34 | 35 | 36 | 37 | 38 | 39 | 40 | 41 | 42 | 43 | 44 | 45 |
|---|---|---|---|---|---|---|---|---|---|---|---|---|---|---|---|---|
| Monomer sequence | 5' | 1 | 1 | 1 | 1 | 1 | 1 | 1 | 1 | 4 | 4 | 4 | 4 | 4 | 4 | 4 |
| | | 1 | 1 | 1 | 1 | 4 | 4 | 4 | 4 | 1 | 1 | 1 | 1 | 4 | 4 | 4 |
| | 3' | 1 | 1 | 4 | 4 | 1 | 1 | 4 | 4 | 1 | 1 | 4 | 4 | 1 | 1 | 4 |
| | | 1 | 4 | 1 | 4 | 1 | 4 | 1 | 4 | 1 | 4 | 1 | 4 | 1 | 4 | 1 |
| Proportion of β-form of deoxyribose (%) | | 100 | 100 | 100 | 100 | 100 | 100 | 100 | 100 | 100 | 100 | 100 | 100 | 100 | 100 | 100 |
| Number of chromophores or luminophores | | 4 | 3 | 3 | 2 | 3 | 2 | 2 | 1 | 3 | 2 | 2 | 1 | 2 | 1 | 1 |
| Proportion of natural nucleic acid base (%) | | 0 | 25 | 25 | 50 | 25 | 50 | 50 | 75 | 25 | 50 | 50 | 75 | 50 | 75 | 75 |

### (5-4) Synthesis of luminescent probes 46 to 60

As shown in Table 6 below, 15 types of oligonucleotides (sequences 46 to 60) having a mixed sequence of the monomer 5 having an α-form sugar structure and the monomer 2 were synthesized by a method similar to the method for synthesizing the above-described luminescent probes 1 to 16. It was confirmed that each of the luminescent probes 46 to 60 emitted fluorescence and excimer light emission by specific excitation light (light having a wavelength of 350 nm).

**[Table 6]**

| Luminescent color dye molecule | | 46 | 47 | 48 | 49 | 50 | 51 | 52 | 53 | 54 | 55 | 56 | 57 | 58 | 59 | 60 |
|---|---|---|---|---|---|---|---|---|---|---|---|---|---|---|---|---|
| Monomer sequence | 5' | 5 | 5 | 5 | 5 | 5 | 5 | 5 | 5 | 2 | 2 | 2 | 2 | 2 | 2 | 2 |
| | | 5 | 5 | 5 | 5 | 2 | 2 | 2 | 2 | 5 | 5 | 5 | 5 | 2 | 2 | 2 |
| | 3' | 5 | 5 | 2 | 2 | 5 | 5 | 2 | 2 | 5 | 5 | 2 | 2 | 5 | 5 | 2 |
| | | 5 | 2 | 5 | 2 | 5 | 2 | 5 | 2 | 5 | 2 | 5 | 2 | 5 | 2 | 5 |
| Proportion of β-form of deoxyribose (%) | | 0 | 0 | 0 | 0 | 0 | 0 | 0 | 0 | 0 | 0 | 0 | 0 | 0 | 0 | 0 |
| Number of chromophores or luminophores | | 4 | 3 | 3 | 2 | 3 | 2 | 2 | 1 | 3 | 2 | 2 | 1 | 2 | 1 | 1 |
| Proportion of natural nucleic acid base (%) | | 0 | 0 | 0 | 0 | 0 | 0 | 0 | 0 | 0 | 0 | 0 | 0 | 0 | 0 | 0 |

### 2-2. Analysis using luminescent probe

### (1) Analysis using luminescent probes 1 to 15

### • Blank information acquisition step

A plurality of 96-well microplates in each of which wells each having 7 mm opening diameter were disposed at the interval of 9 mm in 12 columns and × 8 rows were prepared. In the 96-well microplates, 100 µl of each of the luminescent probes 1 to 15 described above was placed for all the targets by an automatic dispenser (NichiMart CUBE, manufactured by NICHIRYO Co., Ltd) to form a plurality of reaction fields.

The 96-well microplate on which the above-described luminescent probes were placed was irradiated with excitation light (wavelength 350 nm), and the fluorescence spectrum was obtained as first signal information (blank information) for each luminescent probe.

### • Interaction step

On the 96-well microplates after the blank information acquisition step, 20 µl of each of seven types and 95 brands of beverages (type I, 12 brands, type II, 23 brands, type III, 6 brands, type IV, 10 brands, type V, 20 brands, type VI, 13 brands, type VII, and 11 brands, all of which are targets) was placed in the same manner as described above.

### • Light detection step

The 96-well microplate containing the above-described luminescent probes and targets was irradiated with excitation light (wavelength 350 nm), and the fluorescence spectrum was obtained as second signal information for each luminescent probe.

### • Analysis step

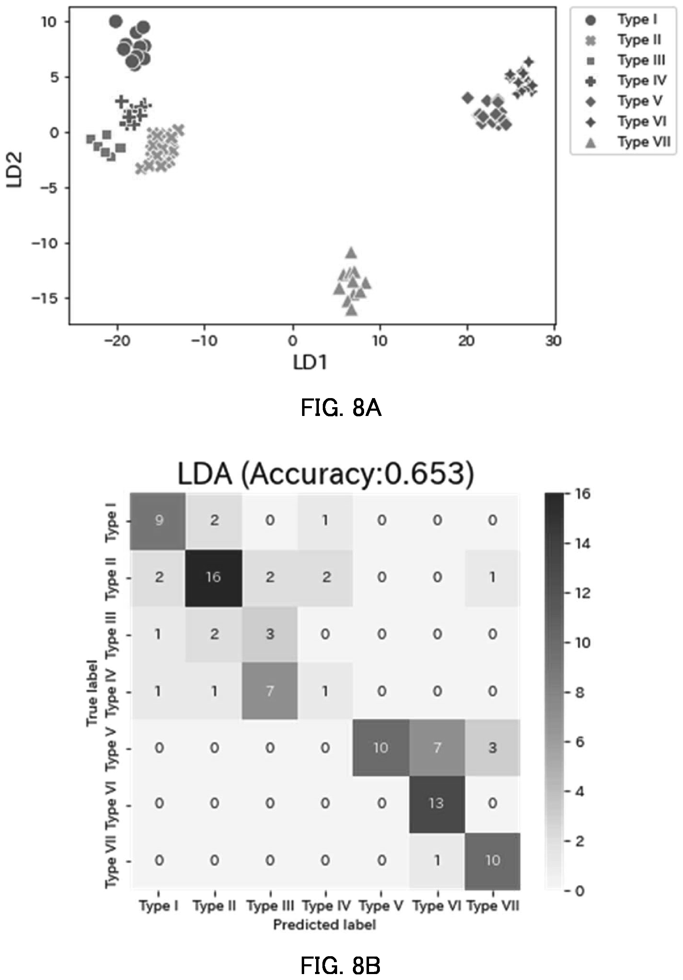

Analysis data was calculated by subtracting the first signal information acquired in the blank information acquisition step from the second signal information acquired in the light detection step. Then, learning was performed using the analysis data as explanatory variables and the classification data of the beverage as objective variables, and a discriminant model was created by linear discriminant analysis (LDA). The obtained linear discriminant analysis model plot is illustrated in Fig. 8A. Thereafter, calculation of a correct answer rate and creation of a confusion matrix were performed by cross-validation of 6 divisions, and generalization performance of the discriminant model was quantified. The confusion matrix is illustrated in Fig. 8B.

### • Results

As illustrated in Fig. 8B, the discriminant model was able to classify the types of 95 brands of beverages with an accuracy of about 65%.

### (2) Analysis using luminescent probes 1 and 17 to 31

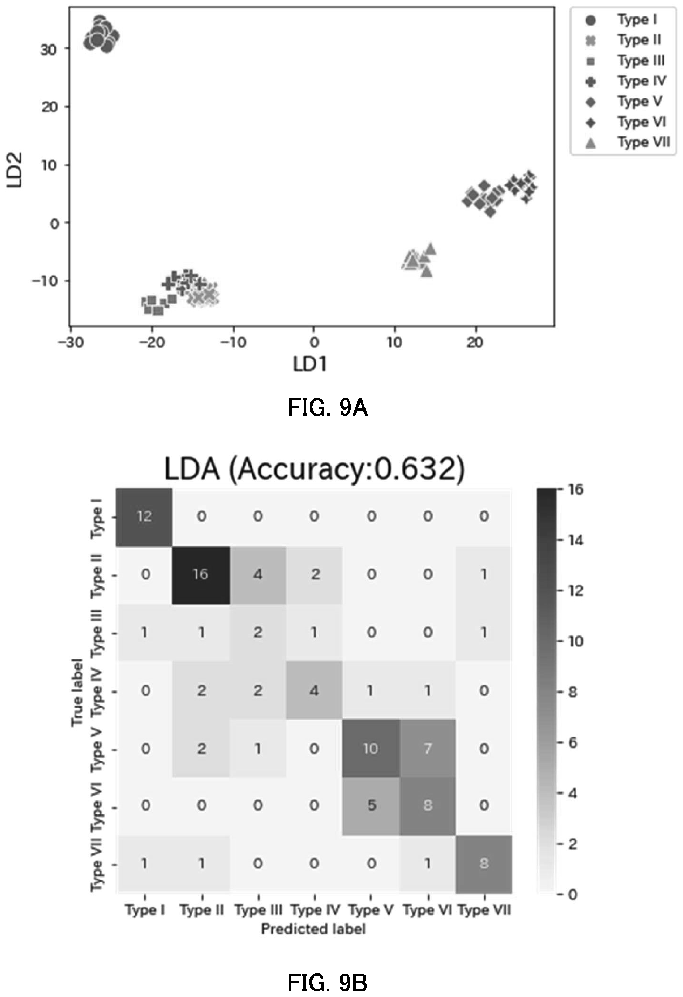

The blank information acquisition step to the analysis step described above were performed in the same manner using the luminescent probes 1 and 17 to 31 (16 types). The obtained linear discriminant analysis model plot is illustrated in Fig. 9A. Then, calculation of a correct answer rate and creation of a confusion matrix were performed by cross-validation of six divisions, and generalization performance of the discriminant model was quantified. The confusion matrix is illustrated in Fig. 9B.

### • Results

As illustrated in Fig. 9B, the discriminant model was able to classify the types of 95 brands of beverages with an accuracy of about 63%.

### (3) Analysis using Luminescent probes 1 to 15 and 17 to 31

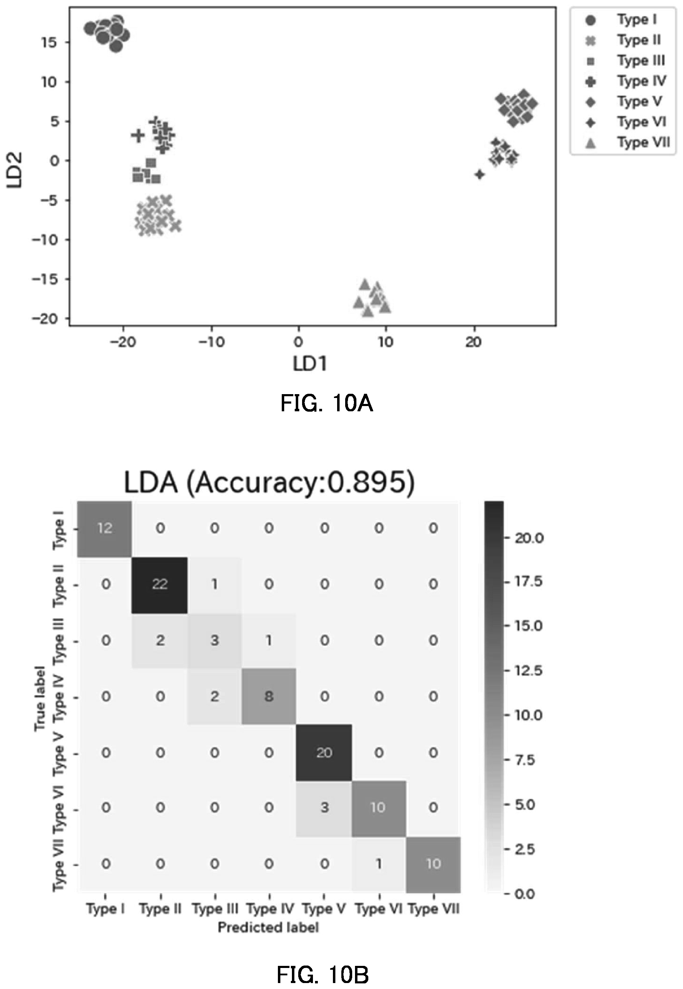

The blank information acquisition step to the analysis step described above were performed in the same manner using the luminescent probes 1 to 15 and 17 to 31 (30 types). The linear discriminant analysis model plot thus obtained is illustrated in Fig. 10A. Then, calculation of a correct answer rate and creation of a confusion matrix were performed by cross-validation of six divisions, and generalization performance of the discriminant model was quantified. The confusion matrix is illustrated in Fig. 10B.

### • Results

As illustrated in Fig. 10B, the discriminant model was able to classify the types of 95 brands of beverages with an accuracy of about 90%.

### (4) Analysis using luminescent probes 1 and 32 to 45

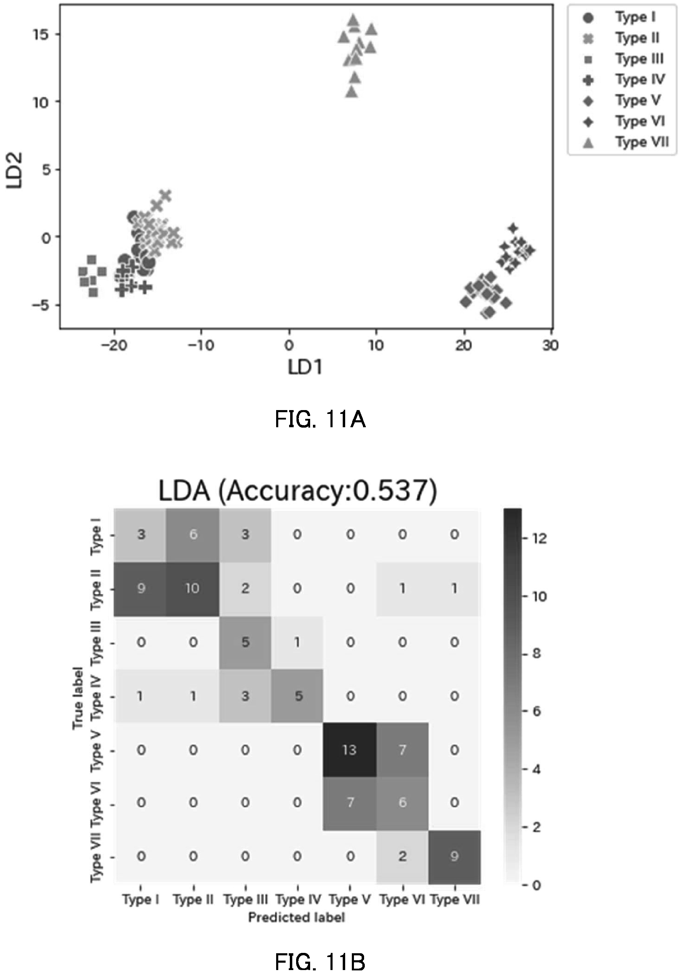

The blank information acquisition step to the analysis step described above were performed in the same manner using luminescent probes 1 and 32 to 45 (15 types). The linear discriminant analysis model plot thus obtained is illustrated in Fig. 11A. Then, calculation of a correct answer rate and creation of a confusion matrix were performed by cross-validation of six divisions, and generalization performance of the discriminant model was quantified. The confusion matrix is illustrated in Fig. 11B.

### • Results

As illustrated in Fig.11B, according to the above-described discriminant model, 95 brands could be classified with an accuracy of about 54% for each type.

### (5) Analysis using luminescent probes 46 to 60

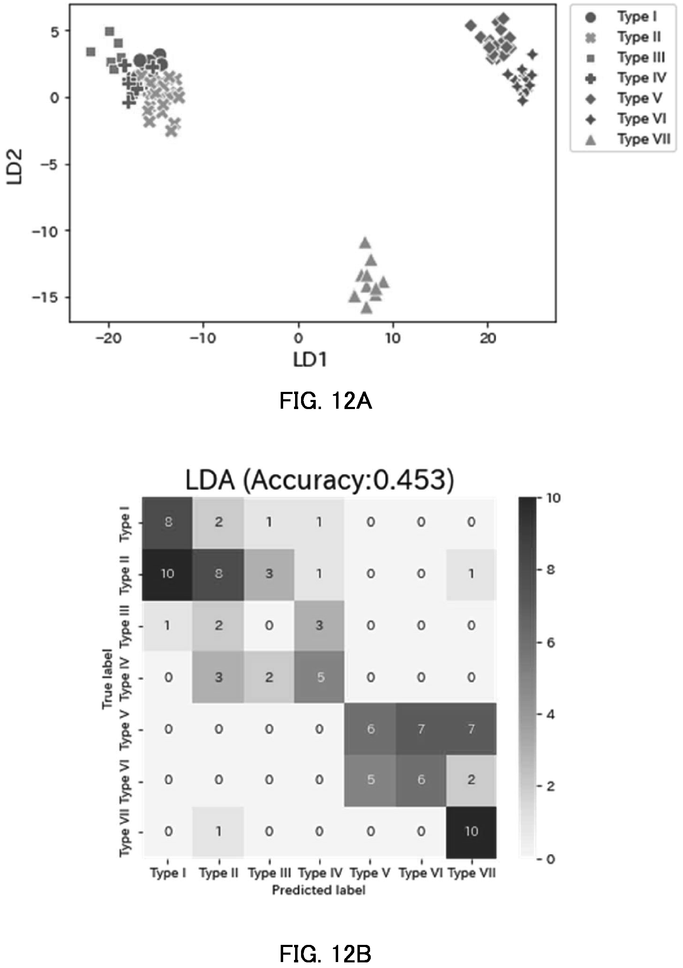

The blank information acquisition step to the analysis step described above were performed in the same manner using luminescent probes 46 to 60 (15 types). The linear discriminant analysis model plot thus obtained is illustrated in Fig. 12A. Then, calculation of a correct answer rate and creation of a confusion matrix were performed by cross-validation of six divisions, and generalization performance of the discriminant model was quantified. The confusion matrix is illustrated in Fig. 12B.

### • Results

As illustrated in Fig.12B, according to the above-described discriminant model, 95 brands could be classified with an accuracy of about 45% for each type. The reason why the accuracy was lower than those of the other analyses can be exemplified by a small ratio of the β-form sugar structure in the luminescent probe.

### (6) Analysis when explanatory variables are randomly permuted

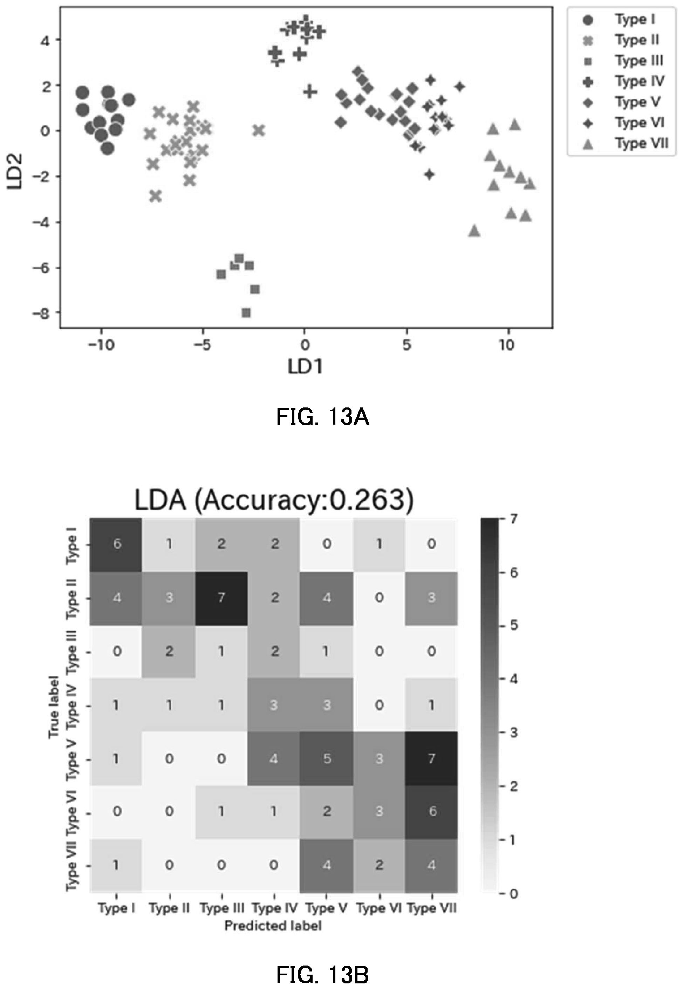

In order to clarify the effect of the present invention, a model was created by randomly permuting explanatory variables as a negative control experiment. Specifically, the above-described measurement and analysis steps were carried out using luminescent probes 1 to 15 (15 types), and the explanatory variables obtained were randomly permuted to create a discrimination model. The obtained linear discriminant analysis model plot is illustrated in Fig. 13A. Then, calculation of a correct answer rate and creation of a confusion matrix were performed by cross-validation of six divisions, and generalization performance of the discriminant model was quantified. The confusion matrix is illustrated in Fig. 13B.

### • Results

As illustrated in Fig. 13B, the discriminant model in which the explanatory variables were randomly permuted was able to classify the types of 95 brands of beverages at about 25%. This result indicates that the above-described accuracy was not achieved by chance when using the above-described luminescent probes and a discrimination model in which explanatory variables and objective variables were correctly set. From the above results, it is understood that various compounds can be analyzed with high accuracy according to the discriminant model in which the explanatory variables and the objective variables are correctly set using the luminescent probes.

Furthermore, from the above results and the like, it was possible to separate the data on the soft drinks by type by using, as the luminescent probes, a probe including a light emitter of the order of nanometers whose light emission behavior changes chemically and/or physically by interaction with a target and further combining a plurality of light emitters different in size from each other. That is, such a change in physicochemical phenomenon (state information of the target) could be acquired in a short time and by a simple method by analysis using the luminescent probe.

### 3. Example 3

The following monomers X1 to X13 were prepared by applying the monomer synthesis methods described in example 1 and example 2. In addition, when the analysis method was performed using oligomers using the above monomers, discrimination was possible in the same manner as described above.

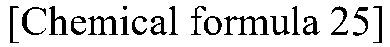

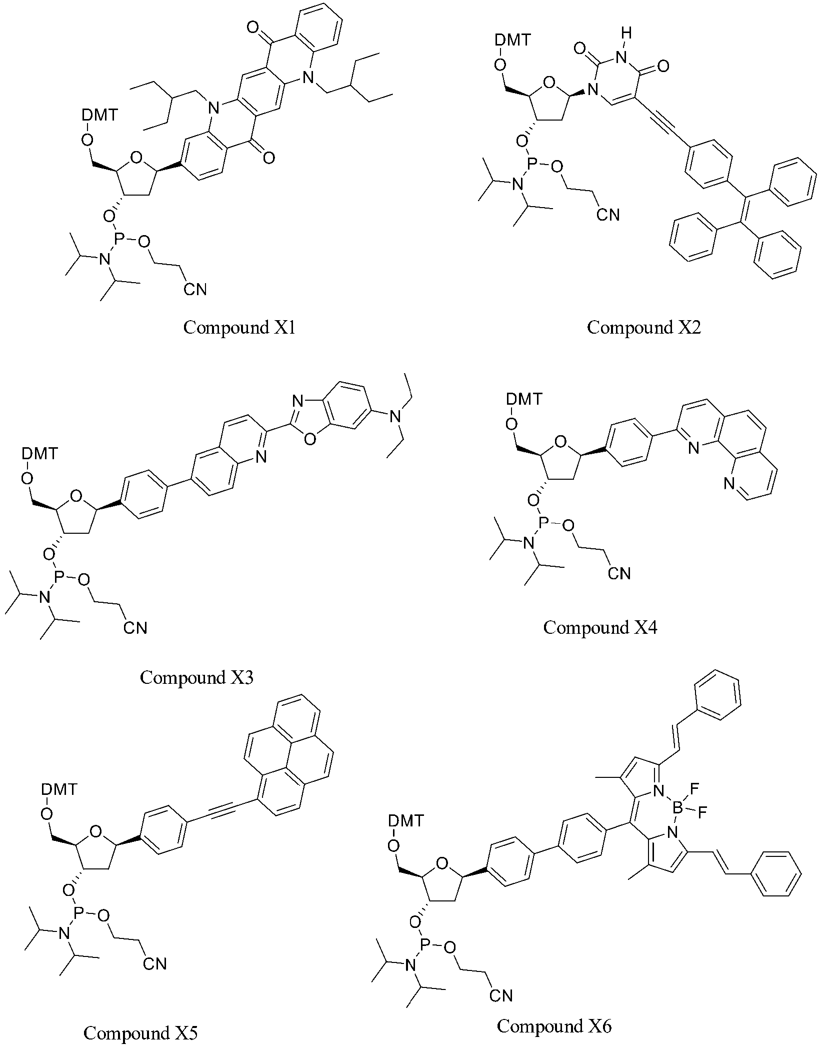

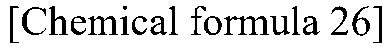

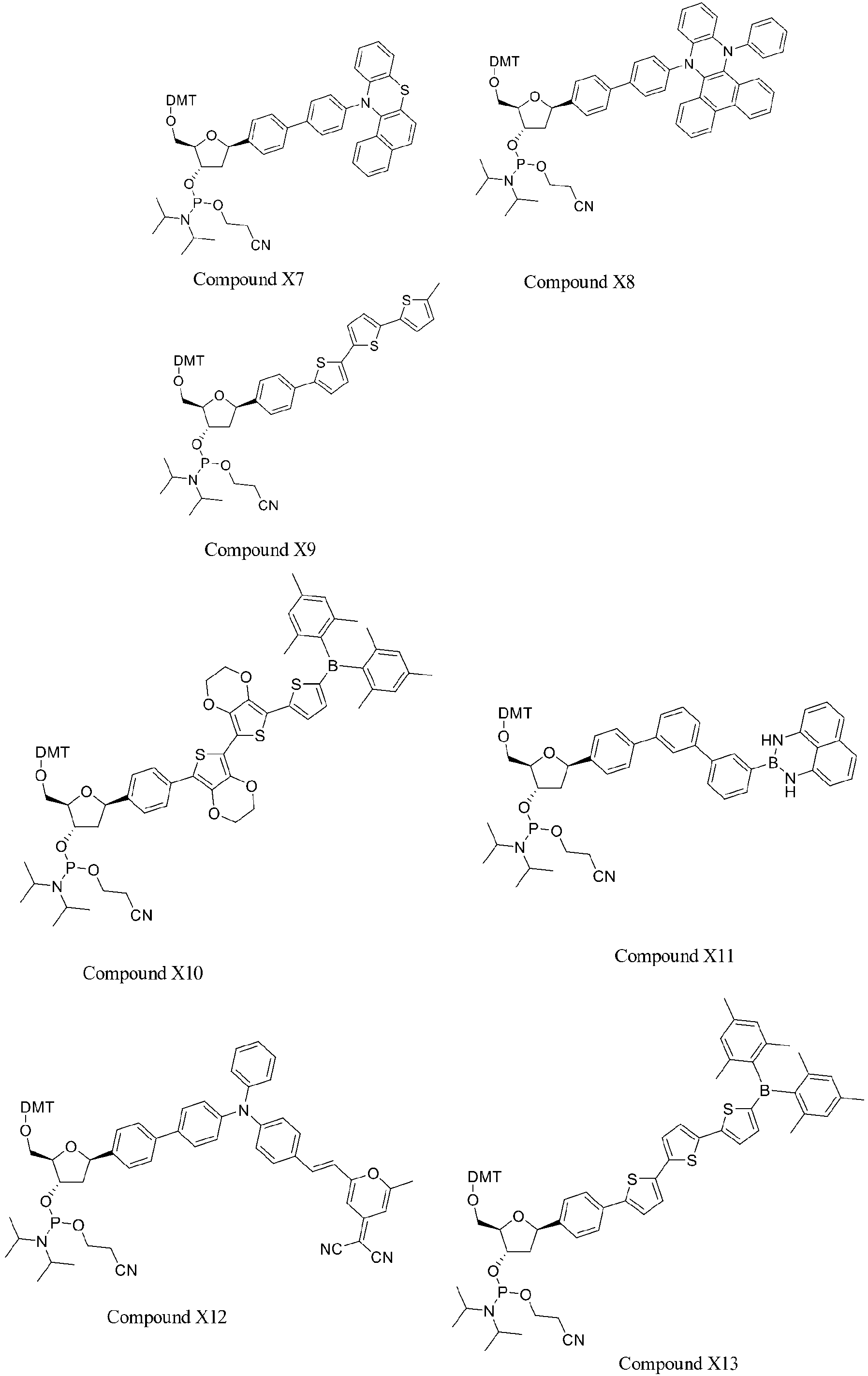

### (Measurement of size of light emitter of monomers X1 to X13)

For the monomers X1 to X13, the size of the structure (light emitter contributing to light emission) represented by R in the following general formula was calculated by the same method as described above. The results are shown in Table 3.

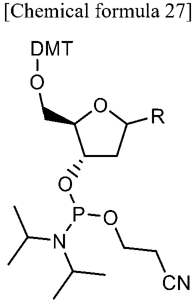

**[Table 7]**

| Compound | Molecular size of substituent group R (nm) |
|---|---|
| Compound X1 | 1.56 |
| Compound X2 | 2.00 |
| Compound X3 | 2.15 |
| Compound X4 | 1.37 |
| Compound X5 | 1.65 |
| Compound X6 | 2.20 |
| Compound X7 | 1.49 |
| Compound X8 | 1.72 |
| Compound X9 | 1.95 |
| Compound X10 | 2.40 |
| Compound X11 | 1.95 |
| Compound X12 | 2.28 |
| Compound X13 | 2.26 |

### 4. Example 4

### < Production of sensing plate >

A substrate in which an indium tin oxide (ITO) film was formed to be 100 nm thick on a 30 mm × 30 mm × 0.7 mm glass substrate was patterned, then ultrasonically washed with isopropyl alcohol, dried with dry nitrogen gas, and subjected to UV ozone cleaning for 5 minutes, thereby obtaining a transparent support substrate provided with an ITO transparent electrode (anode).

On the formed anode, a 1.0% dilute solution of polystyrene (manufactured by ACROS ORGANICS Co., Ltd., molecular weight = 260000) as an insulating polymer and as a solute dissolved in N-propyl acetate as a solvent was formed into a film by spin coating at 500 rpm for 30 seconds, and then dried at 120°C for 30 minutes, thereby providing an ink receiving layer with a thickness of 50 nm

### < Setting of test material (target) on sensing plate >

As test materials, three types of commercially available drinking water (drinking water A, drinking water B, and drinking water C) were prepared. The sensing plate was immersed for 1 minute in a petri dish into which each liquid had been poured, and then the liquid was removed with an air gun and dried.

### < Setting of luminescent compound (luminescent probe) to sensing plate >

A luminescent compound (luminescent probe: blue phosphorescence emitting compound Epl described below) was mixed at a concentration of 10 mg/mL with N-propyl acetate as a solvent, and the mixture was heated with ultrasound for 30 minutes and then filtered through a 0.2-µm filter to remove aggregated components, thereby producing a luminescent ink.

Next, by an inkjet printing method under the following conditions, a liquid (luminescent ink) containing a phosphorescent compound Ep1 having the structure shown below was dropped onto the ink receiving layer to form a detection region, followed by drying for 30 min at 120°C to evaporate the solvent.

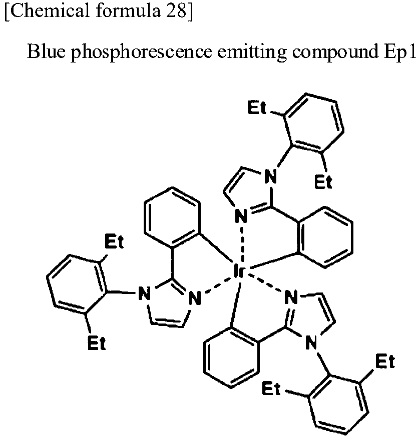

The conditions of the inkjet printing method were as follows. As the inkjet printing system, IJCS-1 manufactured by Konica Minolta, Inc. was used, and as the inkjet head, KM512 manufactured by Konica Minolta, Inc. was used. The number of ejection shots was 2, the pitch of ejection nozzles from the head was 140 µm, and the head scanning speed was 90 mm/sec for the printing.

Next, the resultant was attached to a vacuum vapor deposition apparatus, the pressure of the vacuum tank was reduced to 4 × 10⁻⁴ Pa, and then an electron injection layer and a cathode were formed under the following conditions. The electron injection layer was formed by vapor depositing potassium fluoride at a film formation rate of 0.1 Å/sec so as to have a thickness of 2.0 nm. The cathode was formed by vapor depositing Al at a film formation rate of 4 Å/sec so as to have a 100 nm.

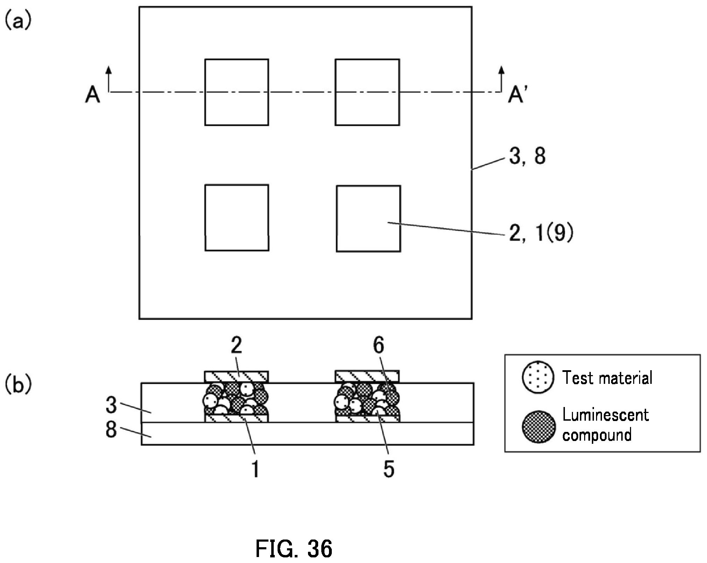

Through the above steps, a sensing element (sensing device) was produced. Part (a) in Fig. 36 is a plan view of a sensing element, and part (b) in Fig. 36 is a schematic cross-sectional view taken along line A-A'. Note that reference numerals in Fig. 36 are the same as those in Fig. 6 and the like, and reference numeral 8 denotes a substrate and reference numeral 9 denotes an electrode region (a region where the anode 1 and the cathode 2 overlap each other in a plan view) as reference numerals which are not illustrated in Fig. 6 and the like. Here, the electrode 1 represents an anode, and the electrode 2 represents a cathode.

In the produced sensing element, four detection regions were present in the 30 × 30 mm (2 × 2mm) area, and the detection regions (inkjet regions) had the same size as the electrodes.

Since the polystyrene, namely the receiving layer 3, is insoluble in the test material, a very small amount of solid components of the test material is present on the polystyrene surface layer at the time of immersion and drying. Thereafter, at the time when the luminescent compound is applied by inkjet coating, the inkjet ink solvent dissolves the polystyrene and reaches the lower electrode while forming a dispersed state with the test material. As a result, as illustrated in part (b) in Fig. 36, a detection region in which the luminescent compound and the test material are dispersed at a molecular level is formed. (Note that the test material present on the receiving layer outside the detection region is not illustrated)

In the present example, two sensing elements were prepared for each type of test material, and the number of measurement points for each test material was eight (for a total of 24 measurement points for each type). A voltage can be applied to the anode and the cathode through wiring (not illustrated).

### < Data acquisition >

The spectral-radiance spectrum [W·sr⁻¹·m⁻²·nm⁻¹] and the current density [mA/cm²] at each drive voltage of the fabricated sensing element 1 were measured. The drive voltage was changed from 1 to 10v in 1v increments, and the current at each voltage was measured.

The luminance was measured with a spectral radiance meter CS-2000 (manufactured by Konica Minolta, Inc). For the current measurement, 6243 DC VOLTAGE CURRENT SOURCE/MONITOR (manufactured by ADCMT) was used.

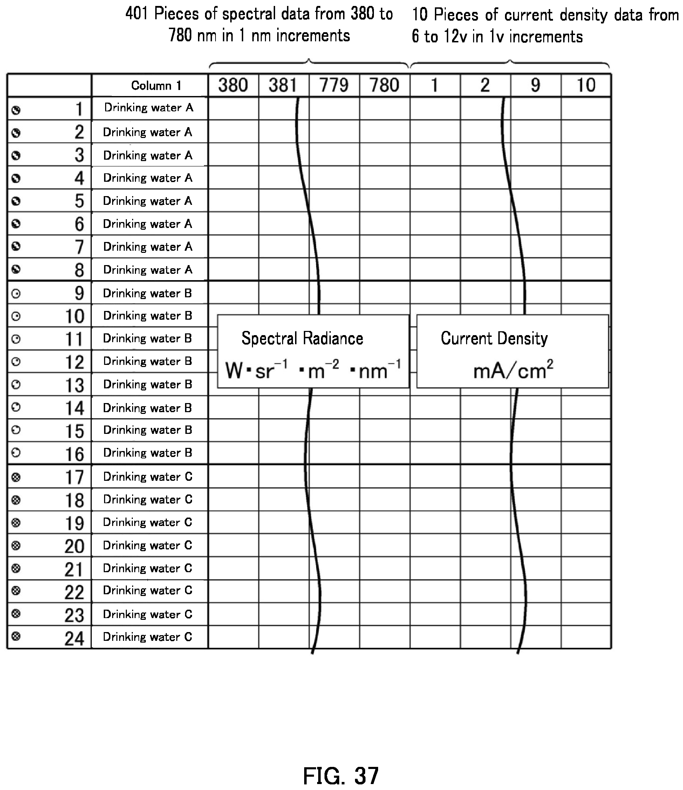

The resulting data set is illustrated in Fig. 37. In the row direction, eight rows are formed for each type of test material, that is, 24 rows are formed in total. In the column direction, there are 401 pieces of spectral data from 380 to 780 nm in 1 nm increments and 10 pieces of current density data from 1 to 10v in 1v increments. The spectral data were normalized by setting the value at the wavelength at which the radiance was the highest to 1. The voltage was normalized with the current density value at the 10v being 1, to reduce the influence of variations among elements.

### < Discriminant analysis >

Discriminant analysis was performed on the basis of the above data sets.

Linear discriminant analysis (LDA) was performed using statistical analyzing software, JMP16. 2, available from SAS Institute Japan Corporation. The discriminant analysis is described in detail in, for example, "p173-193 (chapter 6)" of "JMP's Multivariable Data Collection, Kaibundo Publishing Co., Ltd."

From the measurement data, a canonical function 1 and a canonical function 2 were obtained, and 24 discriminant scores corresponding to the canonical axis 1 and 24 discriminant scores corresponding to the canonical axis 2 were obtained. Next, discriminant plots (plots with the canonical axes 1 and 2 as axes) were created using 42 discriminant scores corresponding to the rows 2 to 8, the rows 10 to 16, and the rows 18 to 24 of the data set. Then, the three remaining discrimination scores for verification corresponding to rows 1, 9, and 17 were determined from the Mahalanobis distance to determine which group in the discrimination plot was closest to them, and the number of correct answers was counted.

Next, the combinations were changed from rows 1 and 3 to 8, from rows 9 and 11 to 16, and from rows 17 and 19 to 24, and a discrimination plot consisting of 42 corresponding discrimination scores was created. Then, the three discriminant scores for verification corresponding to remaining rows 2, 10, and 18 were determined from the Mahalanobis distance to determine which group in the discriminant plot was closest to, and the number of correct answers was counted.

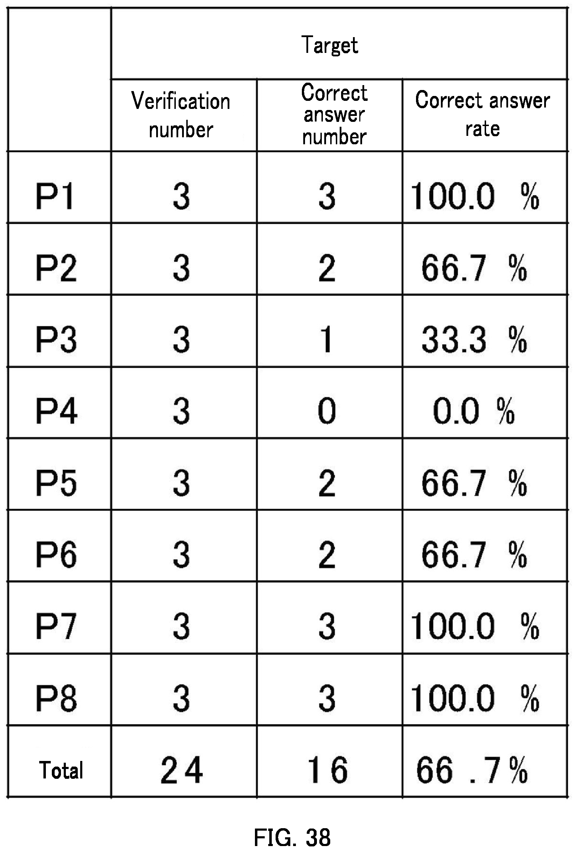

In the same manner, the combination was changed eight times (discriminant analysis was performed eight times), and the number of times that the three types of samples (drinking water A, drinking water B, drinking water C) gave correct answers was counted for 24 verifications. The results are illustrated in Fig. 38.

The number of correct answers out of 24 verifications was 16, and the discrimination rate (number of correct answers/number of verifications) was 66.7%. This result greatly exceeds the probability of chance of 33.3%, indicating that the present sensing system is effective.

Note that, in the JMP software, the operation was performed in the following procedures.
1. The following table (data set) is read from the "file" menu.
2. The 1, 9, and 17th rows of the table are selected, and "exclude" is selected by right-clicking.
3. "Multivariate" to "discriminant analysis" are specified from the "analysis" menu.
4. The "column 1" is assigned to "X, category," the "remaining columns" are assigned to "Y, covariate," and the discriminant method is assigned to "linear horizontally long data." After performing "verify model with excluded rows" in a detail option, the "OK" button was pressed to perform the calculation.
5. The number of correct answers was calculated by subtracting the "number of misclassifications" for "exclusion" in the "summary of scores" from 3.
6. The sequence 2 was changed to the 2, 10, and 18th rows (the combination was changed), and the procedures 2 to 5 were performed.

This was repeated a total of eight times.

In addition, when the above-described analysis method was performed using oligomers using the above-described compounds P-1 to P-11 in place of the phosphorescent compound Epl, discrimination was possible in the same manner as described above.

**[Table 8]**

| Compound | Molecular size (nm) |
|---|---|
| p-1 | 2.1417 |
| p-2 | 2.0453 |
| p-3 | 2.5104 |
| p-4 | 2.0854 |
| p-5 | 1.2603 |
| p-6 | 1.2224 |
| p-7 | 1.7797 |
| p-8 | 1.2057 |
| p-9 | 1.5494 |
| p-10 | 1.5947 |
| p-11 | 1.3924 |

### 5. Example 5

### < Production of sensing plate >

A substrate in which an indium tin oxide (ITO) film was formed to be 100 nm thick on a 30 mm × 30 mm × 0.7 mm glass substrate was patterned. Thereafter, the film was subjected to ultrasonic cleaning with isopropyl alcohol, dried with dry nitrogen gas, and subjected to UV ozone cleaning for 5 minutes, thereby obtaining a transparent support substrate provided with an ITO transparent electrode (anode).

On the formed anode, a 1.0% dilute solution of polystyrene (manufactured by ACROS ORGANICS Co., Ltd., molecular weight = 260000) as an insulating polymer and as a solute dissolved in N-propyl acetate as a solvent was formed into a film by spin coating at 500 rpm for 30 seconds. Thereafter, the coating film was dried at 120°C for 30 minutes to provide a 50 nm thick ink receiving layer.

### < Setting of test material (target) on sensing plate >

Mg ion aqueous solutions having concentrations of 10ppb, 100ppb, 1 ppm, 10 ppm, 100 ppm, and 1000 ppm were prepared as test materials (targets). The sensing plate was immersed for 1 minute in a petri dish into which each liquid had been poured, and then the liquid was removed with an air gun and dried.

### < Setting of luminescent compound (luminescent probe) to sensing plate >

A luminescent compound (luminescent probe, the same blue phosphorescence emitting compound Ep1 as in example 4) was mixed at a concentration of 10 mg/mL with N-propyl acetate as a solvent, and the mixture was heated with ultrasound for 30 minutes and then filtered through a 0.2-µm filter to remove aggregated components, thereby producing a luminescent ink.

Next, a liquid (luminescent ink) containing the phosphorescent compound Epl was dropped onto the ink receiving layer by an inkjet printing method under the following conditions to form a detection region, followed by drying for 30 min at 120°C to evaporate the solvent.

The conditions of the inkjet printing method were as follows. As the inkjet printing system, IJCS-1 manufactured by Konica Minolta, Inc. was used, and as the inkjet head, KM512 manufactured by Konica Minolta, Inc. was used. The number of ejection shots was 2, the pitch of ejection nozzles from the head was 140 µm, and the head scanning speed was 90 mm/sec for the printing.

Next, the resultant was attached to a vacuum vapor deposition apparatus, the pressure of the vacuum tank was reduced to 4 × 10⁻⁴ Pa, and then an electron injection layer and a cathode were formed under the following conditions. The electron injection layer was formed by vapor depositing potassium fluoride at a film formation rate of 0.1 Å/sec so as to have a thickness of 2.0 nm. The cathode was formed by vapor depositing Al at a film formation rate of 4 Å/sec so as to have a 100 nm.

Through the above steps, the sensing element (sensing device) illustrated in Fig. 36 was produced. Part (a) in Fig. 36 is a plan view of a sensing element, and part (b) Fig. 36 is a schematic cross-sectional view taken along line A-A'.

In the produced sensing element, four detection areas were present in the 30 × 30 mm (2 × 2mm) area, and the detection areas (inkjet regions) had the same size as the electrodes.

Since the polystyrene, namely the receiving layer 3, is insoluble in the test material, a very small amount of solid components of the test material (target) is present on the polystyrene surface layer at the time of immersion and drying. Thereafter, at the time when the luminescent compound (luminescent probe) is applied by inkjet coating, the inkjet ink solvent dissolves the polystyrene and reaches the lower electrode while forming a dispersed state with the test material. As a result, as illustrated in part (b) in Fig. 36, a detection region in which the luminescent compound and the test material are dispersed at a molecular level is formed. (Note that the test material present on the receiving layer outside the detection region is not illustrated)

In this example, two sensing elements 1 were prepared for each type of test material, and the number of measurement points of each test material was eight (three types, a total of 24 measurement points). A voltage can be applied to the anode and the cathode through wiring (not illustrated).

The spectral-radiance spectrum [W·sr⁻¹·m⁻²·nm⁻¹] and the current density [mA/cm²] at each drive voltage of the fabricated sensing element 1 were measured. The drive voltage was changed from 1 to 10v in 1v increments, and the current at each voltage was measured.

The luminance was measured with a spectral radiance meter CS-2000 (manufactured by Konica Minolta, Inc). For the current measurement, 6243 DC VOLTAGE CURRENT SOURCE/MONITOR (manufactured by ADCMT) was used.

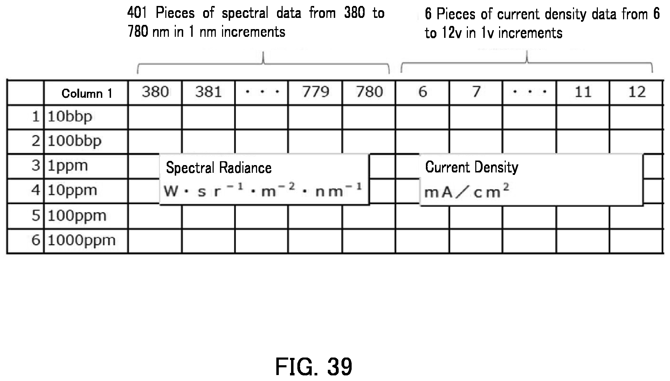

The resulting data set is illustrated in Fig. 39. In the row direction, eight rows are formed for each type of test material, that is, 24 rows are formed in total. In the column direction, there are 401 pieces of spectral data from 380 to 780 nm in 1 nm increments and 6 pieces of current density data from 6 to 12v in 1v increments. The spectral data were normalized by setting the value at the wavelength at which the radiance was the highest to 1. The voltage was normalized with the current density value at the 10v being 1, to reduce the influence of variations among elements.

### < Discriminant analysis >

Discriminant analysis was performed on the basis of the above data sets.

Linear discriminant analysis (LDA) was performed using statistical analyzing software, JMP16. 2, available from SAS Institute Japan Corporation. The discriminant analysis is described in detail in, for example, "p173-193 (chapter 6)" of "JMP's Multivariable Data Collection, Kaibundo Publishing Co., Ltd."

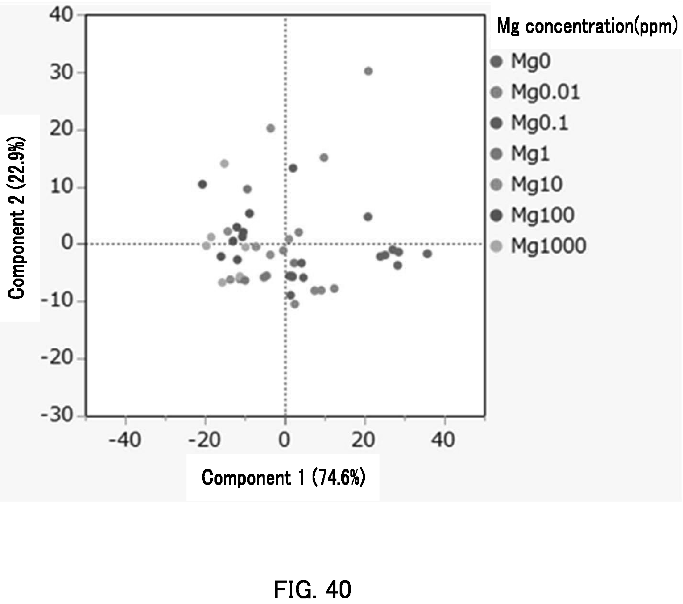

Principal component analysis was performed on the measurement data, and mapping was performed using the main component 1 and the main component 2. Further, correlation analysis with the main components 1 and 2 with respect to each ion concentration was performed on the main component mapping. The results are illustrated in Fig. 40.

The correlation with the ion concentration on the main component mapping was remarkably observed particularly in the main component 1. In addition, with respect to the relationship between the common logarithm of the Mg ion concentration and the main component 1, when a correlation coefficient was calculated by dividing each of these covariances by the product of the standard deviations thereof, the correlation coefficient was -0.88.

As described above, it has become clear that the signal data obtained from the present sensing system is strongly correlated with the Mg ion concentration in the sample in a very wide range from 10ppb to 1000 ppm, which indicates that the present sensing system is effective.

Note that, in the JMP software, the operation was performed in the following procedures.
1. The following table (data set) is read from the "file" menu.
2. The 1, 9, and 17th rows of the table are selected, and "exclude" is selected by right-clicking.
3. "Multivariate" to "discriminant analysis" are specified from the "analysis" menu.
4. The "column 1" is assigned to "X, category," the "remaining columns" are assigned to "Y, covariate," and the discriminant method is assigned to "linear horizontally long data." After performing "verify model with excluded rows" in a detail option, the "OK" button was pressed to perform the calculation.
5. The number of correct answers was calculated by subtracting the "number of misclassifications" for "exclusion" in the "summary of scores" from 3.
6. The sequence 2 was changed to the 2, 10, and 18th rows (the combination was changed), and the procedures 2 to 5 were performed. This was repeated a total of eight times.

This application claims the benefit of Japanese Patent Application No. 2023-121744, filed Jul. 26, 2023. The entire contents of the specification and drawings of the application are incorporated herein by reference.

### Industrial Applicability

The present invention provides the most fundamental technology for transforming mass production into on-demand production and long-term development into immediate development in order to realize a cyclic society. The present invention is intended to describe, record and evaluate, with high sensitivity, the states of substances related to the production, processing and quality assurance of chemicals, materials, bio-related substances, foods, beverages and the like, the control of wastewater/sewage treatment and the like, and furthermore, the traceability and IDs of raw materials and production of agricultural products, dairy products and processed products thereof and the like, based on the nm scale and the Å scale.

### Reference Signs List

1, 2 Electrode
3 Receiving layer
4, 4A, 4B Container
5 Target
6 Luminescent probe
8 Substrate
9 Electrode region
10 Light source unit
11 Anode
12 Insulator
13 Light emitter
14 Cathode
15 Sealing member
16 Barrier material
17 Circuit
18 Power receiver
19 Receiving layer
20 Multi-well unit
21 Cured layer
22 Microwell structure part
31 Support
50 Organic matter
51 Microorganisms (activated sludge)
100 Analysis apparatus
110 Microarray device

## Claims

1. An analysis method, comprising:
causing a target and at least one type of luminescent probe to interact with each other by mixing the target and the at least one type of luminescent probe whose light emission behavior changes by interaction with the target;
exciting the at least one type of luminescent probe in an interaction state with the target to generate two or more types of light emission, and simultaneously detecting the two or more types of light emission; and
specifying a state of the target by analyzing the detected the two or more types of light emission.

2. The analysis method according to claim 1, wherein
the at least one type of luminescent probe includes a light emitter that is excited by irradiation with excitation light to emit light; and
a size of the light emitter is 100 nm or less.

3. The analysis method according to claim 2, wherein:
the causing the target and the at least one type of luminescent probe to interact with each other is causing the target and two or more types of luminescent probes to interact with each other; and
the two or more types of luminescent probes respectively include two or more of the light emitters whose sizes are different from each other.

4. The analysis method according to claim 1, wherein:
the causing the target and the at least one type of luminescent probe to interact with each other by mixing is carried out on a surface of or inside a receiving layer of an organic EL element that includes a pair of electrodes and the receiving layer held between the electrodes; and
in the receiving layer after the causing the target and the at least one type of luminescent probe to interact with each other by mixing, light emission is generated at a light emission site formed on the surface of or inside the receiving layer.

5. The analysis method according to claim 4, wherein:
the receiving layer after the causing the target and the at least one type of luminescent probe to interact with each other by mixing includes a plurality of the light emission sites; and
at least one of the light emission sites contains the luminescent probe and a host material, and a type of at least one of the luminescent probe and the host material is different between one light emission site and another light emission site among the light emission sites.

6. The analysis method according to claim 4, wherein
in the causing the target and the at least one type of luminescent probe to interact with each other by mixing,
a plurality of the light emission sites including different contents of the target are formed in the receiving layer.

7. The analysis method according to claim 4, wherein
in the causing the target and the at least one type of luminescent probe to interact with each other by mixing,
the light emission site further includes a substance that changes a carrier recombination position.

8. The analysis method according to claim 4, wherein
in the causing the target and the at least one type of luminescent probe to interact with each other by mixing,
an ink liquid is prepared and ejected from an inkjet head, the ink liquid containing at least one of the luminescent probe, a host material, the target, and a substance that changes a carrier recombination position.

9. The analysis method according to claim 8, wherein:
the receiving layer after the causing the target and the at least one type of luminescent probe to interact with each other by mixing includes a plurality of the light emission sites; and
the receiving layer includes 100 or more of the light emission sites per 1 cm².

10. The analysis method according to claim 4, further comprising:
preparing, as a comparison element, another organic EL element not including the target, wherein
in the specifying the state of the target, a difference between light emission detected in the comparison element and the light emission detected in the organic EL element is acquired and analyzed as a relative value.

11. The analysis method according to claim 1, wherein
the specifying the state of the target is analyzing the two or more types of light emission with a trained model constructed in advance.

12. The analysis apparatus, comprising:
a holder including at least one container for accommodating a target and at least one type of luminescent probe whose light emission behavior changes by interaction with the target;
an exciter for exciting the at least one type of luminescent probe disposed in the at least one container; and
a detector for simultaneously detecting two or more types of light emitted by the at least one type of luminescent probe.

13. The analysis apparatus according to claim 12, wherein:
the exciter includes a light source for exciting the at least one type of luminescent probe; and
the holder is a plate including the at least one container.

14. The analysis apparatus according to claim 12, wherein:
the exciter includes a pair of electrodes; and
the holder is held between the electrodes.

15. The analysis apparatus according to claim 12, further comprising:
an analyzer for analyzing information on the two or more types of light detected by the detector with a trained model constructed in advance to analyze a state of the target.
